# EUROPEAN PATENT APPLICATION

(11) **EP 4 495 241 A1**
(43) Date of publication of application: **22.01.2025**
(21) Application number: 23770816.9
(22) Date of filing: 15.03.2023
(51) Int. Cl.: C12N 15/113, A61K 31/713, A61K 47/54, A61K 48/00, A61P 43/00

(54) **CHEMICALLY-MODIFIED OLIGONUCLEOTIDE HAVING RNAI ACTIVITY**

(30) Priority: 16.03.2022 JP 2022041865
(71) Applicant: Daiichi Sankyo Company, Limited, Tokyo 103-8426 (JP)
(72) Inventor: KOIZUMI, Makoto, Tokyo 103-8426 (JP); SHOJI, Takao, Tokyo 103-8426 (JP); FUKUNAGA, Taichi, Tokyo 103-8426 (JP)
(74) Representative: Marks & Clerk LLP
(86) International application number: PCT/JP2023/009974
(87) International publication number: WO 2023/176863

(57) **Abstract**

There is provided an oligonucleotide or a pharmaceutically acceptable salt thereof with a novel chemical modification pattern, which has a RNA interfering action and/or a gene expression suppressing action. A sense strand region has a specific modification pattern with a 3'-modified nucleoside, and an antisense strand region has, in a specific region, a modification pattern in which DNA, RNA, 2'-O,4'-C-bridging-modified nucleosides, 2'-MOE RNA or the like are appropriately combined.

## Description

### Technical Field

The present invention relates to an oligonucleotide comprising a chemically modified nucleoside having a RNA interfering action and/or a gene expression suppressing action, use of the oligonucleotide, a method for suppressing gene expression using the oligonucleotide, a medical drug comprising the oligonucleotide, and the like.

### Background Art

Methods for inhibiting expression of a target gene in cells, tissues or individuals include methods in which double-stranded RNA is introduced into the cells, tissues or individuals. The introduction of double-stranded RNA degrades mRNA homologous to the sequence of the double-stranded RNA, and inhibits expression of a target gene. This effect is called "RNA interference" or "RNAi". Double-stranded RNA having an overhang with two nucleotides at the 3'-terminus, and a sense strand and an antisense strand each having 21 nucleotides (small interfering RNA: siRNA) has been reported to have a RNA interfering action in cultured cells of a vertebrate (see, for example, Non-Patent Literature 1).

siRNA is useful for identification of a gene function, screening of a cell strain suitable for production of a useful substance, control of a gene involved in disease, and the like, but is easily degraded by a ribonuclease, and therefore has the disadvantage that it is difficult to maintain activity in the body and the cost for synthesis is high. For this reason, various chemical modifications have been attempted for development of an oligonucleotide which has high stability against a ribonuclease, can be produced at low cost, and retains RNAi activity.

A phosphorothioate (PS) bond obtained by replacing a non-bridging oxygen atom of a phosphate group of a phosphodiester bond in an oligonucleotide by a sulfur atom is known to have resistance to a nuclease. However, it has been reported that an increased number of PS bonds in an oligonucleotide is not preferred because double-stranded RNA is thermodynamically destabilized and nonspecifically binds to protein (see, for example, Non-Patent Literature 2).

Attempts have been also made to obtain stable siRNA by replacing natural RNA with modified RNA. For example, many derivatives have been reported in which the 2'-OH group of RNA is alkylated to obtain a 2'-O-alkylnucleoside which does not form a substrate for RNase. The 2'-O-methylnucleotide, which is a naturally occurring modified nucleotide that is also found in tRNA, has been studied since the early days of antisense studies (see, for example, Non-Patent Literature 3).

Replacement of all nucleotides in one or both of the sense strand and the antisense strand of siRNA by 2'-O-methylnucleotides has been reported to reduce or completely eliminate RNAi activity (see, for example, Non-Patent Literatures 4, 5, 6 and 7). It has been reported that introduction of three 2'-O-methylnucleotides in a row does not decrease the activity when introduced into the sense strand, but decreases the activity when introduced into the antisense strand, and particularly significantly decreases the activity when introduced at the 5'-terminus of the sense strand (see, for example, Non-Patent Literature 8).

An oligonucleotide with a 2'-deoxy-2'-fluoronucleotide (2'-F or 2'-F RNA), which is artificially synthesized modified RNA, preferentially forms the same N-conformation as a ribonucleotide, and has increased affinity to RNA, but when the oligonucleotide has a phosphodiester bond, it does not have nuclease resistance, and therefore replacement by phosphorothioate is required to impart nuclease resistance (see, for example, Non-Patent Literature 9).

ENA (2'-O,4'-C-ethylene-bridged nucleic acid) is a modified nucleic acid having stability against a nuclease, but it has been reported that introduction of ENA into the two nucleotides of an overhang site at the 3'-terminus of one or both of the sense strand and the antisense strand of siRNA decreases the RNAi activity (see, for example, Non-Patent Literature 10).

There have been many reports on siRNA in which a plurality of modified nucleic acids are combined. For example, it has been reported that a 2'-O-methylnucleotide and 2'-F are introduced at every other nucleotide in the sense strand and antisense strand of siRNA, and RNAi activity equivalent to or higher than that of unmodified siRNA can be obtained, and relatively stably maintained in the serum (see, for example, Non-Patent Literature 11).

Double-stranded oligonucleotides in which DNA, 2'-O-methyl RNA and 2'-F are combined, in particular, siRNA in which the 14th residue from the 5'-terminus in the antisense strand is 2'-F, have been reported (see, for example, Patent Literatures 1 and 2). Double-stranded oligonucleotides in which DNA, 2'-O-methyl RNA, **2'-F** RNA and LNA (2'-O,4'-C-methylene-bridged nucleic acid) are combined, in particular, siRNA in which the 2nd or 14th residue from the 5'-terminus in the antisense strand is 2'-F, DNA or LNA, have been reported to have RNAi activity (see, for example, Patent Literatures 1 and 3).

### Citation List

### Patent Literatures

Patent Literature 1: International Patent Publication No. WO 2012/058210
Patent Literature 2: International Patent Publication No. WO 2013/074974, U.S. Patent No. 9,399,775, and U.S. Patent No. 9,796,974
Patent Literature 3: International Patent Publication No. WO 2016/028649, and U.S. Patent No. 10,612,024 Non-Patent Literatures

Non-Patent Literature 1: Nature, 2001, Vol. 411, p. 494-498
Non-Patent Literature 2: Antisense Nucleic Acid Drug Development, 2000, Vol. 10, p. 117-121
Non-Patent Literature 3: Nucleic Acids Research, 1987, Vol. 15, p.6131-6148
Non-Patent Literature 4: EMBO Journal, 2001, Vol. 20, p. 6877-6888
Non-Patent Literature 5: RNA, Vol. 9, 2003, p. 1034-1048
Non-Patent Literature 6: Biochemistry, 2003, Vol. 42, p. 7967-7975
Non-Patent Literature 7: Nucleic Acids Research, 2003, Vol. 31, p. 2705-2716
Non-Patent Literature 8: Journal of Medical Chemistry, 2005, Vol. 48, p. 4247-4253
Non-Patent Literature 9: Journal of Medical Chemistry, 1993, Vol. 36, p. 831-841
Non-Patent Literature 10: Antisense and Nucleic Acid Drug Development, 2002, Vol. 12, p.301-309
Non-Patent Literature 11: Journal of Medicinal Chemistry., 2005, Vol. 48, p. 901-904

### Summary of Invention

### Technical Problem

An object of the present invention is to provide an oligonucleotide having a novel chemical modification pattern which has a RNA interfering action and/or a gene expression suppressing action (these actions are sometimes referred to as a knockdown action). In conventional techniques, there have been many reports of possible reduction and elimination of RNA interfering action in siRNA with a modified nucleic acid. For this reason, an oligonucleotide which has a novel modification pattern, and is stable and pharmacologically active in the body to the extent of being applied to a pharmaceutical product has been desired.

### Solution to Problem

The present inventors have conducted diligent studies for obtaining an oligonucleotide which has a RNA interfering action and is stable against a ribonuclease. Consequently, the present inventors found that an oligonucleotide having an oligonucleotide unit in which DNA, LNA, ENA, 2'-O-methyl RNA, 2'-F RNA and 3'-O-methyl RNA are appropriately combined can solve the above-described problem. In this way, the present invention has been completed.

Specifically, the present invention provides the following.
[1] An oligonucleotide or a pharmaceutically acceptable salt thereof comprising a sense strand region and an antisense strand region, and having the following characteristics (a) to (g), the sense strand region consisting of an oligonucleotide represented by the following formula (I), the antisense strand region consisting of an oligonucleotide represented by the following formula (II):
   sense strand region: 5' S_{O5}-Sₐ-S₁₉-S₁₉-S₁₇-S₁₆-S₁₅-S₁₄-S₁₃-S₁₂-S₁₁-S₁₀-S₉-S₈-S₇-S₆-S₅-S₄-S₃-S₂-S₁-S_{O3} 3' (I)
   antisense strand region: 5' A₀₅-A₁-A₂-A₃-A₄-A₅-A₆-A₇-A₈-A₉-A₁₀-A₁₁-A₁₂-A₁₃-A₁₄ -A₁₅-A₁₆-A₁₇-A₁₈-A₁₉-Aₐ-A₀₃ 3' (II)

   (a) S₁ is a 3'-modified nucleoside, S₂ to S₁₉ each represents a nucleoside, and are each independently 2'-OMe RNA, 2'-F RNA or DNA, Sₐ represents 0 to 5 nucleosides, where the nucleosides each independently represent 2'-OMe RNA, 2'-F RNA or DNA, S_{O3} and S_{O5} each independently represent 0 to 5 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
   (b) A₁₁, A₁₂, A₁₃, A₁₄ and A₁₅ each represents a nucleoside, one of A₁₁, A₁₂, A₁₃, A₁₄ and A₁₅ is DNA, RNA, a 2'-O,4'-C-bridging-modified nucleoside or 2'-MOE RNA, the others are 2'-OMe RNA or 2'-F RNA, A₁ to A₁₀ and A₁₆ to A₁₉ each represents a nucleoside, and each independently represent 2'-OMe RNA, 2'-F RNA or DNA, Aₐ represents 0 to 5 nucleosides, where the nucleosides each independently represent 2'-OMe RNA, 2'-F RNA or DNA, A_{O3} and A_{O5} each independently represent 0 to 5 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
   (c) the nucleotide sequence between A₂ and Aₐ consists of a nucleotide sequence substantially complementary to a target sequence, A₁ is a nucleoside having a base complementary to a corresponding nucleoside of the target sequence, a nucleoside comprising adenine, a nucleoside comprising thymine, or a nucleoside comprising uracil, and when A₀₃ and A₀₅ are present, the nucleotide sequences thereof are each independently a nucleotide sequence selected independently of a corresponding nucleoside of the target sequence;
   (d) the nucleotide sequence between S₁ and Sₐ and the nucleotide sequence between A₁ and Aₐ are nucleotide sequences substantially complementary to each other, and form a double-stranded structure;
   (e) when both S_{O5} and A_{O3} are present, S_{O5} and A_{O3} are nucleotide sequences not complementary to each other;
   (f) when both S_{O3} and A_{O5} are present, S_{O3} and A_{O5} are nucleotide sequences not complementary to each other; and
   (g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.
[2] The oligonucleotide or pharmaceutically acceptable salt thereof according to [1], wherein in formula (II), S₁ is 3'-OMe RNA.
[3] The oligonucleotide or pharmaceutically acceptable salt thereof according to [1] or [2], wherein the sense strand region and the antisense strand region are independent oligonucleotides and form a double-stranded oligonucleotide.
[4] The oligonucleotide or pharmaceutically acceptable salt thereof according to any one of [1] to [3], wherein the 2'-O,4'-C-bridging-modified nucleoside is LNA or ENA.
[5] The oligonucleotide or pharmaceutically acceptable salt thereof according to any one of [1] to [4], wherein in formula (II), two or more of A₁₁, A₁₂, A₁₃, A₁₄ and A₁₅ are the same or different, and are DNA, RNA, a 2'-O,4'-C-bridging-modified nucleoside or 2'-MOE RNA.
[6] The oligonucleotide or pharmaceutically acceptable salt thereof according to [5], wherein in formula (II), A₁₄ is RNA or a 2'-O,4'-C-bridging-modified nucleoside.
[7] The oligonucleotide or pharmaceutically acceptable salt thereof according to [6], wherein in formula (II), A₁₃ is a 2'-O,4'-C-bridging-modified nucleoside or 2'-OMe RNA, and A₁₄ is RNA.
[8] The oligonucleotide or pharmaceutically acceptable salt thereof according to [7], wherein in formula (II), A₁₁-A₁₂-A₁₃-A₁₄-A₁₅ is one of:
   A₁₁ (2'-OMe RNA) -A₁₂ (2'-OMe RNA) -A₁₃ (ENA) -A₁₄ (RNA) -A₁₅ (2'-OMe RNA),
   A₁₁ (2'-F RNA) -A₁₂ (2'-OMe RNA) -A₁₃ (ENA) -A₁₄ (RNA) -A₁₅(2'-OMe RNA),
   A₁₁ (2'-OMe RNA) -A₁₂ (2'-F RNA) -A₁₃ (ENA) -A₁₄ (RNA) -A₁₅(2'-OMe RNA),
   A₁₁ (2'-OMe RNA) -A₁₂ (2' -OMe RNA) -A₁₃ (LNA) -A₁₄ (RNA) -A₁₅(2'-OMe RNA),
   A₁₁ (2' -F RNA) -A₁₂ (2'-OMe RNA) -A₁₃ (LNA) -A₁₄ (RNA) -A₁₅ (2'-OMe RNA),
   A₁₁ (2'-OMe RNA) -A₁₂ (2'-F RNA) -A₁₃ (LNA) -A₁₄ (RNA) -A₁₅ (2'-OMe RNA),
   A₁₁ (2'-F RNA)-A₁₂ (2'-OMe RNA)-A₁₃ (2'-OMe RNA)-A₁₄ (RNA)-A₁₅ (2'-OMe RNA), or
   A₁₁ (2'-OMe RNA)-A₁₂(2'-F RNA)-A₁₃ (2'-OMe RNA)-A₁₄ (RNA)-A₁₅ (2'-OMe RNA) .
[9] The oligonucleotide or pharmaceutically acceptable salt thereof according to [6], wherein in formula (II), A₁₂ is DNA, and A₁₄ is a 2'-O,4'-C-bridging-modified nucleoside.
[10] The oligonucleotide or pharmaceutically acceptable salt thereof according to [9], wherein in formula (II), A₁₁-A₁₂-A₁₃-A₁₄-A₁₅ is one of:
   A₁₁ (2'-F RNA) -A₁₂ (DNA)-A₁₃ (2'-OMe RNA) -A₁₄ (ENA)-A₁₅(2'-OMe RNA),
   A₁₁ (2'-OMe RNA) -A₁₂ (DNA)-A₁₃ (2'-OMe RNA) -A₁₄ (ENA)-A₁₅(2'-OMe RNA),
   A₁₁ (2'-F RNA) -A₁₂ (DNA)-A₁₃ (2'-OMe RNA) -A₁₄ (LNA)-A₁₅(2'-OMe RNA), or
   A₁₁ (2'-OMe RNA) -A₁₂ (DNA)-A₁₃ (2'-OMe RNA) -A₁₄ (LNA)-A₁₅(2'-OMe RNA).
[11] The oligonucleotide or pharmaceutically acceptable salt thereof according to any one of [1] to [10], wherein in formula (II), A₂, A₆ and A₁₆ are 2'-F RNA.
[12] The oligonucleotide or pharmaceutically acceptable salt thereof according to [11], wherein in formula (II), one or two nucleosides selected from A₈, A₉ and A₁₀ are 2'-F RNA, and the nucleosides A₁, A₃ to A₅, A₇, A₁₇ to A₁₉ and Aₐ are 2'-OMe RNA.
[13] The oligonucleotide or pharmaceutically acceptable salt thereof according to [12], wherein in formula (II), A₂, A₆, A₈, A₁₀ and A₁₆ are 2'-F RNA, and the nucleosides A₁, A₃ to A₅, A₇, A₈, A₁₇ to A₁₉ and Aₐ are 2'-OMe RNA.
[14] The oligonucleotide or pharmaceutically acceptable salt thereof according to any one of [1] to [13], wherein in formula (I), S₁₁, S₁₂, S₁₃ and S₁₅ are 2'-F RNA.
[15] The oligonucleotide or pharmaceutically acceptable salt thereof according to [14], wherein in formula (I), S₂ to S₁₀, S₁₄, S₁₆ to S₁₉ and Sₐ are 2'-OMe RNA.
[16] The oligonucleotide or pharmaceutically acceptable salt thereof according to any one of [1] to [15], wherein when RNA is used in formula (II), the bond between the RNA and a 3' adjacent nucleoside is a phosphorothioate bond.
[17] The oligonucleotide or pharmaceutically acceptable salt thereof according to any one of [1] to [16], wherein in 2 to 5 nucleotides from each of the 5'-terminus and the 3'-terminus of the oligonucleotide, each inter-nucleoside bond is a phosphorothioate bond.
[18] The oligonucleotide or pharmaceutically acceptable salt thereof according to any one of [1] to [17], wherein the number of nucleosides in each of S_{O3}, S_{O5}, A_{O5} and A_{O3} is 0 to 2.
[19] The oligonucleotide or pharmaceutically acceptable salt thereof according to [18], wherein the number of nucleosides in S_{O3} is 0.
[20] The oligonucleotide or pharmaceutically acceptable salt thereof according to [19], wherein the number of nucleosides in each of S_{O3}, S_{O5} and A_{O5} is 0, and the number of nucleosides in A_{O3} is 2.
[21] The oligonucleotide or pharmaceutically acceptable salt thereof according to any one of [1] to [20], wherein a chemical modification at the 5'-position of the nucleoside at the 5'-terminus and/or the 3'-position of the nucleoside at the 3'-terminus of the oligonucleotide, or the 2'-position thereof in the case where the nucleoside at the 3'-terminus is a 3'-modified nucleoside is a modification with a unit for delivery to an intended tissue.
[22] The oligonucleotide or pharmaceutically acceptable salt thereof according to [21], wherein the unit for delivery to an intended tissue is a GalNAc unit or a fatty acid unit.
[23] The oligonucleotide or pharmaceutically acceptable salt thereof according to [22], wherein the GalNAc unit is a unit represented by: 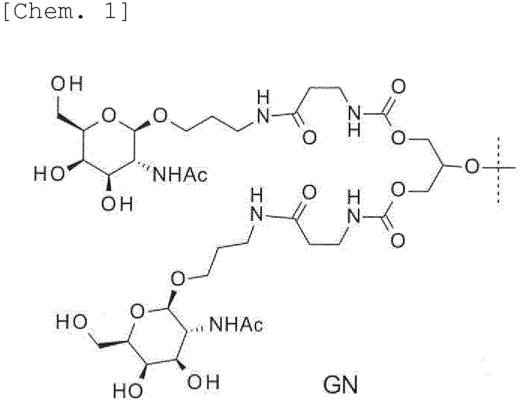 wherein the broken line represents a phosphodiester bond formed with a phosphate group at the 5'-terminus and/or a phosphate group at the 3'-terminus (a phosphate group at the 2'-position in the case of a nucleotide modified at the 3'-position) of an adjacent nucleotide.

### Advantageous Effects of Invention

The present invention provides an oligonucleotide with a novel modification pattern which has a sustained RNA interfering action and/or a gene expression suppressing action in an animal body. The modification pattern has been applied to oligonucleotides for a plurality of target sequences, and confirmed to exhibit the same effect. Thus, it is possible to analyze the functions of various genes using the oligonucleotide, and to provide pharmaceutical products for various diseases which contain the oligonucleotide.

### Brief Description of Drawings

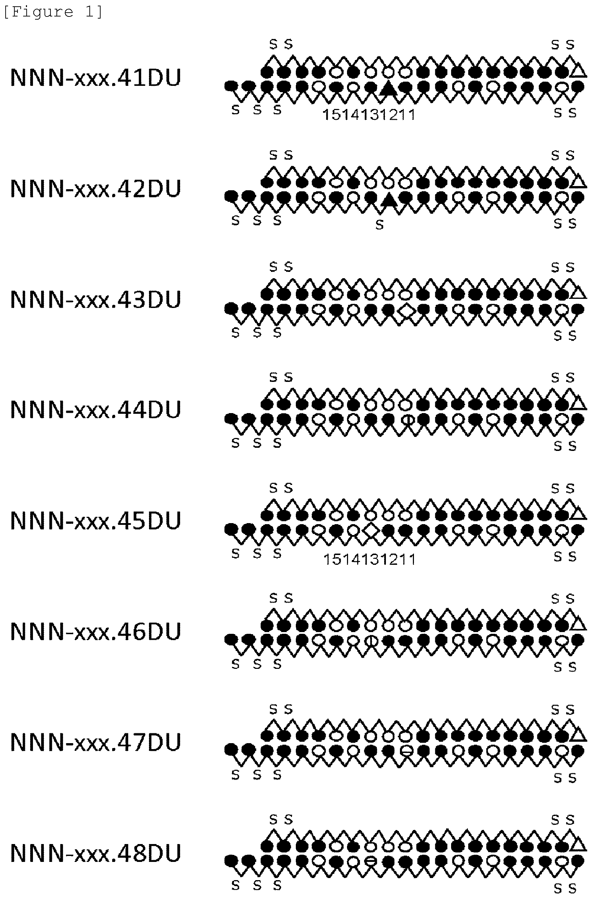

[Figure 1] Figure 1 shows modification patterns of double-stranded siRNA. In the figure, the black circle represents 2'-O-methyl RNA, the white circle represents 2'-deoxy-2'-fluoro RNA, the black triangle represents DNA, the white triangle represents 3'-O-methyl RNA, the white diamond represents ENA, the black diamond represents RNA, the white circle with a vertical bar represents LNA, the white circle with a horizontal bar represents 2'-O-methoxyethyl RNA, and the line connecting marks represents a phosphodiester bond, where the line connecting marks represents a phosphorothioate bond when given "S". In the figure, the upper strand represents a sense strand (or a passenger strand), the left terminus of the sense strand represents a 5'-terminus, and the right terminus of the sense strand represents a 3'-terminus. The lower strand represents an antisense strand (or a guide strand), the right terminus of the antisense strand represents a 5'-terminus, and the left terminus of the antisense strand represents a 3'-terminus. Each siRNA is represented by NNN-xxx, and the numbers and alphabets written after NNN-xxx indicate an abbreviation of each of the modification patterns shown in Figures 1 to 6.
[Figure 2] Figure 2 shows modification patterns of double-stranded siRNA in which A₁₄ of the antisense strand is DNA or RNA. The symbols, arrangements, modifications and the like are shown in the same form as in Figure 1.
[Figure 3] Figure 3 shows modification patterns of double-stranded siRNA in which A₈ and A₉ of the antisense strand are 2'-deoxy-2'-fluoro RNA. The symbols, arrangements, modifications and the like are shown in the same form as in Figure 1.
[Figure 4] Figure 4 shows modification patterns of double-stranded siRNA in which A₁₄ of the antisense strand is ENA. The symbols, arrangements, modifications and the like are shown in the same form as in Figure 1.
[Figure 5] Figure 5 shows modification patterns of double-stranded siRNA in which A₈ and A₉ of the antisense strand are 2'-deoxy-2'-fluoro RNA and A₁₃ of the antisense strand is ENA. The symbols, arrangements, modifications and the like are shown in the same form as in Figure 1.
[Figure 6] Figure 6 shows modification patterns of double-stranded siRNA in which A₈ of the antisense strand is 2'-O-methyl RNA and A₉ of the antisense strand is 2'-deoxy-2'-fluoro RNA. The symbols, arrangements, modifications and the like are shown in the same form as in Figure 1.
[Figure 7] Figure 7 shows the change of the iron concentration in plasma after treatment with GalNAc-conjugated TfR2 siRNA in normal mice.

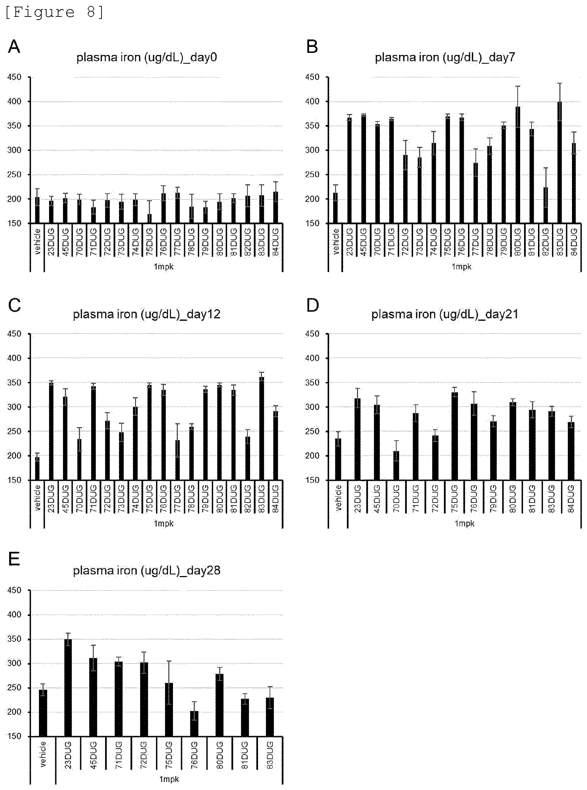

[Figure 8] Figure 8A shows the iron concentration in plasma before treatment with GalNAc-conjugated TfR2 siRNA in normal mice (Day 0). Figure 8B shows the iron concentration in plasma 7 days after treatment with GalNAc-conjugated TfR2 siRNA in normal mice (Day 7). Figure 8C shows the iron concentration in plasma 12 days after treatment with GalNAc-conjugated TfR2 siRNA in normal mice (Day 12). Figure 8D shows the iron concentration in plasma 21 days after treatment with GalNAc-conjugated TfR2 siRNA in normal mice (Day 21). Figure 8E shows the iron concentration in plasma 28 days after treatment with GalNAc-conjugated TfR2 siRNA in normal mice (Day 28).

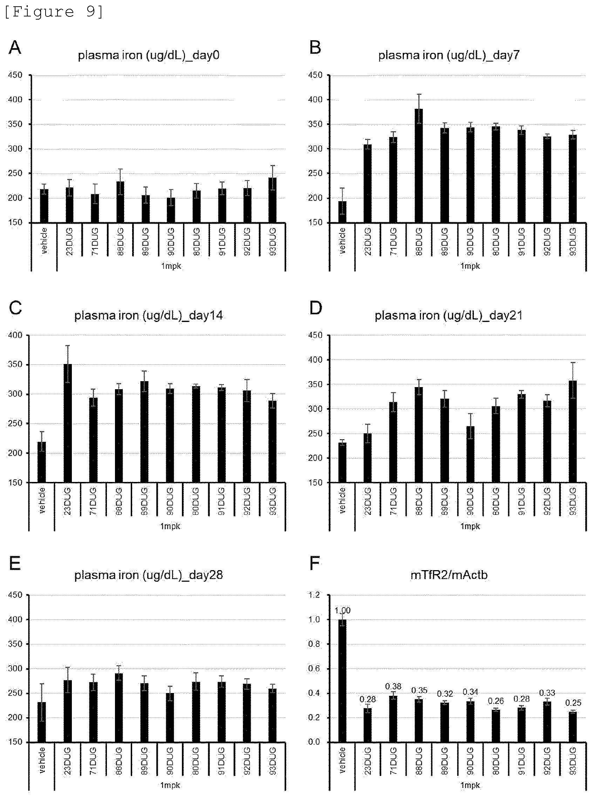

[Figure 9] Figure 9A shows the iron concentration in plasma before treatment with GalNAc-conjugated TfR2 siRNA in normal mice (Day 0). Figure 9B shows the iron concentration in plasma 7 days after treatment with GalNAc-conjugated TfR2 siRNA in normal mice (Day 7). Figure 9C shows the iron concentration in plasma 14 days after treatment with GalNAc-conjugated TfR2 siRNA in normal mice (Day 14). Figure 9D shows the iron concentration in plasma 21 days after treatment with GalNAc-conjugated TfR2 siRNA in normal mice (Day 21). Figure 9E shows the iron concentration in plasma 28 days after treatment with GalNAc-conjugated TfR2 siRNA in normal mice (Day 28). Figure 9F shows the level of expression of mTfR2 mRNA in the liver 28 days after treatment with GalNAc-conjugated TfR2 siRNA in normal mice.

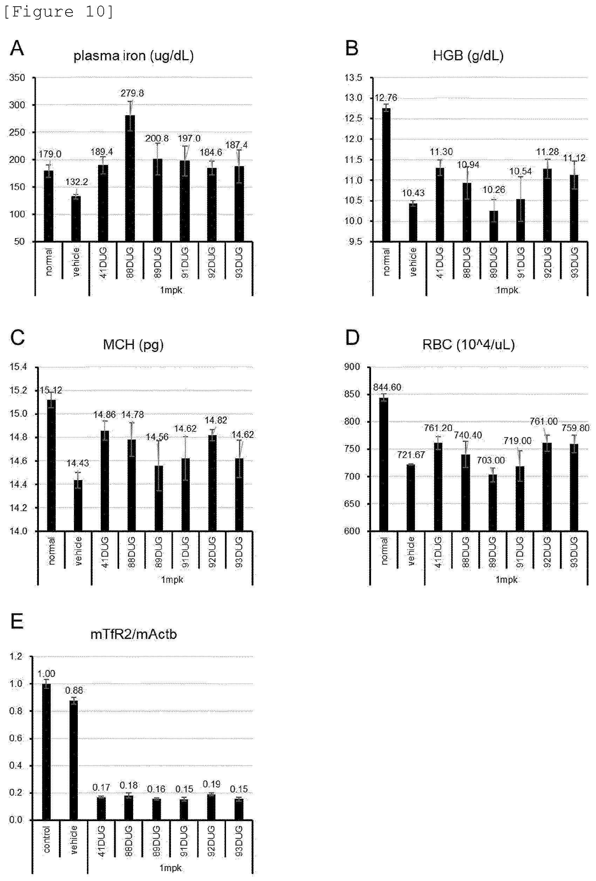

[Figure 10] Figure 10A shows the iron concentration in plasma after treatment with GalNAc-conjugated TfR2 siRNA in models of anemia associated with inflammation. Figure 10B shows the HGB concentration after treatment with GalNAc-conjugated TfR2 siRNA in models of anemia associated with inflammation. Figure 10C shows the MCH level after treatment with GalNAc-conjugated TfR2 siRNA in models of anemia associated with inflammation. Figure 10D shows the RBC number after treatment with GalNAc-conjugated TfR2 siRNA in models of anemia associated with inflammation. Figure 10E shows the level of expression of mTfR2 mRNA in the liver after treatment with GalNAc-conjugated TfR2 siRNA in models of anemia associated with inflammation.
[Figure 11] Figure 11 shows the hTfR2 knockdown activity of siRNA in Hep G2 cells.

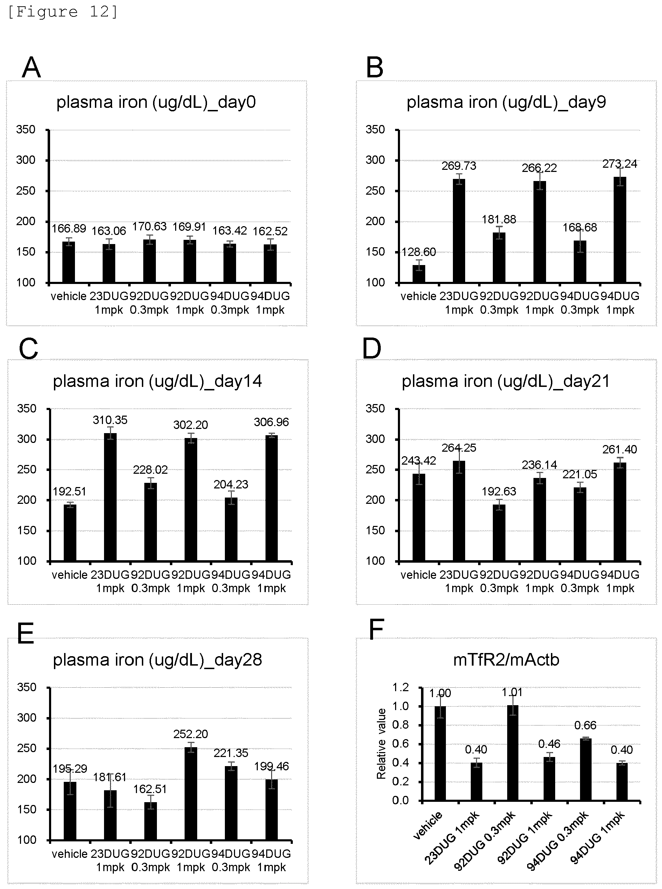

[Figure 12] Figure 12A shows the iron concentration in plasma before treatment with GalNAc-conjugated TfR2 siRNA in normal mice (Day 0). Figure 12B shows the iron concentration in plasma 9 days after treatment with GalNAc-conjugated TfR2 siRNA in normal mice (Day 9). Figure 12C shows the iron concentration in plasma 14 days after treatment with GalNAc-conjugated TfR2 siRNA in normal mice (Day 14). Figure 12D shows the iron concentration in plasma 21 days after treatment with GalNAc-conjugated TfR2 siRNA in normal mice (Day 21). Figure 12E shows the iron concentration in plasma 28 days after treatment with GalNAc-conjugated TfR2 siRNA in normal mice (Day 28). Figure 12F shows the level of expression of mTfR2 mRNA in the liver 28 days after treatment with GalNAc-conjugated TfR2 siRNA in normal mice.
[Figure 13] Figure 13 shows the level of expression of hTfR2 mRNA in the liver 7 days after treatment with GalNAc-conjugated TfR2 siRNA in hTfR2 Tg mice.

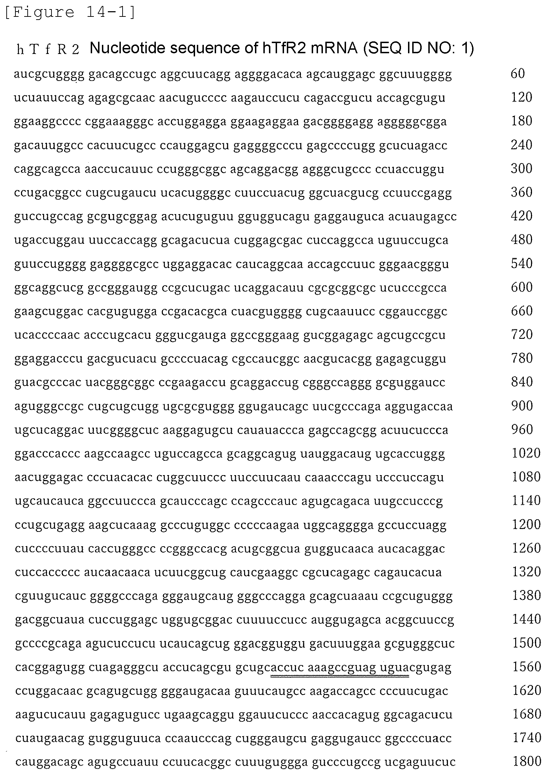

[Figure 14-1] Figure 14-1 shows the nucleotide sequence of mRNA of a gene encoding human transferrin receptor 2 (hTfR2) (SEQ ID NO: 1). The underlined sequence (the sequence from positions 2847 to 2865) indicates a sequence targeted by TfR2-019, and the double-underlined sequence (the sequence from positions 1536 to 1554) indicates a sequence targeted by TfR2-039.

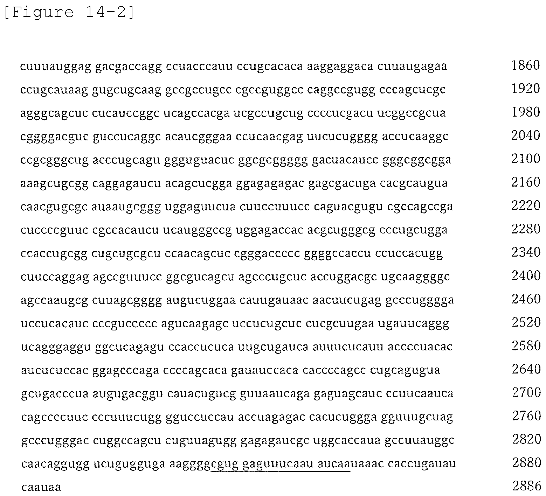

[Figure 14-2] Figure 14-2 shows a continuation of Figure 14-1.

### Description of Embodiments

### <1. Description of terms>

In the present specification, the "target gene" is not limited as long as it is RNA in cells, tissues or individuals into which the target gene is introduced (hereinafter, sometimes referred to as a "body to be subjected to introduction"). It may be mRNA that is translated into protein, or non-coding RNA that is not translated into protein. Examples of the non-coding RNA include functional RNA, for example, non-translated regions of mRNA, tRNA, rRNA, mRNA-like non-coding RNA (mRNA-type ncRNA), long non-coding RNA (long ncRNA), small nuclear RNA (snRNA), small nucleolar RNA (snoRNA), and micro-RNA (miRNA). Specifically, the target gene may be a gene that is inherent in a body to be subjected to introduction of the gene, or an exogeneous gene that is introduced by a method such as gene introduction. The target gene may be a gene that is present on a chromosome, or a gene that is outside a chromosome. Examples of the exogeneous gene include, but are not limited to, those derived from viruses, bacteria, fungi or protozoans that can be transmitted to a body to be subjected to introduction. The function of the gene may be known or unknown.

Examples of the target gene can include genes whose expression is specifically increased and/or which are specifically varied in patients having a specific disease. Examples of the disease can include central nerve diseases (for example, Alzheimer disease, dementia, and eating disorders), inflammatory disease (for example, allergies, rheumatism, osteoarthritis, and lupus erythematosus), cardiovascular diseases (for example, hypertension, cardiac enlargement, angina, arteriosclerosis, and hypercholesterolemia), cancers (for example, non-small cell lung cancer, ovarian cancer, prostate cancer, stomach cancer, pancreas cancer, liver cancer, bladder cancer, breast cancer, uterine cervix cancer, bowel cancer, colon cancer, and rectum cancer), respiratory diseases (for example, pneumonia, bronchitis, asthma, and chronic obstructive lung disease), diabetes, diabetic retinopathy, diabetic nephropathy, anemia (for example, anemia associated with chronic, and sideroachrestic iron-deficiency anemia), age-related macular degeneration, immune system diseases (for example, Crohn's disease, atopic dermatitis, autoimmune disease, immunodeficiency, and leukemia), liver/gallbladder diseases (for example, non-alcoholic steatohepatitis, liver cirrhosis, hepatitis, liver failure, cholestasis, and calculus), digestive tract diseases (for example, ulcer, enteritis, and malabsorption), infection, obesity, and fibrosis (lung fibrosis, liver fibrosis, kidney fibrosis, and bone-marrow fibrosis). Examples of a gene responsible for the diseases can include, but are not limited to, kinesin spindle protein (KSP), vascular endothelial growth factor, (VEGF), transthyretin (TTR), proprotein convertase subtilisn/kexin type 9(PCSK9), polo-like kinase(PLK), ApoB-100, ribonucleotide reductase M2 subunit (RRM2), clusterin, heat shock protein 27 (Hsp27), survivin, eukaryotic initiation factor-4E (eIF-4E), intercellular adhesion molecule 1(ICAM-1), alpha subunit of the interleukin 4 receptor (IL-4R-alpha), Factor XI, Factor VII, N-ras, H-ras, K-ras, bcl-2, bcl-xL, Her-1, Her-2, Her-3, Her-4, MDR-1, human β-catenin genes, DDX3 (DEAD (Asp-Glu-Ala-Asp) box polypeptide 3, X-linked), Myeloid Cell Leukemia Sequence 1 (MCL1) genes, PKR (Eif2ak2), Hsp 47 (Serpinh 1), Hepcidin, activated protein C (APC), survivin, signal transducer, and activator of transcription (STAT 3).

In the present specification, the nucleic acid or nucleic acid molecule is a general term for nucleosides, nucleotides, oligonucleotides and polynucleotides.

The term "nucleoside" means a chemical structure moiety in which a base moiety important for genetic information and a sugar moiety important for physicochemical properties of the molecule are bound.

The term "nucleotide" refers to a compound in which the hydroxyl group at the 3'-position (2'-position in the case of modification at the 3'-position) of a sugar moiety of a nucleoside forms an ester with a phosphate group.

The term "oligonucleotide" refers to an oligomer having a structure in which there are two or more nucleotides, and the phosphate group moiety of one of the nucleotides and the hydroxyl group at the 5'-position of the sugar moiety of another nucleotide are bound (phosphodiester bond). However, the residues at the 3'-position (2'-position in the case of a nucleotide modified at the 3'-position) of the nucleotide at the 3'-terminus and/or the 5'-position of the nucleotide at the 5'-terminus may be hydroxyl groups or phosphate groups, and these groups may be chemically modified. The phosphodiester bond between the nucleosides may be chemically modified. In the present specification, the term "oligonucleotide" and the term "polynucleotide" are used interchangeably with each other.

The nucleobase is adenine (A), guanine (G), cytosine (C), uracil (U) or thymine (T), A and U or T, G and C, respectively, forms Watson-Crick base pairs through hydrogen bonding, and the relationship between bases which form a base pair is referred to as "complementary". Each base moiety may be chemically modified, and the modified base may be identical in its base pair forming ability to the original base. As typical modified bases, 5-methylcytosine (5C), 5-methyluracil (5U) and the like are known. 5U is identical in structure to T. In the sequence listing attached to the present specification, 5C is represented as C, and 5U is represented as T or U.

In the present specification, "symbols for bases (symbols for sugar moieties)" may be used in the notation for nucleic acids. The symbols for sugar moieties will be described in the following sections where various nucleosides are described.

In the present specification, as long as no specific indication is given, the term "modification pattern" of the oligonucleotide means an arrangement of modification forms of sugar moieties of nucleosides forming an oligonucleotide, independently of the arrangement of bases. If a specific indication is given, this term means a modification pattern further combined with an inter-nucleoside bond form. Since the structure of the sugar moiety and the inter-nucleoside bond are elements that do not depend on the target sequence, and influence the stability of molecules, and bond performance associated with the stability, a modification pattern confirmed to have an action and effect on a target sequence is likely to exhibit the same action and effect on another target sequence (see, for example, Mol Ther. (2018) 26: 708-717).

In the present specification, the term "natural nucleoside" refers to a 2'-deoxynucleoside or a ribonucleoside. The 2'-deoxynucleoside (also referred to as "DNA"; the symbol representing a sugar moiety is (D), and may be expressed by a lowercase mark for a base moiety) corresponding to each base has a structure represented by one of the following formulae. 2'-deoxyadenosine may be represented as A(D) or a, 2'-deoxyguanosine may be represented as G(D) or g, 2'-deoxycytidine may be represented as C(D) or c, thymidine may be represented as T(D) or t, 2'-deoxy-5-methylcytidine may be represented as 5C(D) or 5c, and 2'-deoxyuridine may be represented as U(D) or u. A 2-deoxynucleoside whose base is not specified may be represented as N(D). 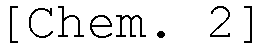 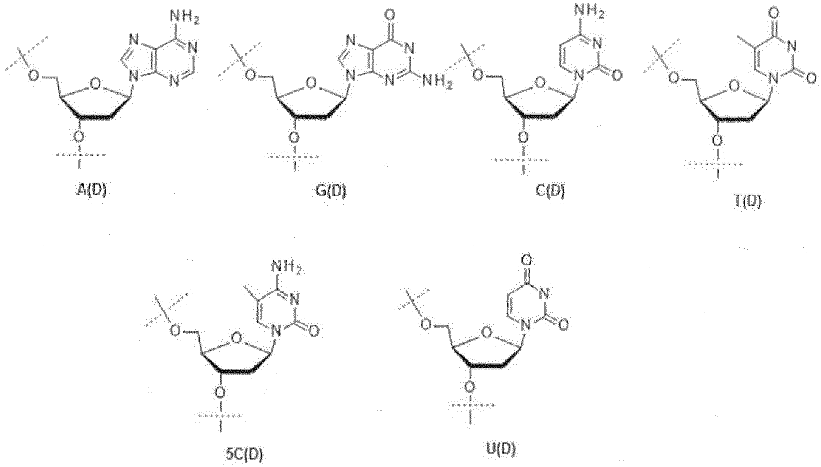 wherein the broken line indicates a point of attachment.

The ribonucleoside (also referred to as "RNA"; the symbol representing a sugar moiety is (R), and may be represented as a capital indicating a base moiety) corresponding to each base has a structure represented by one of the following formulae. Adenosine may be represented as A(R) or A, guanosine may be represented as G(R) or G, cytidine may be represented as C(R) or C, and uridine may be represented as U(R) or U. A ribonucleoside whose base is not specified may be represented as N(R). 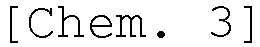 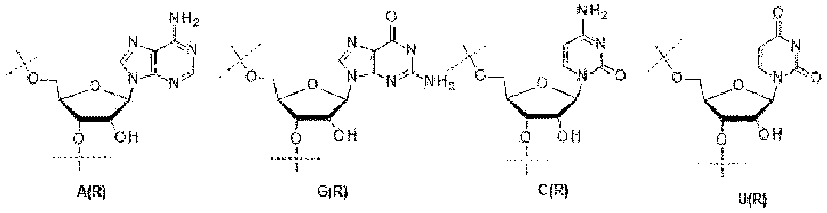 wherein the broken line indicates a point of attachment.

In the present specification, the terms "modified nucleoside" and "modified nucleotide" mean a nucleoside or a nucleotide containing at least one chemically modified structure in the base moiety, the sugar moiety or the phosphodiester moiety of a natural nucleoside.

In the present specification, the term "phosphodiester bond that may be chemically modified" means a phosphodiester bond used as a natural inter-nucleoside bond, or a chemically modified phosphodiester bond. The natural inter-nucleoside bond is a phosphodiester bond represented by the following formula (P), and is shown with no character and symbol between nucleosides in the display of a nucleotide sequence. In the present specification, the term "chemically modified phosphodiester bond" means a bond form in which the phosphorus atom contained in the phosphodiester bond is chemically modified. Examples of the chemically modified phosphodiester bond include a phosphorothioate bond represented by the following formula (PS), a phosphoramidate bond that is a modified bond in which a nitrogen atom is added to a phosphorus atom, an alkyl phosphonate bond that is a modified bond in which a replaceable alkyl group is added to a phosphorus atom (for example, a methyl phosphonate bond when the alkyl group is a methyl group), and a phosphotriester bond that is a modified bond in which a replaceable alkyl group is added to an oxygen atom of a noncovalent bond of a phosphate group. When a phosphorothioate bond is adopted, "^" is shown between nucleosides in the display of a nucleotide sequence. 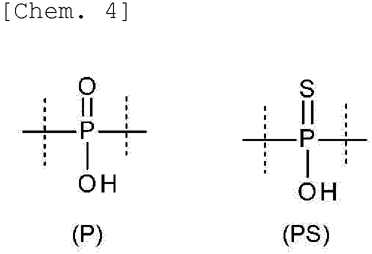 wherein the broken line indicates a point of attachment, the point of attachment on one side represents an ester bond formed with the oxygen atom at the 3'-hydroxyl group (oxygen atom at the 2'-position in the case of a nucleoside modified at the 3'-position) of a 5' adjacent nucleoside, or the adjacent structural unit, and the point of attachment on the other side represents an ester bond formed with the oxygen atom of the 5'-hydroxyl group of a 3' adjacent nucleoside, or the structural unit.

In the present specification, the term "sugar-modified nucleoside" refers to a nucleoside in which the sugar moiety of the nucleoside is modified. The sugar-modified nucleoside includes all forms of sugar modification that are known in the technical field to which the present invention belongs. Examples of sugar-modified nucleosides include 2'-modified nucleosides, 3'-modified nucleosides, 4'-thio-modified nucleosides, 4'-thio-2'-modified nucleosides, and 2'-O,4'-C-bridged modified nucleosides. Examples of preferred sugar modifications are 2'-O-alkylation (methylation, ethylation, propylation, isopropylation, butylation and the like), 2'-O-alkoxyalkylation (methoxyethylation, methoxypropylation, methoxybutylation and the like), 2'-halogenation (chlorination, fluorination and the like), 3'-O-alkylation (methylation, ethylation, propylation, isopropylation, butylation and the like), 3'-O-alkoxyalkylation (methoxyethylation, methoxypropylation, methoxybutylation and the like), 3'-halogenation (chlorination, fluorination and the like), 2'-O,4'-C-bridging (alkylene-bridging and the like), and the like.

In the present specification, the term "2'-modified nucleoside" refers to a nucleoside in which ribofuranose contained in the nucleoside is chemically modified at the 2'-position. Examples of a relevant modification are 2'-O-alkylated (for example, methylated, ethylated, propylated, isopropylated and butylated) nucleosides, 2'-O-alkoxyalkylated (for example, methoxyethylated, methoxypropylated and methoxybutyl) nucleosides, 2'-halogenated (for example, chlorinated and fluorinated) nucleosides, 2'-allylated nucleosides, 2'-aminated nucleosides, 2'-azidated nucleosides, 2'-O-allylated nucleosides, 2'-OCF₃ nucleosides, 2'-O(CH₂)₂SCH₃ nucleosides, 2'-O-(CH₂)₂-O-N-(Rₘ) (Rₙ) nucleosides, or 2'-O-CH₂-C(=O)-N(Rₘ) (Rₙ) nucleosides (each Rₘ and Rₙ are independently H, an amino protective group, or a substituted or unsubstituted C₁-C₁₀ alkyl). The preferred 2'-modified nucleoside is a 2'-O-alkylated nucleoside, a 2'-O-alkoxyalkylated nucleoside, or a 2'-halogenated nucleoside.

Examples of the 2'-O-alkylation modification for a sugar-modified nucleoside include 2'-O-methylnucleoside (sometimes referred to as "2'-OMe RNA" or "2'-O-methyl RNA"; the symbol representing a sugar moiety is (M)). The 2'-O-methylnucleoside corresponding to each base has a structure represented by one of the following formulae: 2'-O-methyladenosine may be represented as A(M), 2'-O-methylguanosine may be represented as G(M), 2'-O-methylcytidine may be represented as C(M), 2'-O-methyl-5-methylcytidine may be represented as 5C(M), 2'-O-methyluridine may be represented as U(M), and 2'-O-methyl-5-methyluridine may be represented as T(M). A 2'-O-methylnucleoside whose base is not specified may be represented as N(M). 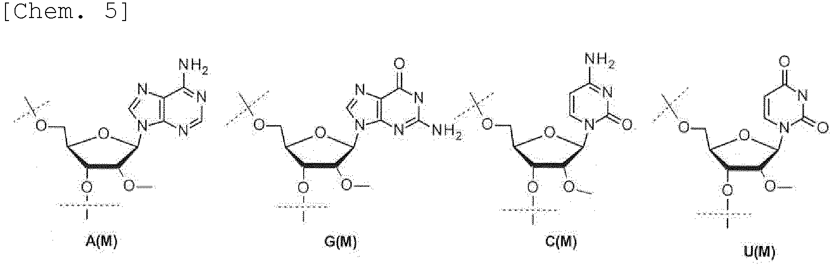 wherein the broken line indicates a point of attachment.

Examples of a 2'-O-alkoxyalkylation modification include 2'-O-methoxyethylnucleosides (sometimes referred to as "2'-MOE RNA", "2'-O-methoxyethyl RNA" or "2'-methoxyethoxy RNA"; the symbol representing a sugar moiety is (m)). The 2'-O-methoxyethylnucleoside corresponding to each base has a structure represented by one of the following formulae: 2'-O-methoxyethyladenosine may be represented as A(m), 2'-O-methoxyethylguanosine may be represented as G(m), 2'-O-methoxyethyl-5-methylcytidine may be represented as C(m) (written as C(m) for convenience although the structure of the base is 5C; 2'-O-methoxyethylcytidine can also be used in replacement of the 2'-O-methoxyethyl-5-methylcytidine), 2'-O-methoxyethyluridine may be represented as U(m), and 2'-O-methoxyethyl-5-methyluridine may be represented as T(m). A 2'-O-methoxyethylnucleoside whose base is not specified may be represented as N(m). 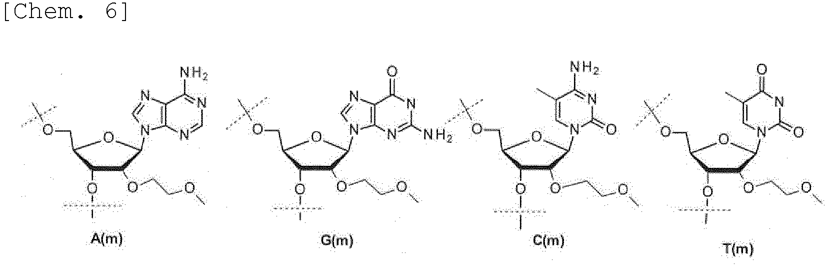 wherein the broken line indicates a point of attachment.

Examples of a 2'-halogenation modification include 2'-deoxy-2'-fluoronucleosides ("sometimes referred to as "2'-F RNA" or "2'-deoxy-2'-fluoro RNA"; the symbol representing a sugar moiety is (F)). The 2'-deoxy-2'-fluoronucleoside corresponding to each base has a structure represented by one of the following formulae: 2'-deoxy-2'-fluoroadenosine may be represented as A(F), 2'-deoxy-2'-fluoroguanosine may be represented as G(F), 2'-deoxy-2'-fluorocytidine may be represented as C(F), 2'-deoxy-2'-fluoro-5-methylcytidine may be represented as 5mC(F), 2'-deoxy-2'-fluorouridine may be represented as U(F), and 2'-deoxy-2'-fluoro-5-methyluridine may be represented as T(F). A 2'-deoxy-2'-fluoronucleoside whose base is not specified may be represented as N(F). 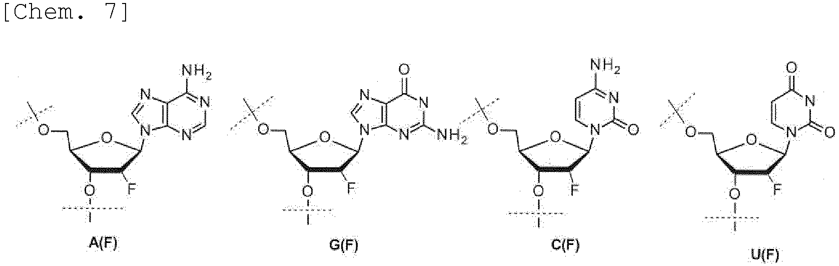 wherein the broken line indicates a point of attachment.

In the present specification, the term "3'-modified nucleoside" refers to a nucleoside in which ribofuranose contained in the nucleoside is chemically modified at the 3'-position. The hydroxyl group at the 2'-position in the 3'-modified nucleoside may be modified. Examples of the relevant modification are 3'-O-alkylated (for example, methylated, ethylated, propylated, isopropylated and butylated) nucleosides, 3'-O-alkoxyalkylated (for example, methoxyethylated, methoxypropylated and methoxybutyl) nucleosides, 3'-halogenated (for example, chlorinated and fluorinated) nucleosides, 3'-allylated nucleosides, 3'-aminated nucleosides, 3'-azidated nucleosides, 3'-O-allylated nucleosides, 3'-OCF₃ nucleosides, 3'-O(CH₂)₂SCH₃ nucleosides, 3'-O-(CH₂)₂-O-N-(Rₘ) (Rₙ) nucleosides, or 3'-O-CH₂-C(=O)-N(Rₘ) (Rₙ) nucleosides (each Rₘ and Rₙ are independently H, an amino protective group, or a substituted or unsubstituted C₁-C₁₀ alkyl). The preferred 3'-modified nucleoside is a 3'-O-alkylated nucleoside, a 3'-O-alkoxyalkylated nucleoside, or a 3'-halogenated nucleoside.

Examples of a 3'-O-alkylation modification for the 3'-modified nucleoside include 3'-O-methylnucleoside (sometimes referred to as "3'-OMe RNA"; the symbol representing a sugar moiety is (3M)). The 3'-O-methylnucleoside corresponding to each base has a structure represented by one of the following formulae: 3'-O-methyladenosine may be represented as A(3M), 3'-O-methylguanosine may be represented as G(3M), 3'-O-methylcytidine may be represented as C(3M), 3'-O-methyl-5-methylcytidine may be represented as 5mC(3M), 3'-O-methyluridine may be represented as U(3M), and 3'-O-methyl-5- methyluridine may be represented as T(3M). A 3'-O-methylnucleoside whose base is not specified may be represented as N(3M). 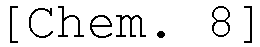 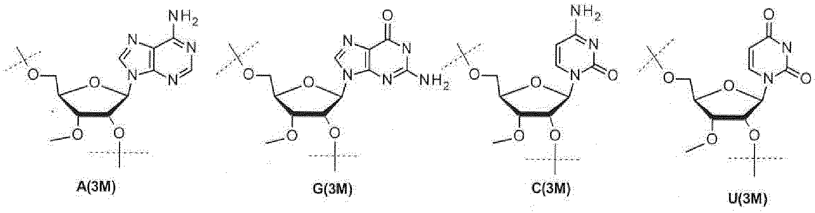 wherein the broken line indicates a point of attachment.

Examples of a 3'-O-alkoxyalkylation modification include 3'-O-methoxyethylnucleosides (sometimes referred to as "3'-MOE RNA", "3'-O-methoxyethyl RNA" or "3'-methoxyethoxy RNA"; the symbol representing a sugar moiety is (3m)). The 3'-O-methoxyethylnucleoside corresponding to each base has a structure in which a methoxyethyl group is bonded in place of the methyl group bonded to the oxygen atom at the 3'-position in the structural formula of the 3'-O-methylnucleoside corresponding to each base. For the 3'-O-methoxyethylnucleoside corresponding to each base, 3'-O-methoxyethyladenosine may be represented as A(3m), 3'-O-methoxyethylguanosine may be represented as G(3m), 3'-O-methoxyethyl-5-methylcytidine may represented as C(3m) (written as C(3m) for convenience although the structure of the base is 5C; 3'-O-methoxyethylcytidine can also be used in replacement of the 3'-O-methoxyethyl-5-methylcytidine), 3'-O-methoxyethyluridine may be represented as U(3m), and 3'-O-methoxyethyl-5-methyluridine may be represented as T(3m). A 3'-O-methoxyethylnucleoside whose base is not specified may be represented as N(3m).

Examples of a 3'-halogenation modification include 3'-deoxy-3'-fluoronucleosides (sometimes referred to as "3'-F RNA" or "3'-deoxy-3'-fluoro RNA"; the symbol representing a sugar moiety is (3F)). The 3'-deoxy-3'-fluoronucleoside corresponding to each base has a structure in which a fluorine atom is bonded in place of the methoxy group bonded to the carbon atom at the 3'-position in the structural formula of the 3'-O-methylnucleoside corresponding to each base. For the 3'-deoxy-3'-fluoronucleoside corresponding to each base, 3'-deoxy-3'-fluoroadenosine may be represented as A(3F), 3'-deoxy-3'-fluoroguanosine may be represented as G(3F), 3'-deoxy-3'-fluorocytidine may be represented as C(3F), 3'-deoxy-3'-fluoro-5-methylcytidine may be represented as 5mC(3F), 3'-deoxy-3'-fluorouridine may be represented as U(3F), and 3'-deoxy-3'-fluoro-5-methyluridine may be represented as T(3F). A 3'-deoxy-3'-fluoronucleoside whose base is not specified may be represented as N(3F).

In the present specification, the term "4'-thio-modified nucleoside" refers to a nucleoside in which the oxygen atom at the 4'-position of a ribofuranose contained in the nucleoside is replaced by a sulfur atom. Examples of the 4'-thio-modified nucleoside can include β-D-ribonucleosides in which the 4'-oxygen atom is replaced by a sulfur atom (Hoshika, S. et al. FEBS Lett. 579, p.3115-3118, (2005); Dande, P. et al. J. Med. Chem. 49, p.1624-1634 (2006); Hoshika, S. et al. ChemBioChem. 8, p.2133-2138, (2007)).

In the present specification, the term "4'-thio-2'-modified nucleoside" refers to a nucleoside in which a ribofuranose contained in the nucleoside is chemically modified at the 2'-position, and the oxygen atom at the 4'-position of the ribofuranose is replaced by a sulfur atom. Examples of the 4'-thio-2'-modified nucleoside can include 4'-thio-2'-modified nucleosides holding 2'-H or 2'-O-methyl (Matsugami, et al. Nucleic Acids Res. 36, 1805 (2008)).

Examples of a 2'-O,4'-C-bridging modification for the sugar-modified nucleoside include 2'-O,4'-C-ethylene-bridged nucleosides (sometimes referred to as "ENA" or an "ENA unit"; the symbol representing a sugar moiety is (E)) (Morita, K. et al. Bioorg. Med. Chem. Lett., 12, p.73 (2002).; Morita, K. et al. Bioorg. Med. Chem., 11, p.2211 (2003).). The 2'-O,4'-C-ethylene-bridged nucleoside corresponding to each base has a structure represented by one of the following formulae: 2'-O,4'-C-ethylene-bridged adenosine may be represented as A(E), 2'-O,4'-C-ethylene-bridged guanosine may be represented as G(E), 2'-O,4'-C-ethylene-bridged-5-methylcytidine may be represented as C(E) (written as C(E) for convenience although the structure of the base is 5C; 2'-O,4'-C-ethylene-bridged cytidine can also be used in replacement of the 2'-O,4'-C-ethylene-bridged-5-methylcytidine), 2'-O,4'-C-ethylene-bridged uridine may be represented as U(E), and 2'-O,4'-C-ethylene-bridged-5-methyluridine may be represented as T(E). A 2'-O,4'-C-ethylene-bridged nucleoside whose base is not specified may be represented as N(E). 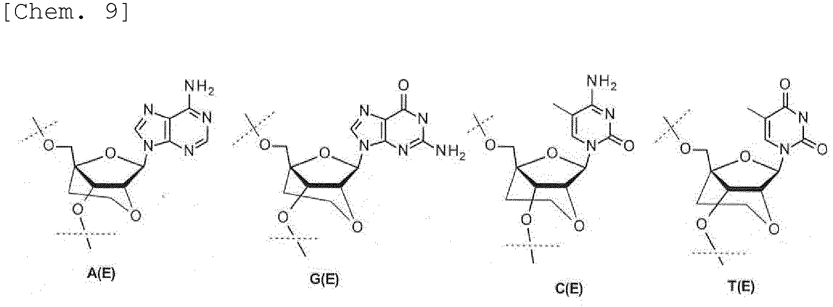 wherein the broken line indicates a point of attachment.

Another example of a 2'-O,4'-C-bridging modification is, for example, a 2'-O,4'-C-methylene-bridged nucleoside (sometimes referred to as "LNA" or a "LNA unit"; the symbol representing a sugar moiety is (L)) (Obika, S. et al. Tetrahedron Lett., 38, p.8735- (1997).; Obika, S. et al., Tetrahedron Lett., 39, p.5401- (1998).; A. A. Koshkin, A. A. et al. Tetrahedron, 54, p.3607 (1998).; Obika, S. Bioorg. Med. Chem., 9, p.1001 (2001).). The 2'-O,4'-C-methylene-bridged nucleoside corresponding to each base has a structure represented by one of the following formulae: 2'-O,4'-C-methylene-bridged adenosine may be represented as A(L), 2'-O,4'-C-methylene-bridged guanosine may be represented as G(L), 2'-O,4'-C-methylene-bridged-5-methylcytidine may be represented as C(L) (written as C(L) for convenience although the structure of the base is 5C; 2'-O,4'-C-methylene-bridged cytidine can also be used in replacement of the 2'-O,4'-C-methylene-bridged-5-methylcytidine), 2'-O,4'-C-methylene-bridged uridine may be represented as U(L), and 2'-O,4'-C-methylene-bridged-5-methyluridine may be represented as T(L). A 2'-O,4'-C-methylene-bridged nucleoside whose base is not specified may be represented as N(L). 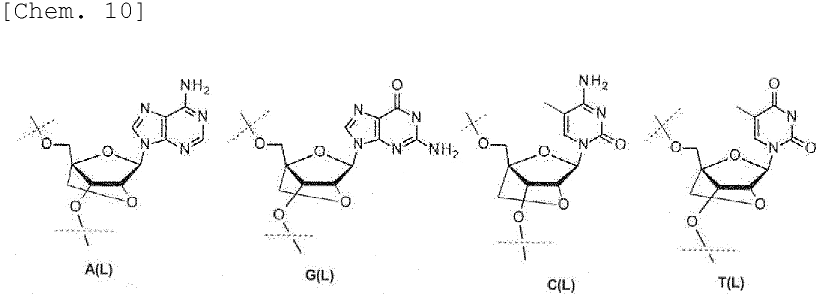 wherein the broken line indicates a point of attachment.

The oligonucleotide of the present invention or a salt thereof has an antisense strand region (sometimes referred to simply as an antisense strand) having a nucleotide sequence substantially complementary to the nucleotide sequence of a target sequence, and a sense strand region (sometimes referred to simply as a sense strand) having a nucleotide sequence substantially complementary to the nucleotide sequence of the complementary region of the antisense strand region. The sense strand region and the antisense strand region form a double-stranded structure in the nucleotide sequence of the complementary region, where the sense strand region and the antisense strand region may form a double-stranded oligonucleotide as oligonucleotides independent of each other, or the 5'-position of the nucleotide at the 5'-terminus of one of the sense strand region and the antisense strand region and the 3'-position (2'-position in the case of a nucleotide modified at the 3'-position) of the nucleotide at the 3'-terminus of the other may be connected to form a single-stranded oligonucleotide. That is, the oligonucleotide of the present invention may be a bimolecular oligonucleotide, or a monomolecular oligonucleotide. When the oligonucleotide of the present invention is a monomolecular oligonucleotide, the form of connection between the sense strand region and the antisense strand region is not limited as long as the oligonucleotide of the present invention has a RNA interfering action and/or a gene expression suppressing action. Examples thereof include connection between the 5'-position of the nucleotide at the 5'-terminus of one of the sense strand region and the antisense strand region and the 3'-position (2'-position in the case of a nucleotide modified at the 3'-position) of the nucleotide at the 3'-terminus of the other via a desired chemical linker or oligonucleotide linker.

The term "substantially identical nucleotide sequence" in the present specification includes not only a nucleotide sequence consisting of bases that are all identical to those of a nucleotide sequence of interest, but also a nucleotide sequence having a sequence identity of 70% or more to a nucleotide sequence of interest, and a nucleotide sequence which contains one or several non-identical bases, but enables the oligonucleotide to exhibit a desired function. Here, the term "identical base" includes a base fully identical to an original base, and a base having an ability to form a base pair that is equivalent or superior than an original base. As such a base, for example, a chemically modified base, which has an ability to form a base pair that is equivalent or superior than an original base, is considered identical to the original base (for example, C and 5C, or U and T(5U)). The term "sequence identity" refers to a sequence having an identity of preferably 80% or more, more preferably 90% or more, even more preferably 91%, 92%, 93%, 94%, 95%, 96%, 97%, 98% or 99%, or a mismatch in 3 bases, 2 bases or 1 base, to a nucleotide sequence of interest. The identity of a nucleotide sequence can be calculated using known gene analysis software such as BLAST (registered trademark).

The term "substantially complementary nucleotide sequence" in the present specification includes not only a nucleotide sequence consisting of nucleotides that are all complementary to those of a nucleotide sequence of interest, but also a nucleotide sequence in which one or several (for example, five, four, three or two) nucleotides are not complementary nucleotides, but the nucleotide sequence allows the oligonucleotide to form a base pair. In the present specification, the term "complementary base" refers to a base capable of forming a Watson-Crick base pair to a base of interest, and the base may be an unmodified base, or a chemically modified base. For example, 5-methyluracil and adenine are bases complementary to each other, and 5-methylcytosine and guanine are bases complementary to each other.

The term "double-stranded structure" of oligonucleotides in the present specification means that nucleotide sequences substantially complementary to each other form a Watson-Crick base pair to give a structure with double strands. The nucleotide sequences forming a double-stranded structure may be substantially complementary nucleotide sequences of two different oligonucleotide molecules, or substantially complementary sequences in a single-stranded oligonucleotide.

In the present specification, the term "structural unit" refers to a chemical structural unit that imparts a desired function to an oligonucleotide. Examples of a desired function imparted to an oligonucleotide include a function of delivering an oligonucleotide to target tissues or target cells (also referred to as target tissues or the like), and a function of improving kinetics of an oligonucleotide in the body. The structural unit can be bound to an oligonucleotide by a known method, and for example, by providing an amidite structure in the structural unit, the structural unit can be bound to the 3'-position (2'-position in the case of a nucleotide modified at the 3'-position) of the nucleotide at the 3'-terminus or the 5'-position of the nucleotide at the 5'-terminus of the oligonucleotide. The ability of delivery to target tissues or the like can be imparted to an oligonucleotide by providing a structure for promoting delivery to a target tissue or the like, such as a ligand, in the structural unit, and examples of the structure include a GalNAc unit for delivery to liver cells, and a fatty acid unit for delivery to muscular tissues. In the present specification, such a structural unit for imparting the ability of delivery to target tissues or the like may be referred to, in particular, as a "unit for delivery".

### <2. RNAi-oligonucleotide>

In the present specification, the term "RNAi-oligonucleotide" refers to an oligonucleotide in which substantially complementary nucleotides contained in a sense strand region and an antisense strand region form Watson-Crick base pairs, and the oligonucleotide contains complementary regions forming a double-stranded structure, and has a RNA interfering action and/or a gene expression suppressing action on a target gene. As long as the oligonucleotide has a RNA interfering action and/or a gene expression suppressing action on a target gene, not all the bases of the complementary region contained in the RNAi-oligonucleotide are required to form Watson-Crick base pairs. In the present specification, the RNAi-oligonucleotide may be referred to as "siRNA".

In the present specification, the term "nucleotide sequence substantially identical to a target sequence" refers to a nucleotide sequence identical to a target sequence, and includes not only a sequence fully identical to a target sequence, but also a nucleotide sequence substantially identical to a target sequence as long as the RNAi-oligonucleotide has a RNA interfering action and/or a gene expression suppressing action on a target gene.

In the present specification, the term "nucleotide sequence substantially complementary to a target sequence" refers to a nucleotide sequence complementary to a target sequence, and includes not only a sequence fully complementary to a target sequence, but also a nucleotide sequence substantially complementary to a target sequence as long as the RNAi-oligonucleotide has a RNA interfering action and/or a gene expression suppressing action on a target gene.

The oligonucleotide containing a nucleotide sequence substantially complementary to a target sequence and having a RNA interfering action and/or a gene expression suppressing action on a target gene refers to a RNAi-oligonucleotide for a target gene.

The nucleotide sequence of the RNAi-oligonucleotide for a target gene is not limited as long as the oligonucleotide has a RNA interfering action and/or a gene expression suppressing action on the target gene. It can be determined on the basis of sequences predicted to have a RNA interfering action and/or a gene expression suppressing action on the target gene by using, for example, computer software (for example, GENETYX (registered trademark): manufactured by GENETYX CORPORATION). It is also possible to perform the determination by further confirming the RNA interfering action and/or gene expression suppressing action of the RNAi-oligonucleotide prepared on the basis of the selected sequences.

In the present specification, the gene expression suppressing action includes not only an action of fully suppressing the expression of a gene, but also an action of reducing the expression of a gene as compared to a control, and the gene expression suppressing action also includes gene silencing. In the present specification, the term "gene expression suppressing action" and the term "gene expression suppressing activity" are used interchangeably with each other.

The RNA interfering action and/or gene expression suppressing action can be confirmed by a method that is normally carried out by those skilled in the art. For example, a protein that is a translated product of a target gene is quantified by western blot analysis after a certain period of time after administration of a RNAi-oligonucleotide for the target gene to cells in which the target gene is expressed, and the level of expression of the protein is compared to that of a control to confirm the RNA interfering action and/or gene expression suppressing action. It is also possible to confirm the RNA interfering action and/or gene expression suppressing action by measuring the level of expression of the target gene in real time by a real-time PCR method after administration of the RNAi-oligonucleotide for the target gene to the target gene.

In the present specification, the term "target sequence" means a sequence of 18 or more nucleotides of any part of a nucleotide sequence of a target gene, which is targeted by the RNAi-oligonucleotide of the present invention. When the target sequence is known to have SNPs or the like, the target sequence also includes a sequence having such a variation. In the RNAi-oligonucleotide of the present invention, at least a part of the antisense strand region includes a sequence substantially complementary to a target sequence (hereinafter, a "target-matching sequence").

In the present specification, the term "complementary region" means regions in which the nucleotide sequences of the sense strand region and the antisense strand region contained in the RNAi-oligonucleotide are substantially complementary to each other. The sense strand region and the antisense strand region are not necessarily fully complementary in their complementary regions, but at least the terminal bases of the complementary regions are complementary to each other. In the RNAi-oligonucleotide of the present invention, the complementary region of the antisense strand region includes a target-matching sequence, and may maintain complementarity to the sense strand region in regions obtained by further extending the 5'-terminus and/or the 3'-terminus of the complementary region.

The chain length of the target-matching sequence in the antisense strand region forming the RNAi-oligonucleotide in the present invention is not limited as long as the oligonucleotide has a RNA interfering action and/or a gene expression suppressing action. It may be any length that is equal to or longer than 18 nucleotides and equal to or smaller than the full length of the open reading frame (ORF) of a target gene. For example, the chain length of the target-matching sequence can be 19 to 29 nucleotides, and is preferably 18 to 24, 18 to 23, or 18 to 22, more preferably 18 to 21, and even more preferably 18 or 19.

The chain length of the complementary region in each of the sense strand region and the antisense strand region forming the RNAi-oligonucleotide in the present invention is not limited as long as the oligonucleotide has a RNA interfering action and/or a gene expression suppressing action. It may be any length. For example, the chain length of the target region can be 19 to 29 nucleotides, and is preferably 19 to 24, 19 to 23, or 19 to 22, more preferably 19 to 21, and even more preferably 19.

The chain length of each of the sense strand region and the antisense strand region forming the RNAi-oligonucleotide in the present invention is not limited as long as the oligonucleotide has a RNA interfering action and/or a gene expression suppressing action. It may be any length. The chain length of the sense strand region can be, for example, 19 to 39 nucleotides, and is preferably 19 to 29, more preferably 19 to 24, 19 to 23, or 19 to 22, even more preferably 19 to 21, and most preferably 19. The chain length of the antisense strand region can be, for example, 19 to 39 nucleotides, and is preferably 19 to 29, more preferably 19 to 24, 19 to 23, or 19 to 22, even more preferably 21 to 23, and most preferably 21.

In the RNAi-oligonucleotide of the present invention, when the sense strand region and the antisense strand region are present in different oligonucleotide molecules, the oligonucleotide is not required to have a double-stranded structure as a whole, and may have an overhang structure in which a part of the nucleosides at the 5'- and/or 3'-terminus protrudes, or a double-stranded structure is not formed. Examples of the overhang structure include structures consisting of, for example, 1 to 5 nucleosides, preferably 1 to 3 nucleosides, and more preferably 2 nucleosides. The overhang structure may be present at all or only a part of the termini of the double-stranded oligonucleotide. In the present specification, the overhang structure may be expressed by representing the 5'-terminus of the sense strand region as S_{O5}, the 3'-terminus of the sense strand region as S_{O3}, the 5'-terminus of the antisense strand region as A₀₅, and the 3'-terminus of the antisense strand region as A₀₃. The nucleotide sequence of the overhang structure is not limited. Oligo T or oligo U can be used.

The RNAi-oligonucleotide has at least one property selected from the following (i) to (iv):
(i) the oligonucleotide has a RNA interfering action and/or a gene expression suppressing action on a target gene;
(ii) the oligonucleotide is stable against a ribonuclease, and has a RNA interfering action and/or a gene expression suppressing action on a target gene;
(iii) the oligonucleotide is stable against an exonuclease, and has a RNA interfering action and/or a gene expression suppressing action on a target gene; and
(iv) the oligonucleotide is stable against a ribonuclease and an exonuclease, and has a RNA interfering action and/or a gene expression suppressing action on a target gene.

An example of the RNAi-oligonucleotide is derived from a double-stranded oligonucleotide having an antisense strand region for a target gene, and a sense strand region having a nucleotide sequence substantially complementary to the antisense strand region, where the 5'-position of the nucleotide at the 5'-terminus of the antisense strand region and the 3'-position (2'-position in the case of a nucleotide modified at the 3'-position) of the nucleotide at the 3'-terminus of the sense strand region may be bound via a linker forming a phosphodiester bond at both ends, which may be chemically modified. Examples of the oligonucleotide can include oligonucleotides forming the structure of oligonucleotide 3'-P(=O) (OH) - [linker] -P(=O)(OH)-5'-oligonucleotide [herein, "oligonucleotide 3'" indicates a structure in which the hydrogen atom on the hydroxyl group is not present at the 3'-position (2'-position in the case of a nucleotide modified at the 3'-position) of the nucleotide at the 3'-terminus of the oligonucleotide, and "5'-oligonucleotide" indicates a structure in which the hydrogen atom on the hydroxyl group is not present at the 5'-position of the nucleotide at the 5'-terminus of the oligonucleotide]. Such a linker is described in, for example, WO 2012/074038 and the like, and can be synthesized by reference to the descriptions of that document.

Each of the nucleosides forming the RNAi-oligonucleotide may be a natural nucleoside, or a sugar-modified nucleoside may be used. The inter-nucleoside bond is a phosphodiester bond that may be chemically modified. For example, a phosphodiester bond or a phosphorothioate bond can be appropriately selected.

### <3. S3M-oligonucleotide>

The present invention provides a RNAi-oligonucleotide (hereinafter, also referred to as a "S3M-oligonucleotide") or a pharmaceutically acceptable salt thereof in which a sense strand region consists of an oligonucleotide represented by the following formula (I), an antisense strand region consists of an oligonucleotide represented by the following formula (II), and the RNAi-oligonucleotide or pharmaceutically acceptable salt thereof has the following characteristics (a) to (g):
sense strand region: 5' S_{O5}-Sₐ-S₁₉-S₁₈-S₁₇-S₁₆-S₁₅-S₁₄-S₁₃-S₁₂-S₁₂-S₁₀-S₉-S₈-S₇-S₆-S₅-S₄-S₃-S₂-S₁-S_{O3} 3' (I)
antisense strand region: 5' A₀₅-A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-Aₐ-A₀₃ 3' (II)

(a) S₁ to S₁₉ each represent a nucleoside, S₁ is a 3'-modified nucleoside, Sₐ is 0 to 10, preferably 0 to 5 or 0 to 4, and more preferably 0 to 3, 0 to 2, or 0 to 1 nucleosides, S_{O3} and S_{O5} are each independently 0 to 5, preferably 0 to 4, and more preferably 0 to 3, 0 to 2, or 0 to 1 nucleosides, and each inter-nucleoside bond represents a phosphodiester bond that may be chemically modified;
(b) A₁ to A₁₉ each represent a nucleoside, at least one of A₁₁, A₁₂, A₁₃, A₁₄ and A₁₅ is DNA, RNA, a 2'-O,4'-C-bridging-modified nucleoside, or 2'-MOE RNA, Aₐ is 0 to 10, preferably 0 to 5 or 0 to 4, and more preferably 0 to 3, 0 to 2, or 0 to 1 nucleosides, A_{O3} and A_{O5} are each independently 0 to 5, preferably 0 to 4, and more preferably 0 to 3, 0 to 2, or 0 to 1 nucleosides, and each inter-nucleoside bond represents a phosphodiester bond that may be chemically modified;
(c) the nucleotide sequence between A₂ and Aₐ consists of a nucleotide sequence substantially complementary to a target sequence (target-matching sequence), A₁ is a nucleoside having a base complementary to a corresponding nucleoside of the target sequence, a nucleoside having adenine, a nucleoside having thymine, or a nucleoside having uracil, and when A₀₃ and A₀₅ are present, the nucleotide sequences thereof are each independently a nucleotide sequence selected independently of a corresponding nucleoside of the target sequence;
(d) the nucleotide sequence between S₁ and Sₐ and the nucleotide sequence between A₁ and Aₐ are complementary regions consisting of nucleotide sequences substantially complementary to each other, and form a double-stranded structure;
(e) when both S_{O5} and A_{O3} are present, S_{O5} and A_{O3} are nucleosides not complementary to each other;
(f) when both S_{O3} and A_{O5} are present, S_{O3} and A_{O5} are nucleosides not complementary to each other; and
(g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.

### <3-1. Sense strand region>

The sense strand region of the S3M-oligonucleotide refers to a region represented by the above formula (I), that is, S_{O5} to S_{O3}. The complementary region in the sense strand region of the S3M-oligonucleotide is represented as S₁ to S₁₉ numbered in the stated order from the nucleoside at the 3'-terminus, and Sₐ located on the **5'** side of the complementary region. That is, the complementary regions in the sense strand of the S3M-oligonucleotide is the regions S₁ to Sₐ in the above formula (I). S₁ to S₁₉ each represent a nucleoside, and Sₐ represents 0 to 5, preferably 0 to 4, and more preferably 0 to 3, 0 to 2, or 0 to 1 nucleosides. Among the overhang structures of the sense strand region, an overhang structure added at the 5'-terminus is represented as S_{O5}, and an overhang structure added at the 3'-terminus is represented as S_{O3}, and S_{O5} and S_{O3} each represent 0 to 5, preferably 0 to 4, and more preferably 0 to 3, 0 to 2, or 0 to 1 nucleosides.

The S3M-oligonucleotide is characterized in that the nucleoside located at the 3'-terminus of the complementary region in the sense strand region (that is, S₁ in the above formula (I)) is a 3'-modified nucleoside. The 3'-modified nucleoside is not limited as long as the S3M-oligonicleotide has a RNA interfering action and/or a gene expression suppressing action. It is preferably **3'-**deoxy-3'-fluoro RNA (3'-F RNA), 3'-OMe RNA, or 3'-MOE RNA, and more preferably 3'-OMe RNA. The residue at the 2'-position of the 3'-modified nucleoside may be a hydroxyl group, an optionally modified alkoxy group (preferably, a methoxy group, an ethoxy group or a methoxyethoxy group), or an optionally modified phosphate group (preferably, thiophosphate group, dithiophosphate group, methylphosphate group, or ethylphosphate group), and may be bound to the 5'-position of the nucleoside of the overhang structure (that is, S_{O3} in the above formula (I)), or may be bound to a linker structure. Preferably, the residue at the 2'-position is a hydroxyl group.

Nucleosides other than S₁ in the complementary region in the sense strand region of the S3M-oligonucleotide are not limited. Various natural nucleosides or sugar-modified nucleosides can be used. They are preferably, each independently, 2'-OMe RNA, 2'-F RNA or DNA.

An aspect of the complementary region in the sense strand region of the S3M-oligonucleotide is wherein at least one of the 11th, 12th, 13th and 15th nucleosides from the 3'-terminus (that is, S₁₁, S₁₂, S₁₃ and S₁₅ in the above formula (I)) is 2'-F RNA, and other nucleosides are 2'-OMe RNA, 2'-F RNA, DNA or a combination thereof. Preferably, all of S₁₁, S₁₂, S₁₃ and S₁₅ are 2'-F RNA.

A preferred aspect of the complementary region in the sense strand region of the S3M-oligonucleotide is wherein the nucleosides of S₂ to S₁₀, S₁₄, S₁₆ to S₁₅ and Sₐ of the complementary region in the sense strand region are each independently 2'-OMe RNA, 2'-F RNA, DNA, or a modification pattern in which two selected therefrom are arranged alternately, and more preferably 2'-OMe RNA, DNA, or a modification pattern in which two selected therefrom are arranged alternately. More preferably, all of those nucleosides are 2'-OMe RNA. The number of nucleosides in Sₐ can be appropriately selected between 0 and 10, and from the 3' side thereof, nucleosides may be arranged in the order of S₂₀, S₂₁, S₂₂, S₂₃, S₂₄, 3₂₅, S₂₆, S₂₇, S₂₈ and S₂₉ as necessary.

The nucleotide sequence of the complementary region in the sense strand region of the S3M-oligonucleotide (that is, S₁ to Sₐ in the above formula (I)) is substantially complementary to the complementary region in the antisense region (that is, A₁ to Aₐ in the above formula (II)).

The sense strand region of the S3M-oligonucleotide may have overhang structures at the 3'-terminus and/or the 5'-terminus of the complementary region thereof (that is, S_{O3} and S_{O5} in the above formula (I)). S_{O3} and S_{O5} each independently represent 0 to 5 (preferably, 4, 3, 2, 1 or 0) nucleosides, and the nucleosides are each independently 2'-OMe RNA, 2'-F RNA DNA or 2'-0,4'-C-bridging-modified nucleoside. Preferably, S_{O3} and S_{O5} are not present.

When S_{O3} and S_{O5} are present, the bases thereof are not limited as long as a function as an overhang is exhibited. It is preferably oligo U or oligo T.

The following formulae (Ia) to (Ic) show examples of the modification pattern of the sense strand region of the S3M-oligonucleotide:
5'S_{O5}(N(M))-Sₐ(N(M))-S₁₉(N(M))-S₁₈(N(M))-S₁₇(N(M))-S₁₆(N(M))-S₁₅(N(F))-S₁₄(N(M))-S₁₃(N(F))-S₁₂(N(F))-S₁₁(N(F))-S₁₀(N(M))-S₉(N(M))-S₈(N(M))-S₇(N(M))-S₆(N(M))-S₅(N(M)) -S₄(N(M))-S₃(N(M))-S₂(N(M))-S₁(N(3M))-2'H 3' (Ia)
5'Sₐ(N(M))-S₁₉(N(M))-S₁₈(N(M))-S₁₇(N(M))-S₁₆(N(M))-S₁₅(N(F))-S₁₄(N(M))-S₁₃(N(F))-S₁₂(N(F))-S₁₁(N(F))-S₁₀(N(M)) -S₉(N(M))-S₈(N(M))-S₇(N(M))-S₆(N(M))-S₅(N(M))-S₄(N(M))-S₃(N(M))-S₂(N(M)))-S₁(N(3M))-2'H 3' (Ib)
5'S₁₉(N(M))-S₁₈(N(M))-S₁₇(N(M))-S₁₆(N(M))-S₁₅(N(F))-S₁₄(N(M))-S₁₃(N(F))-S₁₂(N(F))-S₁₁(N(F))-S₁₀(N(M))-S₉(N(M)) -S₈(N(M))-S₇(N(M))-S₆(N(M))-S₅(N(M))-S₄(N(M))-S₃(N(M))-S₂(N(M)-S₁(N(3M))-2'H 3' (Ic)
wherein (N(M)) represents 2'-OMe RNA, (N(F)) represents 2'-F RNA, and (N(3M)) represents 3'-OMe RNA. S_{O5}(N(M)) and Sₐ(N(M)) represent a nucleoside consisting of 0 to 3 2'-OMe RNAs. The two inter-nucleoside bonds between the three nucleosides at each of the 5'-terminus and the 3'-terminus of each oligonucleotide are phosphorothioate bonds, and the other inter-nucleoside bonds are phosphodiester bonds. That is, each oligonucleotide has a total of four phosphorothioate bonds, with two phosphorothioate bonds at each of the 3'-terminus and the 5'-terminus.

### <3-2. Antisense strand region>

The antisense strand region of the S3M-oligonucleotide refers to a region represented by the above formula (II), that is, A_{O5} to A_{O3}. The complementary region in the antisense strand region of the S3M-oligonucleotide is represented as A₁ to A₁₉ numbered in the stated order from the nucleoside at the 5'-terminus, and Aₐ located on the 3' side of the complementary region. That is, the complementary region of the antisense strand is the region of A₁ to Aₐ in the above formula (II). The nucleotide sequence of the complementary region in the antisense strand region of the S3M-oligonucleotide is required only to be substantially complementary to a target sequence, and may include, for example, a mismatch in 5 bases, 4 bases, 3 bases, 2 bases or 1 base with the target sequence, but preferably is fully complementary to the target sequence. A₁ to A₁₉ each represent a nucleoside, and Aₐ represents 0 to 5 nucleosides. Among the overhang structures of the antisense strand region, an overhang structure added at the 5'-terminus is represented as A_{O5}, and an overhang structure added at the 3'-terminus is represented as A_{O3}.

A₁ may be substantially complementary to a corresponding base of the target sequence, and may be adenine, uracil or thymine independently of the target sequence. U.S. Patent No. 10093923 and the like indicate that good RNA interfering activity is exhibited when the base at the 3'-terminus in the complementary region of the sense strand of siRNA is adenine or uracil independently of the target sequence. In addition, a document (Nature 465, 818-822 (2010)) indicates that the nucleoside at the 5'-terminus of the antisense strand binds to Ago 2 protein, which is involved in RNA interfering activity, and does not bind to target mRNA. It is also indicated that the nucleoside at the 5'-terminus of the antisense strand more strongly binds to Ago 2 protein when the nucleoside is an adenine or uracil nucleoside than when the nucleoside is a guanine or cytosine nucleoside, and therefore, the nucleoside at the 5'-terminus of the antisense strand is preferably an adenine or uracil nucleoside.

At least one (preferably two or more) of A₁₁, A₁₂, A₁₃, A₁₄ and A₁₅ of the complementary region in the antisense strand region of the S3M-oligonucleotide is selected from DNA, RNA, a 2'-O,4'-C-bridging-modified nucleoside and 2'-MOE RNA. The 2'-O,4'-C-bridging-modified nucleoside used here is not limited. It is preferably LNA or ENA. In the antisense strand complementary region, nucleosides other that nucleosides identified as described above are not limited. Various natural nucleosides or sugar-modified nucleosides can be used. They may be, each independently, 2'-OMe RNA, 2'-F RNA or DNA.

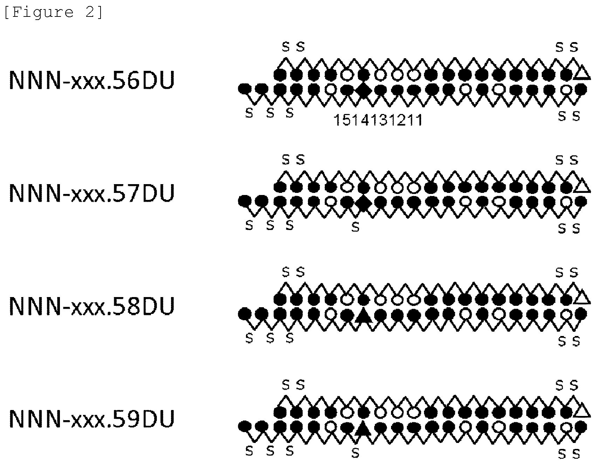

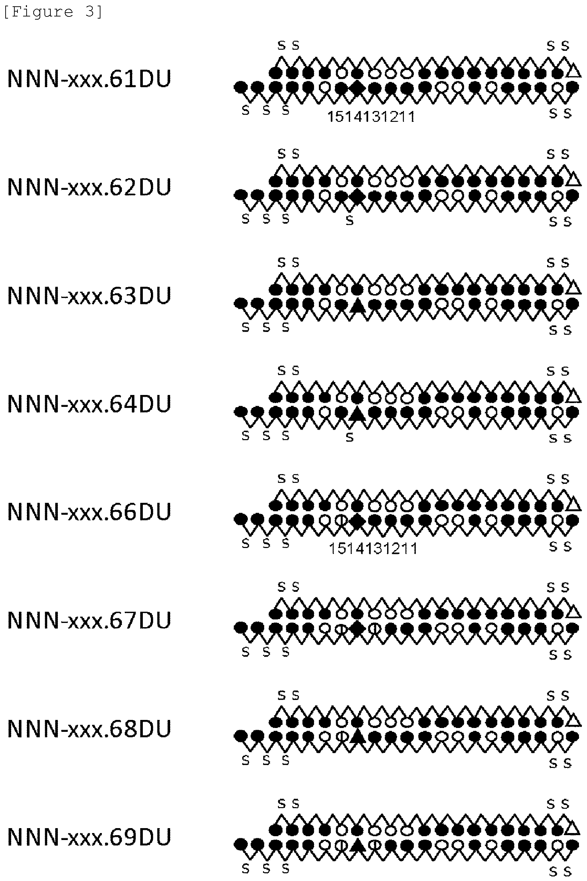

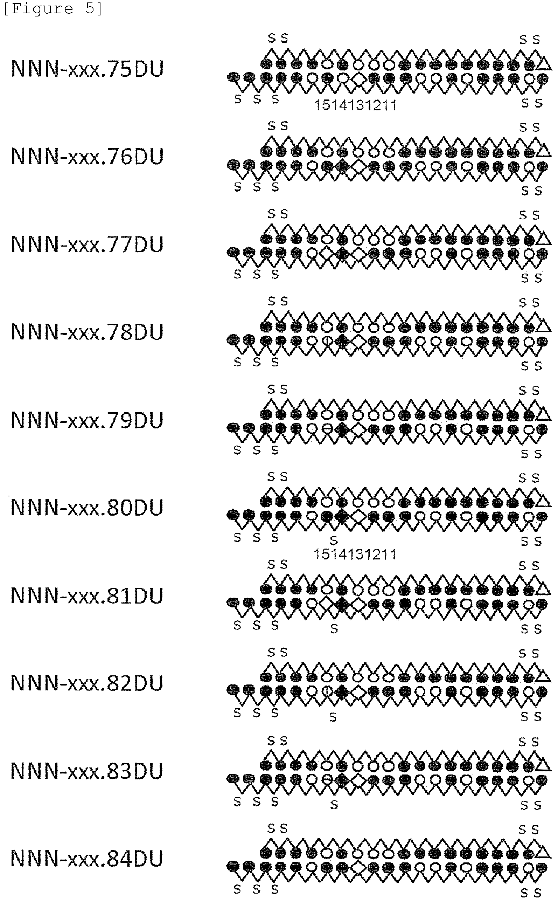

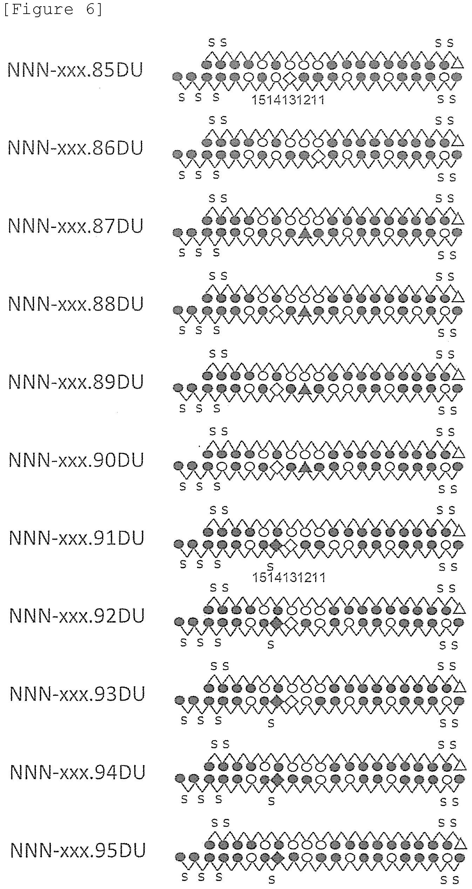

As an example of the present invention, an oligonucleotide is provided in which A₁₄ of the complementary region in the antisense strand region of the S3M-oligonucleotide is RNA. Examples of the modification pattern of such an oligonucleotide include, but are not limited to, those shown as types 56 and 57 of Figure 2, types 61, 62, 66 and 67 of Figure 3, types 75 to 83 of Figure 5, and types 91 to 95 of Figure 6.

A preferred aspect of the antisense strand region of the oligonucleotide in which A₁₄ is RNA is wherein A₁₃ is a 2'-O,4'-C-bridging-modified nucleoside, 2'-OMe RNA or 2'-F RNA, more preferably a 2'-O,4'-C-bridging-modified nucleoside, and even more preferably ENA or LNA (types 76 to 83 of Figure 5 and types 91 to 93 of Figure 6). In such a S3M-oligonucleotide, the modification pattern of A₁₁-A₁₂-A₁₃-A₁₄-A₁₅ is preferably any of the following formulae (IIIa) to (IIIh).
A₁₁(2'-OMeRNA)-A₁₂(2'-OMe RNA)-A₁₃(ENA)-A₁₄(RNA)-A₁₅(2'-OMe RNA) (IIIa)
A₁₁(2'-F RNA)-A₁₂(2'-OMe RNA) -A₁₃(ENA)-A₁₄(RNA)-A₁₅(2'-OMe RNA) (IIIb)
A₁₁(2'-OMe RNA)-A₁₂(2'- F RNA)-A₁₃(ENA)-A₁₄(RNA)-A₁₅(2'-OMe RNA) (IIIc)
A₁₁(2'-OMe RNA)-A₁₂(2'-OMe RNA)-A₁₃(LNA)-A₁₄(RNA)-A₁₅(2'-OMe RNA) (IIId)
A₁₁(2'-F RNA)-A₁₂(2'-OMe RNA)-A₁₃(LNA)-A₁₄(RNA)-A₁₅(2'-OMe RNA) (IIIe)
A₁₁(2'-OMe RNA)-A₁₂(2'-F RNA)-A₁₃(LNA)-A₁₄(RNA)-A₁₅(2'-OMe RNA) (IIIf)
A₁₁(2'-F RNA)-A₁₂(2'-OMe RNA)-A₁₃(2'-OMe RNA)-A₁₄(RNA)-A₁₅(2'-OMe RNA) (IIIg)
A₁₁(2'-OMe RNA)-A₁₂(2'-F RNA)-A₁₃(2'-OMe RNA)-A₁₄(RNA)-A₁₅(2'-OMe RNA) (IIIh)

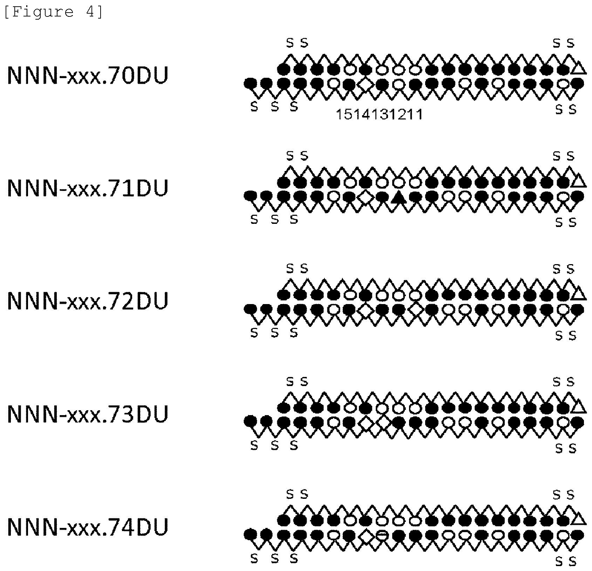

As an example of the present invention, an oligonucleotide is provided in which A₁₄ of the complementary region in the antisense strand region of the S3M-oligonucleotide is a 2'-O,4'-C-bridging-modified nucleoside. Here, the 2'-O,4'-C-bridging-modified nucleoside is preferably ENA or LNA. Examples of the modification pattern of such an oligonucleotide include, but are not limited to, those shown as types 70 to 74 of Figure 4, and types 88 to 90 of Figure 6.

A preferred aspect of the oligonucleotide in which A₁₄ is a 2'-O,4'-C-bridging-modified nucleoside is wherein A₁₂ is DNA. In such a S3M-oligonucleotide, the modification pattern A₁₁-A₁₂-A₁₃-A₁₄-A₁₅ of the antisense strand complementary region is preferably any of the following formulae (IIIi) to (IIIl).
A₁₁(2'-F RNA)-A₁₂(DNA)-A₁₃(2'-OMe RNA) -A₁₄(ENA)-A₁₅(2'-OMe RNA) (IIIi)
A₁₁(2'-OMe RNA)-A₁₂(DNA)-A₁₃(2'-OMe RNA)-A₁₄(ENA)-A₁₅(2'-OMe RNA) (IIIj)
A₁₁(2'-F RNA)-A₁₂(DNA)-A₁₃(2'-OMe RNA)-A₁₄(LNA)-A₁₅(2'-OMe RNA) (IIIk)
A₁₁(2'-OMe RNA) -A₁₂(DNA)-A₁₃(2'-OMe RNA) -A₁₄(LNA)-A₁₅(2'-OMe RNA) (IIIl)

As an example of the present invention, an oligonucleotide is provided in which A₁₄ of the complementary region in the antisense strand region of the S3M-oligonucleotide is DNA. Examples of the modification pattern of such an oligonucleotide include, but are not limited to, those shown as types 58 and 59 of Figure 2, and types 63, 64, 68 and 69 of Figure 3.

As an example of the present invention, an oligonucleotide is provided in which A₁₅ of formula (II), which is the antisense strand region of the S3M-oligonucleotide is 2'-MOE RNA. Examples of the modification pattern of such an oligonucleotide include, but are not limited to, those shown as types 79 and 83 of Figure 5.

As an example of the present invention, an oligonucleotide is provided in which any of the 11th to 13th nucleosides from the 5'-terminus of the complementary region in the antisense strand region of the S3M-oligonucleotide (A₁₁, A₁₂ and A₁₃ of formula (II)) is a 2'-O,4'-C-bridging-modified nucleoside. Examples of the modification pattern of such an oligonucleotide include, but are not limited to, those shown as types 43 and 45 of Figure 1, types 75 to 84 of Figure 4 and types 85 and 86 and 91 to 93 of figure 6 for ENA, and types 44 and 46 of Figure 1 and type 67 and 69 of Figure 3 for LNA.

As an example of the present invention, an oligonucleotide is provided in which any of the 11th to 13th nucleosides from the 5'-terminus of the complementary region in the antisense strand region of the S3M-oligonucleotide (A₁₁, A₁₂ and A₁₃ of formula (II)) is DNA. Examples of the modification pattern of such an oligonucleotide include, but are not limited to, those shown as types 41 and 42 of Figure 1, type 71 of Figure 4, and types 87 to 90 of Figure 6.

As an example of the present invention, an oligonucleotide is provided in which any of the 11th to 13th nucleosides from the 5'-terminus of the complementary region in the antisense strand region of the S3M-oligonucleotide (A₁₁, A₁₂ and A₁₃ of formula (II)) is 2'-MOE RNA. Examples of the modification pattern of such an oligonucleotide include, but are not limited to, those shown as types 47 and 48 of Figure 1, and type 74 of figure 4.

For nucleosides other than the nucleosides specified as described above in the complementary region in the antisense strand region of the S3M-oligonucleotide of the present invention, various types of nucleosides can be appropriately selected, but preferably, at least one (preferably all) of the nucleosides of A₂, A₆ and A₁₆ is 2'-F RNA. A more preferred antisense strand region is such that, in addition to A₂, A₆ and A₁₆, one or two nucleosides selected from the group consisting of A₈, A₉ and A₁₀ are 2'-F RNA, and nucleosides other than those specified as described above (for example, A₁, A₃ to A₅, A₇, A₁₇ to A₁₉ and Aₐ) are 2'-OMe RNA or DNA.

Preferable modification patterns of regions other than A₁₁ to A₁₅ in the complementary region in the antisense strand region of the S3M-oligonucleotide of the present invention are shown in, for example, Tables A-1 and A-2 below.

**[Table A-1]**

| | A₁ | A₂ | A₃ | A₄ | A₅ | A₆ | A₇ | A₈ | A₉ | A₁₀ |
|---|---|---|---|---|---|---|---|---|---|---|
| Pattern 1 | (M) | (F) | (M) | (M) | (M) | (F) | (M) | (M) | (M) | (M) |
| Pattern 2 | (M) | (F) | (M) | (M) | (M) | (F) | (M) | (F) | (M) | (M) |
| Pattern 3 | (M) | (F) | (M) | (M) | (M) | (F) | (M) | (F) | (M) | (M) |
| Pattern 4 | (M) | (F) | (M) | (M) | (M) | (F) | (M) | (M) | (F) | (M) |
| Pattern 5 | (M) | (F) | (M) | (M) | (M) | (F) | (M) | (M) | (M) | (F) |
| Pattern 6 | (M) | (F) | (M) | (M) | (M) | (F) | (M) | (F) | (M) | (F) |
| Pattern 7 | (M) | (F) | (M) | (M) | (M) | (F) | (F) | (M) | (F) | (M) |
| Pattern 8 | (M) | (F) | (M) | (F) | (M) | (F) | (M) | (M) | (M) | (M) |
| Pattern 9 | (M) | (F) | (M) | (F) | (M) | (F) | (M) | (F) | (M) | (M) |
| Pattern 10 | (M) | (F) | (M) | (F) | (M) | (F) | (M) | (F) | (M) | (F) |
| Pattern 11 | (M) | (F) | (M) | (F) | (M) | (F) | (M) | (F) | (F) | (M) |
| Pattern 12 | (M) | (F) | (M) | (F) | (M) | (F) | (M) | (F) | (F) | (F) |
| Pattern 13 | (M) | (F) | (M) | (M) | (M) | (F) | (M) | (F) | (F) | (M) |
| Pattern 14 | (M) | (F) | (M) | (F) | (M) | (F) | (M) | (M) | (F) | (M) |
| Pattern 15 | (M) | (F) | (M) | (M) | (M) | (F) | (M) | (M) | (F) | (M) |
| Pattern 16 | (M) | (F) | (M) | (M) | (M) | (F) | (M) | (M) | (F) | (F) |

**[Table A-2]**

| | A₁₆ | A₁₇ | A₁₈ | A₁₉ | Aₐ | A_{O3} |
|---|---|---|---|---|---|---|
| Pattern 1 | (F) | (M) | (M) | (M) | (M) | (M) |
| Pattern 2 | (F) | (M) | (M) | (M) | (M) | (M) |
| Pattern 3 | (F) | (M) | (M) | (M) | (M) | (M) |
| Pattern 4 | (F) | (M) | (M) | (M) | (M) | (M) |
| Pattern 5 | (F) | (M) | (M) | (M) | (M) | (M) |
| Pattern 6 | (F) | (M) | (M) | (M) | (M) | (M) |
| Pattern 7 | (F) | (M) | (M) | (M) | (M) | (M) |
| Pattern 8 | (F) | (M) | (M) | (M) | (M) | (M) |
| Pattern 9 | (F) | (M) | (M) | (M) | (M) | (M) |
| Pattern 10 | (F) | (M) | (M) | (M) | (M) | (M) |
| Pattern 11 | (F) | (M) | (M) | (M) | (M) | (M) |
| Pattern 12 | (F) | (M) | (M) | (M) | (M) | (M) |
| Pattern 13 | (F) | (M) | (M) | (M) | (M) | (M) |
| Pattern 14 | (F) | (M) | (M) | (M) | (M) | (M) |
| Pattern 15 | (F) | (M) | (F) | (M) | (M) | (M) |
| Pattern 16 | (F) | (M) | (M) | (M) | (M) | (M) |

[In the tables above, (M) is 2'-OMe RNA, and (F) is 2'-F RNA. Aₐ is 0 to 5 nucleosides, where all the nucleosides are the same except for the case of 0 nucleosides. A_{O3} is 0 to 5 nucleosides, where all the nucleosides are the same except for the case of 0 nucleosides. For the inter-nucleoside bond, a phosphodiester bond or a phosphorothioate bond is appropriately selected.]

The antisense strand region contained in the S3M-oligonucleotide may have overhang structures at the 3'-terminus and/or the 5'-terminus of the complementary region thereof (that is, A_{O3} and A_{O5} in the above formula (II)). A_{O3} and A_{O5} each independently represent 0 to 5 (preferably, 3, 2, 1 or 0) nucleosides, where the nucleosides are each independently 2'-OMe RNA, 2'-F RNA, DNA or a 2'-O,4'-C-bridging-modified nucleoside. Preferably, A_{O3} is an overhang consisting of two or three 2'-OMe RNAs, or an overhang consisting of one or two 2'-OMe RNAs and one or two 2'-O,4'-C-bridging-modified nucleosides, and A_{O5} is not present.

When A_{O3} and A_{O5} are present, the bases thereof are not limited as long as a function as an overhang is exhibited. They are preferably oligo U or oligo T.

### <3-3. Inter-nucleoside bond>

For the form of each inter-nucleoside bond in the S3M-oligonucleotide of the present invention, a phosphodiester bond that may be chemically modified can be appropriately selected, and a phosphodiester bond or a phosphorothioate bond is preferred. Those bonds can be used alone, or in combination. A combination of phosphodiester bonds or phosphorothioate bonds is preferred, and the phosphorothioate bond content in inter-nucleoside bonds contained in each of the sense strand region and the antisense strand region is 50% or less, 40% or less, 30% or less, 29% or less, 28% or less, 27% or less, 26% or less, or 25% or less.

When RNA and/or 2'-F RNA are used, the bond between the relevant nucleoside and a 3' adjacent nucleoside may be a phosphorothioate bond. When RNA is used, the bond between the relevant nucleoside and a 3' adjacent nucleoside is preferably a phosphorothioate bond.

In the S3M-oligonucleotide of the present invention, the phosphorothioate bond is preferably used for bonds between the 1st and 2nd nucleosides up to between the 4th and 5th nucleosides (the number of inter-nucleoside bonds is 1, 2, 3 or 4) from the 5'-terminus and/or the 3'-terminus of the oligonucleotide. In the oligonucleotide of the present invention, when the sense strand region and the antisense strand region are contained in different oligonucleotides, bonds between the 1st and 2nd nucleosides up to between the 4th and 5th nucleosides from each of the 5'-terminus and the 3'-terminus of the sense strand region and bonds between the 1st and 2nd nucleotides up to between the 4th and 5th nucleotides from each of the 5'-terminus and the 3'-terminus of the antisense strand region may be phosphorothioate bonds. Preferably, bonds between the 1st and 2nd nucleosides up to between the 2nd and 3rd nucleosides (the number of inter-nucleoside bonds is 2) from each of the 5'-terminus and the 3'-terminus of the sense strand region, and bonds between the 1st and 2nd nucleosides up to between the 2nd and 3rd nucleosides (the number of inter-nucleoside bonds is 2) from the 5'-terminus and bonds between the 1st and 2nd nucleotides up to between the 3rd and 4th nucleosides (the number of inter-nucleoside bonds is 3) from the 3'-terminus of the antisense strand region are phosphorothioate bonds.

### <4. Method for synthesizing oligonucleotide>

The method for preparing the oligonucleotide of the present invention is not limited as long as it enables synthesis of a desired oligonucleotide. A known chemical synthesis method (phosphotriester method, phosphoramidite method, a H-phosphonate method or the like) can be used. For example, the oligonucleotide can be synthesized with commercially available reagents for use in DNA/RNA using a commercially available nucleic acid synthesizer.

When a normal phosphoramidite method is used, an oligonucleotide having a desired nucleotide sequence can be synthesized by a method described in the document Nucleic Acids Research, 12, 4539 (1984) using a DNA synthesizer, for example, Model 392 based on phosphoramidite method from PerkinElmer, Inc.

Amidite reagents corresponding to various nucleosides may be obtained by purchasing those that are commercially available, or performing synthesis by a known method.

The oligonucleotide of the present invention can be synthesized by a method described in the document Nucleic Acids Research, 12, 4539 (1984) using a commercially available DNA synthesizer (for example, Model 392 based on phosphoramidite method from PerkinElmer, Inc.). Phosphoramidite reagents used here may be commercially available phosphoramidite reagents for natural nucleosides and 2'-O-methylnucleosides (that is, 2'-O-methylguanosine, 2'-O-methyladenosine, 2'-O-methylcytidine and 2'-O- methyluridine). The following applies to 2'-0-alkylguanosines, 2'-0-alkyladenosines, 2'-0-alkylsytidines and 2'-O-alkyluridines in which the number of carbon atoms in the 2'-O-alkyl group is 2 to 6.

For 2'-0-aminoethylguanosine, 2'-O-aminoethyladenosine, 2'-O-aminoethylcytidine and 2'-O-aminoethyluridine, corresponding phosphoramidite reagents can be synthesized according to the document Blommers et al. Biochemistry (1998), 37, 17714-17725.

For 2'-O-propylguanosine, 2'-O-propyladenosine, 2'-O-propylcytidine and 2'-O-propyluridine, corresponding phosphoramidite reagents can be synthesized according to the document Lesnik, E.A. et al. Biochemistry (1993), 32, 7832-7838.

For 2'-O-allylguanosine, 2'-O-allyladenosine, 2'-O-allylcytidine and 2'-O-allyluridine, commercially available phosphoramidite reagents can be used.

For 2'-O-methoxyethylguanosine, 2'-O-methoxyethyladenosine, 2'-0-methoxyethyl-5-methylcytidine and 2'-0-methoxyethyl-5-methyluridine for 2'-MOE RNA, corresponding phosphoramidite reagents can be synthesized according to patent no. US 6261840 or the document Martin, P. Helv. Chim. Acta. (1995) 78, 486-504, and commercially available phosphoramidite reagents can be used.

For 2'-O-butylguanosine, 2'-0-butyladenosine, 2'-O-butylcytidine and 2'-O-butyluridine, corresponding phosphoramidite reagents can be synthesized according to the document Lesnik, E.A. et al. Biochemistry (1993), 32, 7832-7838.

For 2'-0-pentylguanosine, 2'-0-pentyladenosine, 2'-O-pentylcytidine and 2'-O-pentyluridine, corresponding phosphoramidite reagents can be synthesized according to the document Lesnik, E.A. et al. Biochemistry (1993), 32, 7832-7838.

For 2'-0-propargylguanosine, 2'-O-propargyladenosine, 2'-0-propargylcytidine and 2'-O-propargyluridine, commercially available phosphoramidite reagents can be used.

For 2'-deoxy-2'-fluoroguanisine, 2'-deoxy-2'-fluoroadenosine, 2'-deoxy-2'-fluorocytidine and 2'-deoxy-2'-fluorouridine for 2'-F RNA, corresponding phosphoramidite reagents can be synthesized according to the document J. Med. Chem. 36, 831 (1993), and commercially available phosphoramidite reagents can be used.

For 3'-F RNA that is a 3'-modified nucleoside, corresponding phosphoramidite reagents can be synthesized according to WO 2017027645 for 3'-deoxy-3'-fluoroguanosine, WO2018198076 for 3'-deoxy-3'-fluoroadenosine, and US 2011/0196141 for 3'-deoxy-3'-fluorouridine. For 3'-deoxy-3'-fluorocytidine, the synthesis can be performed by reference to known methods in the above-mentioned documents and the like.

For 3'-OMe RNA that is a 3'-modified nucleoside, 3'-O-methylguanosine, 3'-O-methyladenosine, 3'-O-methylcytidine and 3'-O-methyluridine can be synthesized using commercially available phosphoramidite reagents.

For 3'-0-methoxyethylguanosine, 3'-O-methoxyethyladenosine, 3'-0-methoxyethyl-5-methylcytidine and 3'-0-methoxyethyl-5-methyluridine for 3'-MOE RNA that is a 3'-modified nucleoside, corresponding phosphoramidite reagents can be synthesized according to US 2003/0096979.

For 2'-0,4'-C-methyleneguanosine, 2'-O,4'-C-methyleneadenosine, 2'-O,4'-C-methylenecytidine, 2'-O,4'-C-methylene-5-methylcytidine and 2'-O,4'-C-methylene-5-methyluridine for LNA, corresponding phosphoramidite reagents can be produced by a method described in WO 99/14226.

For 2'-O,4'-C-alkyleneguanosines, 2'-O,4'-C-alkyleneadenosines, 2'-O,4'-C-alkylenecytidines, 2'-O,4'-C-alkylene-5-methylcytidines and 2'-O,4'-C-alkylene-5-methyluridines in which the number of carbon atoms in the alkylene group of the 2'-O,4'-C-bridge moiety is 2 to 5, corresponding phosphoramidite reagents can be produced by a method described in WO 00/47599.

For nucleosides subjected to modification of D-ribofuranose with 2'-deoxy-2'-C,4'-C-methyleneoxymethylene, corresponding phosphoramidite reagents can be synthesized according to the document Wang, G. et al. Tetrahedron (1999), 55, 7707-7724.

For S-cEt (constrained ethyl), corresponding phosphoramidite reagents can be synthesized according to the document Seth, P.P. et al. J. Org. Chem (2010), 75, 1569-1581.

For AmNA, corresponding phosphoramidite reagents can be synthesized according to the document Yahara, A. et al. ChemBioChem (2012), 13, 2513-2516 or WO 2014/109384.

An antisense oligonucleotide having a phosphorothioate bond can be synthesized by coupling phosphoramidite reagents, followed by reaction with a reagent such as sulfur, tetraethylthiuram disulfide (TETD, Applied Biosystems), a Beaucage reagent (Glen Research) or xanthan hydride (Tetrahedron Letters, 32, 3005 (1991), J. Am. Chem. Soc. 112, 1253 (1990), PCT/WO 98/54198).

Commercially available solid-phase supports can be used in a synthesizer, for which controlled pore glass (CPG) and polystyrene are used as solid-phase supports and to which a 2'-O-methylnucleoside and a 3'-O-methylnucleoside are bound. For 2'-O,4'-C-methyleneguanosine, 2'-O,4'-C-methyleneadenosine, 2'-O,4'-C-methylene-5-methylcytidine and 2'-O,4'-C-methylene-5-methyluridine, nucleosides produced by a method described in WO 99/14226 can be bound to CPG, and for 2'-O,4'-C-alkyleneguanosines, 2'-O,4'-C-alkyleneadenosines, 2'-O,4'-C-alkylene-5-methylcytidines and 2'-O,4'-C-alkylene-5-methyluridines in which the number of carbon atoms in the alkylene is 2 to 5, nucleosides produced by a method described in WO 00/47599 can be bound to CPG according to the document Oligonucleotide Synthesis, Edited by M. J. Gait, Oxford University Press, 1984. The synthesis can be performed by bonding any of the above-described phosphoramidite reagents using universal resin. Use of modified CPG (described in Japanese Patent Laid-Open No. 7-87982, Example 12b) enables synthesis of an oligonucleotide in which a 2-hydroxyethylphosphate group is bound to the 3'-terminus. Use of 3'-amino-modifier C3 CPG, 3'-amino-Modifier C7 CPG, Glyceryl CPG, (Glen Research), 3'-specer C3 SynBase CPG 1000 or 3'-spacer C9 SynBase CPG 1000 (link technologies) enables synthesis of an oligonucleotide in which a hydroxyalkylphosphate group or an aminoalkylphosphate group is bound to the 3'-terminus.

When an oligonucleotide analog is thioate-modified, a thioate derivative can be obtained by a method described in the document Tetarhedron Letters, 32, 3005 (1991), J. Am. Chem. Soc., 112, 1253 (1990) using reagents such as tetraethylthiuram disulfide (TETD, Applied BioSystems), a Beaucage reagent, and a phenylacetyl disulfide/pyridine-acetonitrile (1 : 1 v/v) solution (Ravikumar, V.T. et al. Bioorg. Med. Chem. Lett. (2006) 16, p. 2513-2517) in addition to sulfur.

When a silyl-based protective group such as t-butyldimethylsilyl (TBS) is used as a protective group for the 2'-hydroxyl group of RNA, the protection can be eliminated using tetrabutylammonium fluoride (TBAF), HF-pyridine, HF-triethylamine or the like.

Protection of the acyl group of the base moiety and the cyanoethyl group of the phosphate moiety can be eliminated using concentrated aqueous ammonia, methanolic ammonia, ethanolic ammonia, a concentrated aqueous ammonia-ethanol (3 : 1 V/V) mixed liquid, a concentrated aqueous ammonia-40% methylamine aqueous solution (1 : 1 V/V) mixed liquid, methylamine, a 0.5 M LiOH aqueous solution, a 3.5 M triethylamine/methanol solution (1 : 10 V/V) mixed liquid, 0.05 M potassium carbonate-containing methanol or the like. Concentrated aqueous ammonia and a concentrated aqueous ammonia-ethanol mixed liquid (3 : 1 V/V) are preferred.

The oligonucleotide of the present invention can be obtained by performing purification through any purification process for use in normal nucleic acid purification, for example, various kinds of chromatography such as reversed-phase chromatography and ion-exchange chromatography (including high-performance liquid chromatography).

The oligonucleotide of the present invention includes an oligonucleotide to which a unit for delivery for delivering the oligonucleotide to target tissues or target cells is bound. Examples of the oligonucleotide to which a unit for delivery is bound can include oligonucleotides in which a unit with an aminoalkylphosphate group introduced into a desired chemical structure (for example, the alkyl has 3 to 9 carbon atoms) is bound to the 5'-position of the nucleotide at the 5'-terminus and/or the 3'-position (2'-position in the case of a nucleotide modified at the 3'-position) of the nucleotide at the 3'-terminus of the oligonucleotide. As such a unit for delivery, for example, a GalNAc unit binding to asialoglyco-receptors (ASGRPs) of liver parenchymal cells, or a fatty acid (for example, myristic acid, palmitic acid, stearic acid, arachidic acid or behenic acid) unit for delivery to liver and muscular tissues (see, for example, Nucleic Acids Res. (2020) 47, 6029-6044, WO 2017/19267), can be used as appropriate.

The GalNAc unit is a structural unit containing N-acetyl-D-galactosamine (GalNAc) capable of binding to ASGRP, and can be used for delivering the oligonucleotide of the present invention to the liver. The GalNAc unit may have a linear or branched linker structure, and can have, for example, a linear linker structure, or a branched linker structure that diverges into two or more, three or more, or four or more branches, or four or less, three or less, or two or less branches. The GalNAc unit may contain a phosphate group or a thiophosphate group for binding to an oligonucleotide. As long as the ability to bind to ASGRP is maintained, the number of GalNAcs contained in one GalNAc unit is not limited, and the GalNAc structure may be modified. Examples GalNAc units that can be used with the oligonucleotide of the present invention are known GalNAc units described in WO 2021/049504, WO 2009/073809, WO 2014/076196, WO 2014/179620, WO 2015/006740, WO 2015/105083, WO 2016/0556012017/023817, WO 2017/084987, WO 2017/131236, Methods in Enzymology, 1999, Vol. 313, p. 297-321, and Bioorganic & Medicinal Chemistry Letters 26 (2016) 3690-3693, as appropriate.

Examples of the GalNAc unit that can be used include those having the following formula, and can be appropriately adjusted by a method described in WO 2021/049504. 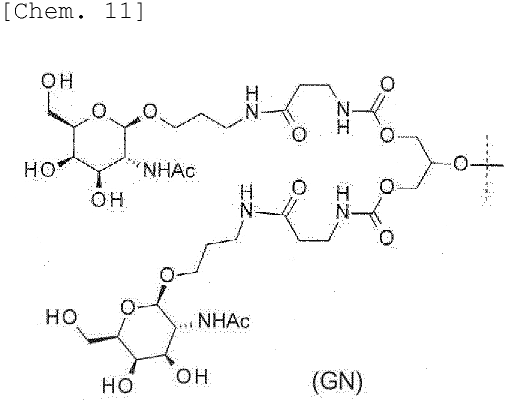 wherein the broken line represents a point of attachment, the oxygen atom at the point of attachment binds to an adjacent nucleotide, and forms a phosphodiester bond with the 5'-phosphate group when binding to the nucleotide at the 5'-terminus, and with the 3'-phosphate group (phosphate group at the 2'-position in the case of a nucleotide modified at the 3'-position) when binding to the nucleotide at the 3'-terminus, and the phosphodiester bond formed may be a chemically modified phosphodiester bond such as a phosphorothioate bond.

The oligonucleotide of the present invention also includes an oligonucleotide in which a cholesterol unit, a lipid unit or a vitamin E unit is introduced into the oligonucleotide (see, for example, Lorenz, C. et al. Bioorg. Med. Chem. Lett., 14, p. 4975-4977 (2004); Soutschek, J., et al. Nature, 432, p. 173-178, (2004); Wolfrum, C. et al. Nature Biotech. 25, p. 1149-1157, (2007)) Kubo, T. et al. Oligonucleotides, 17, p. 1-20, (2007); Kubo, T., et al. Biochem. Biophys. Res. Comm. 365, p. 54-61, (2008); and Nishina, K., et al., Mol. Ther. 16, p. 734-740, (2008)), and an oligonucleotide in which an aptamer, which is a nucleic acid molecule capable of binding to a protein, is bound to a terminus of the oligonucleotide.

The oligonucleotide of the present invention also includes an oligonucleotide in which the oligonucleotide is bound to a monoclonal antibody (or an appropriate binding moiety thereof), or a protein (or an appropriate oligopeptide fragment thereof) (see, for example, Song, et al. Nature Biotech. 23, p. 709-717 (2005); and Xia et al. Pharm. Res. 24, p. 2309-2316 (2007), Kumar, et al. Nature, 448, p. 39-43 (2007)).

The oligonucleotide of the present invention also includes a positively charged complex obtained by adding a cationic polymer to the oligonucleotide (see, as examples of where distribution to organs and cells could be achieved, Leng et al. J. Gen. Med. 7, p. 977-986 (2005), Baigude et al. 2, p. 237-241, and ACS Chem. Biol. (2007), Yadava et al. Oligonucleotide 17, p. 213-222 (2007)).

The oligonucleotide of the present invention includes all pharmaceutically acceptable salts or esters of the oligonucleotides, or salts of such esters. Preferred examples of the pharmaceutically acceptable salt of the oligonucleotide of the present invention can include metal salts such as alkali metal salts such as sodium salts, potassium salts and lithium salts, alkaline earth metals such as calcium salts and magnesium salts, aluminium salts, iron salts, zinc salts, copper salts, nickel salts, and cobalt salts; amine salts such as inorganic amine salts such as ammonium salts, and organic amine salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts such as halogenated hydracid salts such as hydrofluoric acid salts, hydrochloric acid salts, hydrobromic acid salts and hydroiodic acid salts, nitric acid salts, perchloric acid salts, sulfuric acid salts, and phosphoric acid salts; organic acid salts such as lower alkanesulfonic acid salts such as methanesulfonic acids salts, trifluoromethanesulfonic acid salts and ethanesulfonic acid salts, arylsulfonic acid salts such as benzenesulfonic acid salts and p-toluenesulfonic acid salts, and acetic acid salts, malic acid salts, fumaric acid salts, succinic acid salts, citric acid salts, tartaric acid salts, oxalic acid salts, and maleic acid salts; and amino acid salts such as glycine salts, lysine salts, arginine salts, ornithine salts, glutamine acid salts, and asparagine acid salts.

A composition containing the oligonucleotide of the present invention is mixed, encapsulated or conjugated with another molecule, molecular structure, or mixture of compounds to form, for example, as a liposome, a receptor-targeting molecule, an oral, rectal, or local formulation or other formulation for assisting uptake, distribution and/or absorption.

When the oligonucleotide of the present invention is used as a prophylactic drug or a therapeutic drug for a disease, the oligonucleotide or a pharmacologically acceptable salt thereof can be itself administered, or be mixed with a pharmacologically acceptable appropriate excipient, diluent and the like, and administered orally as a tablet, a capsule, a granule, a powder, a syrup or the like, or administered parenterally as an injection, a suppository, a patch, an external preparation or the like.

These preparations are produced by well-known methods using additives such as excipients (examples thereof can include organic excipients such as sugar derivatives such as lactose, white sugar, glucose, mannitol and sorbitol; starch derivatives such as corn starch, potato starch, α-starch and dextrin; cellulose derivatives such as crystalline cellulose; gum arabic; dextran; and pullulan; and inorganic excipients such as silicic acid salt derivatives such as light anhydrous silicic acid, synthetic aluminum silicate, calcium silicate and magnesium aluminometasilicate; phosphoric acid salts such as calcium hydrogen phosphate; carbonic acid salts such as calcium carbonate; and sulfuric acid salts such as calcium sulfate), lubricants (examples thereof can include stearic acid, stearic acid metal salts such as calcium stearate and magnesium stearate; talc; colloid silica; waxes such as bead wax and spermaceti; boric acid; adipic acid; sulfuric acid salts such as sodium sulfate; glycol; fumaric acid; sodium benzoate; DL leucin; lauryl sulfuric acid salts such as sodium lauryl sulfate and magnesium lauryl sulfate; silicic acids such as anhydrous silicic acid and silicic acid hydrate; and the above-described starch derivatives), binders (examples thereof can include hydroxypropylcellulose, hydroxypropylmethylcellulose, polyvinylpyrrolidone, macrogol, and the same compounds as the above-described excipients), disintegrants (examples thereof can include cellulose derivatives such as lowsubstituted hydroxypropylcellulose, carboxymethylcellulose, carboxymethylcellulose calcium and internally cross-linked carboxymethylcellulose sodium; and chemically modified starch/celluloses such as carboxymethyl starch, carboxymethyl starch sodium and cross-linked polyvinylpyrrolidone), emulsifiers (examples thereof can include colloidal clays such as bentonite and veegum; metal hydroxides such as magnesium hydroxide and aluminum hydroxide; anionic surfactants such as sodium lauryl sulfate and calcium stearate; cationic surfactants such as benzalkonium chloride; and nonionic surfactants such polyoxyethylene alkyl ethers, polyoxyethylene sorbitan fatty acid esters and sucrose fatty acid esters), stabilizers (examples thereof can include paraoxybenzoic acid esters such as methylparaben and propylparaben; alcohols such as chlorobutanol, benzyl alcohol and phenylethyl alcohol; benzalkonium chloride; phenols such as phenol and cresol; thimerosal; dehydroacetic acid; and sorbic acid), taste and odor improvers (examples thereof can include sweeteners, sour agents and flavors as normally used), and diluents.

The body to be subjected to introduction of an oligonucleotide prepared as described above is not limited as long as it allows a target gene to be transcribed into RNA in its cell. The body to be subjected to introduction means a cell, a tissue or an individual.

The cell into which the oligonucleotide of the present invention is introduced may be a germline cell, a somatic cell, a differentiating totipotent cell, a multipotent cell, a cleaved cell, a non-cleaved cell, a parenchymal tissue cell, an epidermal cell, an immortalized cell, a transformed cell, a nerve cell, or an immune cell.

The tissue includes a single-cell embryo or a constitutive cell, or a multiple-cell embryo, a fetal tissue and the like. Examples of differentiated cells include adipose cells, fibroblast cells, muscular cells, myocardial cells, endothelial cells, nerve cells, glial cells, blood cells, megakaryocytes, lymphocytes, macrophages, neutrophils, eosinophils, basophils, mast cells, leucocytes, granulocytes, keratinocytes, cartilage cells, osteoblast cells, osteoclastic cells, liver cells, and endocrine or exocrine cells. As examples of these cells, CHO-K1 cells (RIKEN Cell bank), Drosophila S2 cells (Schneider, I. et al., J. Embryol. Exp. Morph., 27, p. 353-365 (1972), human HeLa cells (ATCC: CCL-2), or human HEK 293 cells (ATCC: CRL-1573), and the like are preferably used.

Examples of an individual used as a body to be subjected to introduction of the oligonucleotide of the present invention further include specifically those belonging to plants, animals, protozoa, viruses, bacteria or fungi. The plant may be a monocotyledonous plant, a dicotyledonous plant, or a gymnospermous plant, and the animal may be a vertebrate or an invertebrate. The vertebrate is preferably a mammal including a mouse, a rat, a monkey, a dog, or a human.

As a method for introducing the oligonucleotide of the present invention into a body to be subjected to introduction which is a cell or a tissue, a calcium phosphate method, an electroporation method, a lipofection method, viral infection, immersion in a 3L5-oligonucleotide solution, a transformation method, or the like is used. Examples of a method for introduction into an embryo include microinjection, an electroporation method, and viral infection. When the body to be subjected to introduction is a plant, injection or perfusion into the cavity or interstitial cells or the like of the plant, or a method of spraying is used. In the case of an animal, a method of systemic introduction by oral, topical, subcutaneous, intramuscular, intravenous, parenteral, transvaginal, transrectal, transnasal, transocular or transmembrane administration or the like, an electroporation method, or viral infection is used. As a method for oral introduction, a method can also be used in which the oligonucleotide of the present invention is directly mixed with food for an organism.

In the methods of introducing the oligonucleotide of the present invention into a patient, a colloidal dispersion system can be used in addition to the above. The colloidal dispersion system is expected to have an effect of improving the *in vivo* stability of a compound, or an effect of efficiently delivering a compound to a specific organ, tissue or cell. The colloidal dispersion system is not limited as long as it is normally used. Examples thereof can include lipid-based dispersion systems including polymer complexes, nanocapsules, microspheres, beads, oil-in-water emulsifiers, micelles, mixed micelles and liposomes. A plurality of liposomes and artificial membrane vesicles which have an effect of efficiently delivering a compound to a specific organ, tissue or cell are preferred (Mannino et al., Biotechniques, 1988, 6, p.682-; Blume and Cevc, Biochem. et Biophys. Acta, 1990, 1029, p.91-; Lappalainen et al., Antiviral Res., 1994, 23, p.119-; Chonn and Cullis, Current Op. Biotech., 1995, 6, p.698-). Single-membrane liposomes whose size is in the range of 0.2 to 0.4 µm can encapsulate a significant proportion of an aqueous buffer containing macromolecules, and a compound is encapsulated in the resulting aqueous inner membranes, and delivered in a biologically active form to brain cells (Fraley et al., Trends Biochem. Sci., 1981, 6, p. 77).

The liposome normally has a composition wherein a lipid, in particular, a phospholipid, in particular, a phospholipid having a high phase transition temperature, is combined with one or more steroids, in particular, cholesterols. Examples of the lipid useful for liposome production include phosphatidyl compounds such as phosphatidylglycerol, phosphatidylcholine, phosphatidylserine, sphingolipid, phosphatidylethanolamine, cerebroside and ganglioside.

Diacylphosphatidylglycerol is particularly useful. Here, the lipid moiety contains 14 to 18 carbon atoms, in particular, 16 to 18 carbon atoms, and is saturated (lacks a double bond in a chain of 14 to 18 carbon atoms) .

Typical phospholipids include phosphatidylcholine, dipalmitoyl phosphatidylcholine, and distearoyl phosphatidylcholine.

Targeting by a colloidal dispersion system of liposomes may be passive or active.

The passive targeting is achieved by using the intrinsic tendency of liposomes to be distributed to reticuloendothelial cells of an organ containing sinusoidal capillaries.

On the other hand, examples of active targeting can include bonding a specific ligand such as a viral protein coat (Morishita et al., Proc. Natl. Acad. Sci. (U.S.A), 1993. 90, p. 8474-), a monoclonal antibody (or an appropriate binding moiety thereof), a sugar, a glycolipid or a protein (or an oligopeptide fragment thereof) to liposomes, or a method in which liposomes are modified by changing the composition of the liposomes in order to achieve distribution to organs and cellular forms other than naturally occurring localization sites.

The surface of a targeted colloidal dispersion system can be modified in various ways. In a delivery system in which targeting is performed with liposomes, lipid groups can be incorporated into the lipid bilayers of liposomes in order to maintain a target ligand in close association with the lipid bilayers. Various linking groups can be used for linking a lipid chain to the target ligand.

A target ligand binding to a specific cell surface molecule that is predominantly found on cells for which delivery of the oligonucleotide of the present invention is desired can be, for example, (1) a hormone, a growth factor or an appropriate oligopeptide fragment thereof which is bound to a specific cell receptor that is predominantly expressed by cells for which the delivery is desired, or (2) a polyclonal or monoclonal antibody or an appropriate fragment thereof (for example, Fab or F(ab')2) which specifically binds to an antigenic epitope that is predominantly found on target cells. Two or more biologically active agents can also be combined in a single liposome, and administered.

A drug agent that improves the intracellular stability of contents and/or targeting can also be added to the colloidal dispersion system.

The amount of the oligonucleotide of the present invention or a pharmaceutically acceptable salt thereof used varies depending on the symptom, age and the like. It is desirable that it be administered to an adult one to three times a day, depending on the symptom, in an amount of not less than 1 mg (preferably 30 mg) and not more than 2000 mg (preferably 1500 mg) per dose in the case of oral administration, not less than 0.5 mg (preferably 5 mg) and not more than 500 mg (preferably 250 mg) per dose in the case of intravenous administration or subcutaneous administration, not less than 0.5 mg (preferably 5 mg) and not more than 500 mg (preferably 250 mg) in the case of intratracheal administration, and not less than 0.05 mg (preferably 0.5 mg) and not more than 10 mg (preferably 5 mg) in the case of intraocular administration. It is desirable that the administration be performed, depending on the symptom, one to three times a week in the case of the drug agent having improved stability, and one to three times a month in the case of the drug agent having further improved stability.

The pharmaceutical composition and formulation for topical administration include a transdermal patch, an ointment, a lotion, a cream, a gel, a lozenge, a suppository, a spray, a solution and a powder.

### Examples

### (Examples 1 to 52 and Reference Examples 1 to 3)

The present compounds were synthesized using a phosphoramidite method (Nucleic Acids research, 12, 4539 (1984), Nature Communications 6, Article number: 6317 (2015)). Specific sequences and structures are shown in Tables 1-1 to 1-10. The compound of each of Examples 1 to 17 and 48 to 52 and Reference Examples 2 and 3 is siRNA whose target gene is mRNA for a gene encoding human transferrin receptor 2 (TfR2). The compound of each of Examples 1 to 4 and Reference Example 2 and the compound of each of Examples 5 to 17 and Reference Example 3 are siRNAs whose target sequences are nucleotide sequences consisting of 19 nucleotides from positions 1536 to 1554 and from positions 2847 to 2865, respectively, in the nucleotide sequence of human TfR2 (hTfR2) mRNA set forth as SEQ ID NO: 1, and which have U(M)U(M) bound as an overhang structure to the 3'-terminus of each antisense strand region. The compounds of Examples 18 to 47 and Reference Example 1 are siRNAs in which various chemical modifications are applied to 19 base pairs (that is, a complementary region) in AD-47882 as siRNA for mouse TfR2 (mTfR2) which is described in WO 2012/177921, and which have U(M)U(M) bound as an overhang structure to the 3'-terminus of each antisense strand region.

The compounds of Example 48 (TfR2-019. 94DUG. s1) and Example 49 (TfR2-019. 94DUG. s2) are siRNAs in which each of the overhang structures at the 3'-termini of the antisense strand regions in the compound of Example 16 (TfR2-019. 94DUG) is one nucleotide, or is not present. The compounds of Example 50 (TfR2-019. 94DUG. e1) and Example 51 (TfR2-019. 94DUG. e2) are siRNAs whose target sequences are sequences obtained by extending the target sequence in the compound of Example 16 (TfR2-019. 94DUG) toward the 5'-terminus side by 1 and 2 nucleotides in chain length, respectively (that is, the sequence from positions 2846 to 2865 and the sequence from positions 2845 to 2865, respectively, of SEQ ID NO: 1), and which have complementary region chain lengths of 20 bp and 21 bp, respectively. The compound of Example 52 (TfR2-019. 94DUG. e3) is siRNA in which the overhang structure at the 3'-terminus of the antisense strand region in the compound of Example 16 (TfR2-019. 94DUG) is G(M)U(M).

The moiety "2'-O-methylnucleoside" was synthesized using a phosphoramidite described in Nucleic Acids Research 17, 3373 (1989). The moiety "DNA" was synthesized using a phosphoramidite described in Nucleic Acids Research 11, 4539 (1984). The moiety "3'-O-methylnucleoside" was synthesized using 3'-0-Methyl Adenosine (n-bz) CED phosphoramidite (ChemGene, catalog No: ANP-2901), 3'-O-Methyl Cytidine (n-bz) CED phosphoramidite (ChemGene, catalog No: ANP-2902), 3'-O-Methyl Guanosine (n-ibu) CED phosphoramidite (ChemGene, catalog No: ANP-2903), or 3'-O-Methyl Uridine CED phosphoramidite (ChemGene, catalog No: ANP-2904). The moiety "2'-O,4'-C-methylenenucleoside" was synthesized using a phosphoramidite described in WO 99/14226. The moiety "2'-deoxy-2'-fluoronucleoside" was synthesized using a phosphoramidite described in J. Med. Chem. 36, 831 (1993). The moiety "2'-O-methoxyethylnucleoside" was synthesized using a phosphoramidite described in Helv. Chim. Acta 78, 486-504 (1995). The moiety "GN" was synthesized using a phosphoramidite described in WO 2019/172286, Reference Example 41. The present compounds were identified by negative ion ESI mass spectrometry. Table 1 shows the results.

In the compound of each of Examples and Reference Examples, a sense strand region consisting of a nucleotide sequence shown in Tables 1-1 to 1-7 and an antisense strand region consisting of a nucleotide sequence shown in Tables 1-2 to 1-6 and 1-8, each of which had been separately synthesized, were put in equimolar amounts into a tube, annealed, and then confirmed to form a duplex by using an unmodified gel or HPLC.

**[Table 1-1]**

| Name of sense strand | Nucleotide sequence of sense strand (5'-3') | Molecular weight | Theoretical value | SEQ ID NO |
|---|---|---|---|---|
| TfR2-039. 23SG | GNA(M)^C(M)^C(M)U(M)C(F)A(M)A(F)A(F)G(F)C(M)C(M)G(M)U(M)A(M)G(M)U(M)G(M)^U(M)^A(3M) | 7228.28 | 7228.32 | 2 |
| TfR2-019. 22SG | GNC(M)^G(M)^U(M)G(M)G(F)A(M)G(F)U(F)U(F)U(M)C(M)A(M)A(M)U(M)A(M)U(M)C(M)^A(M)^A(3M) | 7230.30 | 7230.28 | 9 |
| AD47882. 23SG | GNC(M)^C(M)^A(M)C(M)G(F)U(M)G(F)A(F)U(F)U(M)C(M)U(M)C(M)C(M)U(M)U(M)U(M)^C(M)^U(3M) | 7056.15 | 7056.20 | 12 |

**[Table 1-2]**

| Example | Name of compound | Name of sense strand | Name of antisense strand | Nucleotide sequence of antisense strand (5'-3') | Molecular weight | Theoretical value | SEQ ID NO |
|---|---|---|---|---|---|---|---|
| 1 | TfR2-039. 41DUG | TfR2-039. 23SG | TfR2-039. 41AS | U(M)^A(F)^C(M)A(M)C(M)U(F)A(M)C(F)G(M)G(M)C(M)T(D)U(M)U(F)G(M )A(F)G(M)G(M)^U(M)^U(M)^U(M) | 6949.06 | 6949.79 | 3 |
| 2 | TfR2-039. 42DUG | TfR2-039. 23SG | TfR2-039. 42AS | U(M)^A(F)^C(M)A(M)C(M)U(F)A(M)C(F)G(M)G(M)C(M)T(D)^U(M)U(F)G( M)A(F)G(M)G(M)^U(M)^U(M)^U(M) | 6964.35 | 6965.86 | 3 |
| 3 | TfR2-039. 43DUG | TfR2-039. 23SG | TfR2-039. 43AS | U(M)^A(F)^C(M)A(M)C(M)U(F)A(M)C(F)G(M)G(M)C(E)U(M)U(M)U(F)G(M )A(F)G(M)G(M)^U(M)^U(M)^U(M) | 6994.48 | 6991.83 | 4 |
| 4 | TfR2-039. 45DUG | TfR2-039. 23SG | TfR2-039. 45AS | U(M)^A(F)^C(M)A(M)C(M)U(F)A(M)C(F)G(M)G(M)C(M)U(M)T(E)U(F)G(M )A(F)G(M)G(M)^U(M)^U(M)^U(M) | 6994.74 | 6991.82 | 5 |
| 5 | TfR2-019. 85DUG | TfR2-019. 22SG | TfR2-019. 85AS | U(M)^U(F)^G(M)A(M)U(M)A(F)U(M)U(M)G(F)A(M)A(M)A(M)C(E)U(F)C(M )C(F)A(M)C(M)^G(M)^U(M)^U(M) | 6955.94 | 6956.00 | 10 |
| 6 | TfR2-019. 86DUG | TfR2-019. 22SG | TfR2-019. 86AS | U(M)^U(F)^G(M)A(M)U(M)A(F)U(M)U(M)G(F)A(M)A(E)A(M)C(M)U(F)C(M )C(F)A(M)C(M)^G(M)^U(M)^U(M) | 6941.94 | 6941.99 | 10 |
| 7 | TfR2-019. 87DUG | TfR2-019. 22SG | TfR2-019. 87AS | U(M)^U(F)^G(M)A(M)U(M)A(F)U(M)U(M)G(F)A(M)A(M)A(D)C(M)U(F)C(M )C(F)A(M)C(M)^G(M)^U(M)^U(M) | 6899.95 | 6899.98 | 10 |
| 8 | TfR2-019. | TfR2-019. | TfR2-019. | U(M)^U(F)^G(M)A(M)U(M)A(F)U(M)U(F)G(F)A(M)A(M)A(D)C(M)T(E)C(M) | 6926.06 | 6925.99 | 11 |
| | 71DUG | 22SG | 71AS | C(F)A(M)C(M)^G(M)^U(M)^U(M) | | | |
| 9 | TfR2-019. 88DUG | TfR2-019. 22SG | TfR2-019. 88AS | U(M)^U(F)^G(M)A(F)U(M)A(F)U(M)U(M)G(F)A(M)A(M)A(D)C(M)T(E)C(M) C(F)A(M)C(M)^G(M)^U(M)^U(M) | 6926.05 | 6925.99 | 11 |
| 10 | TfR2-019. 89DUG | TfR2-019. 22SG | TfR2-019. 89AS | U(M)^U(F)^G(M)A(M)U(M)A(F)U(M)U(M)G(F)A(F)A(M)A(D)C(M)T(E)C(M) C(F)A(M)C(M)^G(M)^U(M)^U(M) | 6926.11 | 6925.99 | 11 |

**[Table 1-3]**

| Example | Name of compound | Name of sense strand | Name of antisense strand | Nucleotide sequence of antisense strand (5'-3') | Molecular weight | Theoretical value | SEQ ID NO |
|---|---|---|---|---|---|---|---|
| 11 | TfR2-019. 90DUG | TfR2-019. 22SG | TfR2-019. 90AS | U(M)^U(F)^G(M)A(M)U(M)A(F)U(M)U(M)G(F)A(M)A(M)A(D)C(M)T(E)C(M )C(F)A(M)C(F)^G(M)^U(M)^U(M) | 6926.00 | 6925.99 | 11 |
| 12 | TfR2-019. 80DUG | TfR2-019. 22SG | TfR2-019. 80AS | U(M)^U(F)^G(M)A(M)U(M)A(F)U(M)U(F)G(F)A(M)A(M)A(M)C(E)U(R)^C( M)C(F)A(M)C(M)^G(M)^U(M)^U(M) | 6958.09 | 6957.96 | 10 |
| 13 | TfR2-019. 91DUG | TfR2-019. 22SG | TfR2-019. 91AS | U(M)^U(F)^G(M)A(M)U(M)A(F)U(M)U(M)G(F)A(F)A(M)A(M)C(E)U(R)^C( M)C(F)A(M)C(M)^G(M)^U(M)^U(M) | 6957.96 | 6957.96 | 10 |
| 14 | TfR2-019. 92DUG | TfR2-019. 22SG | TfR2-019. 92AS | U(M)^U(F)^G(M)A(M)U(M)A(F)U(M)U(M)G(F)A(M)A(F)A(M)C(E)U(R)^C( M)C(F)A(M)C(M)^G(M)^U(M)^U(M) | 6958.07 | 6957.96 | 10 |
| 15 | TfR2-019. 93DUG | TfR2-019. 22SG | TfR2-019. 93AS | U(M)^U(F)^G(M)A(M)U(M)A(F)U(M)U(M)G(F)A(M)A(M)A(F)C(E)U(R)^C( M)C(F)A(M)C(M)^G(M)^U(M)^U(M) | 6958.06 | 6957.96 | 10 |
| 16 | TfR2-019. 94DUG | TfR2-019. 22SG | TfR2-019. 94AS | U(M)^U(F)^G(M)A(M)U(M)A(F)U(M)U(M)G(F)A(M)A(F)A(M)C(M)U(R)^C( M)C(F)A(M)C(M)^G(M)^U(M)^U(M) | 6935.66 | 6935.69 | 10 |
| 17 | TfR2-019. 95DUG | TfR2-019. 22SG | TfR2-019. 95AS | U(M)^U(F)^G(M)A(M)U(M)A(F)U(M)U(M)G(F)A(M)A(M)A(F)C(M)U(R)^C( M)C(F)A(M)C(M)^G(M)^U(M)^U(M) | 6935.68 | 6935.69 | 10 |
| 18 | AD47882. 41DUG | AD47882. 23SG | AD47882. 41AS | A(M)^G(F)^A(M)A(M)A(M)G(F)G(M)A(F)G(M)A(M)A(M)T(D)C(M)A(F)C(M) G(F)U(M)G(M)^G(M)^U(M)^U(M) | 7119.06 | 7118.1 | 13 |
| 19 | AD47882. 42DUG | AD47882. 23SG | AD47882. 42AS | A(M)^G(F)^A(M)A(M)A(M)G(F)G(M)A(F)G(M)A(M)A(M)T(D)^C(M)A(F)C( M)G(F)U(M)G(M)^G(M)^U(M)^U(M) | 7134.05 | 7134.08 | 13 |
| 20 | AD47882. 43DUG | AD47882. 23SG | AD47882. 43AS | A(M)^G(F)^A(M)A(M)A(M)G(F)G(M)A(F)G(M)A(M)A(E)U(M)C(M)A(F)C(M) G(F)U(M)G(M)G(M)^U(M)^U(M) | 7147.06 | 7146.10 | 14 |
| 21 | AD47882. 44DUG | AD47882. 23SG | AD47882. 44AS | A(M)^G(F)^A(M)A(M)A(M)G(F)G(M)A(F)G(M)A(M)A(L)U(M)C(M)A(F)C(M) G(F)U(M)G(M)G(M)^U(M)^U(M) | 7133.06 | 7132.08 | 14 |

**[Table 1-4]**

| Example | Name of compound | Name of sense strand | Name of antisense strand | Nucleotide sequence of antisense strand (5'-3') | Molecular weight | Theoretical value | SEQ ID NO |
|---|---|---|---|---|---|---|---|
| 22 | AD47882. 45DUG | AD47882. 23SG | AD47882. 45AS | A(M)^G(F)^A(M)A(M)A(M)G(F)G(M)A(F)G(M)A(M)A(M)U(M)C(E)A(F)C(M) G(F)U(M)G(M)G(M)^U(M)^U(M) | 7161.08 | 7160.11 | 14 |
| 23 | AD47882. 46DUG | AD47882. 23SG | AD47882. 46AS | A(M)^G(F)^A(M)A(M)A(M)G(F)G(M)A(F)G(M)A(M)A(M)U(M)C(L)A(F)C(M) G(F)U(M)G(M)G(M)^U(M)^U(M) | 7148.09 | 7146.10 | 14 |
| 24 | AD47882. 47DUG | AD47882. 23SG | AD47882. 47AS | A(M)^G(F)^A(M)A(M)A(M)G(F)G(M)A(F)G(M)A(M)A(m)U(M)C(M)A(F)C(M )G(F)U(M)G(M)G(M)^U(M)^U(M) | 7178.11 | 7178.12 | 14 |
| 25 | AD47882. 48DUG | AD47882. 23SG | AD47882. 48AS | A(M)^G(F)^A(M)A(M)A(M)G(F)G(M)A(F)G(M)A(M)A(M)U(M)C(m)A(F)C(M )G(F)U(M)G(M)G(M)^U(M)^U(M) | 7192.09 | 7192.14 | 14 |
| 26 | AD47882. 70DUG | AD47882. 23SG | AD47882. 70AS | A(M)^G(F)^A(M)A(M)A(M)G(F)G(M)A(F)G(M)A(M)A(M)U(F)C(M)A(E)C(M) G(F)U(M)G(M)^G(M)^U(M)^U(M) | 7146.09 | 7146.10 | 14 |
| 27 | AD47882. 71DUG | AD47882. 23SG | AD47882. 71AS | A(M)^G(F)^A(M)A(M)A(M)G(F)G(M)A(F)G(F)A(M)A(M)T(D)C(M)A(E)C(M) G(F)U(M)G(M)^G(M)^U(M)^U(M) | 7130.08 | 7130.10 | 13 |
| 28 | AD47882. 72DUG | AD47882. 23SG | AD47882. 72AS | A(M)^G(F)^A(M)A(M)A(M)G(F)G(M)A(F)G(F)A(M)A(E)U(M)C(M)A(E)C(M) G(F)U(M)G(M)^G(M)^U(M)^U(M) | 7158.07 | 7158.10 | 14 |
| 29 | AD47882. 73DUG | AD47882. 23SG | AD47882. 73AS | A(M)^G(F)^A(M)A(M)A(M)G(F)G(M)A(F)G(F)A(M)A(M)U(M)C(E)A(E)C(M) G(F)U(M)G(M)^G(M)^U(M)^U(M) | 7172.10 | 7172.11 | 14 |
| 30 | AD47882. 74DUG | AD47882. 23SG | AD47882. 74AS | A(M)^G(F)^A(M)A(M)A(M)G(F)G(M)A(F)G(F)A(M)A(M)U(M)C(m)A(E)C(M) G(F)U(M)G(M)^G(M)^U(M)^U(M) | 7204.09 | 7204.14 | 14 |
| 31 | AD47882. 75DUG | AD47882. 23SG | AD47882. 75AS | A(M)^G(F)^A(M)A(M)A(M)G(F)G(M)A(F)G(F)A(M)A(M)U(M)C(E)A(F)C(M) G(F)U(M)G(M)^G(M)^U(M)^U(M) | 7148.04 | 7148.09 | 14 |
| 32 | AD47882. 76DUG | AD47882. 23SG | AD47882. 76AS | A(M)^G(F)^A(M)A(M)A(M)G(F)G(M)A(F)G(F)A(M)A(M)U(M)C(E)A(R)C(M) G(F)U(M)G(M)^G(M)^U(M)^U(M) | 7146.06 | 7146.10 | 14 |

**[Table 1-5]**

| Example | Name of compound | Name of sense strand | Name of antisense strand | Nucleotide sequence of antisense strand (5'-3') | Molecular weight | Theoretical value | SEQ ID NO |
|---|---|---|---|---|---|---|---|
| 33 | AD47882. 77DUG | AD47882. 23SG | AD47882. 77AS | A(M)^G(F)^A(M)A(M)A(M)G(F)G(M)A(F)G(F)A(M)A(M)U(M)C(E)A(R)C(E) G(F)U(M)G(M)^G(M)^U(M)^U(M) | 7172.08 | 7172.11 | 14 |
| 34 | AD47882. 78DUG | AD47882. 23SG | AD47882. 78AS | A(M)^G(F)^A(M)A(M)A(M)G(F)G(M)A(F)G(F)A(M)A(M)U(M)C(E)A(R)C(L) G(F)U(M)G(M)^G(M)^U(M)^U(M) | 7158.08 | 7158.10 | 14 |
| 35 | AD47882. 79DUG | AD47882. 23SG | AD47882. 79AS | A(M)^G(F)^A(M)A(M)A(M)G(F)G(M)A(F)G(F)A(M)A(M)U(M)C(E)A(R)C(m) G(F)U(M)G(M)^G(M)^U(M)^U(M) | 7204.12 | 7204.14 | 14 |
| 36 | AD47882. 80DUG | AD47882. 23SG | AD47882. 80AS | A(M)^G(F)^A(M)A(M)A(M)G(F)G(M)A(F)G(F)A(M)A(M)U(M)C(E)A(R)^C(M )G(F)U(M)G(M)^G(M)^U(M)^U(M) | 7162.04 | 7162.07 | 14 |
| 37 | AD47882. 81DUG | AD47882. 23SG | AD47882. 81AS | A(M)^G(F)^A(M)A(M)A(M)G(F)G(M)A(F)G(F)A(M)A(M)U(M)C(E)A(R)^C(E )G(F)U(M)G(M)^G(M)^U(M)^U(M) | 7188.05 | 7188.09 | 14 |
| 38 | AD47882. 82DUG | AD47882. 23SG | AD47882. 82AS | A(M)^G(F)^A(M)A(M)A(M)G(F)G(M)A(F)G(F)A(M)A(M)U(M)C(E)A(R)^C(L )G(F)U(M)G(M)^G(M)^U(M)^U(M) | 7174.06 | 7174.07 | 14 |
| 39 | AD47882. 83DUG | AD47882. 23SG | AD47882. 83AS | A(M)^G(F)^A(M)A(M)A(M)G(F)G(M)A(F)G(F)A(M)A(M)U(M)C(E)A(R)^C(m )G(F)U(M)G(M)^G(M)^U(M)^U(M) | 7220.08 | 7220.12 | 14 |
| 40 | AD47882. 84DUG | AD47882. 23SG | AD47882. 84AS | A(M)^G(F)^A(M)A(M)A(M)G(F)G(M)A(F)G(F)A(M)A(M)U(M)C(E)A(M)C(M) G(F)U(M)G(M)^G(M)^U(M)^U(M) | 7160.10 | 7160.11 | 14 |
| 41 | AD47882. 88DUG | AD47882. 23SG | AD47882. 88AS | A(M)^G(F)^A(M)A(F)A(M)G(F)G(M)A(M)G(F)A(M)A(M)T(D)C(M)A(E)C(M) G(F)U(M)G(M)^G(M)^U(M)^U(M) | 7130.10 | 7130.10 | 13 |
| 42 | AD47882. 89DUG | AD47882. 23SG | AD47882. 89AS | A(M)^G(F)^A(M)A(M)A(M)G(F)G(M)A(M)G(F)A(F)A(M)T(D)C(M)A(E)C(M) G(F)U(M)G(M)^G(M)^U(M)^U(M) | 7130.11 | 7130.10 | 13 |
| 43 | AD47882. 90DUG | AD47882. 23SG | AD47882. 90AS | A(M)^G(F)^A(M)A(M)A(M)G(F)G(M)A(M)G(F)A(M)A(M)T(D)C(M)A(E)C(M) G(F)U(M)G(F)^G(M)^U(M)^U(M) | 7130.10 | 7130.10 | 13 |

**[Table 1-6]**

| Example | Name of compound | Name of sense strand | Name of antisense strand | Nucleotide sequence of antisense strand (5'-3') | Molecular weight | Theoretical value | SEQ ID NO |
|---|---|---|---|---|---|---|---|
| 44 | AD47882. 91DUG | AD47882. 23SG | AD47882. 91AS | A(M)^G(F)^A(M)A(M)A(M)G(F)G(M)A(M)G(F)A(F)A(M)U(M)C(E)A(R)^C(M )G(F)U(M)G(M)^G(M)^U(M)^U(M) | 7162.09 | 7162.07 | 14 |
| 45 | AD47882. 92DUG | AD47882. 23SG | AD47882. 92AS | A(M)^G(F)^A(M)A(M)A(M)G(F)G(M)A(M)G(F)A(M)A(F)U(M)C(E)A(R)^C(M )G(F)U(M)G(M)^G(M)^U(M)^U(M) | 7162.09 | 7162.07 | 14 |
| 46 | AD47882. 93DUG | AD47882. 23SG | AD47882. 93AS | A(M)^G(F)^A(M)A(M)A(M)G(F)G(M)A(M)G(F)A(M)A(M)U(F)C(E)A(R)^C(M )G(F)U(M)G(M)^G(M)^U(M)^U(M) | 7162.05 | 7162.07 | 14 |
| 47 | AD47882. 94DUG | AD47882. 23SG | AD47882. 94AS | A(M)^G(F)^A(M)A(M)A(M)G(F)G(M)A(M)G(F)A(M)A(F)U(M)C(M)A(R)^C( M)G(F)U(M)G(M)^G(M)^U(M)^U(M) | 7136.04 | 7136.06 | 14 |
| Reference Example 1 | AD47882. 23DUG | AD47882. 23SG | AD47882. 9AS | A(M)^G(F)^A(M)A(M)A(M)G(F)G(M)A(F)G(M)A(M)A(M)U(M)C(M)A(F)C(M )G(F)U(M)G(M)G(M)^U(M)^U(M) | 7118.03 | 7118.12 | 14 |
| Reference Example 2 | TfR2-039. 23DUG | TfR2-039. 23SG | TfR2-039. 9AS | U(M)^A(F)^C(M)A(M)C(M)U(F)A(M)C(F)G(M)G(M)C(M)U(M)U(M)U(F)G( M)A(F)G(M)G(M)U(M)^U(M)^U(M) | 6950.0 | 6949.72 | 4 |
| Reference Example 3 | TfR2-019. 22DUG | TfR2-019. 22SG | TfR2-019. 22AS | U(M)^U(F)^G(M)A(M)U(M)A(F)U(M)U(M)G(F)A(M)A(M)A(M)C(M)U(F)C(M )C(F)A(M)C(M)G(M)^U(M)^U(M) | 6918.0 | 6917.74 | 10 |

**[Table 1-7]**

| Example | Name of compound | Name of sense strand | Nucleotide sequence of sense strand region (5'-3') | Molecular weight | Theoretical value | SEQ ID NO |
|---|---|---|---|---|---|---|
| 48 | TfR2-019. 94DUG.s1 | TfR2-019. 22SG | GNC(M)^G(M)^U(M)G(M)G(F)A(M)G(F)U(F)U(F)U(M)C(M)A(M)A(M)U(M)A(M)U(M)C(M)^A(M)^A(3M) | 7230.30 | 7230.28 | 9 |
| 49 | TfR2-019. 94DUG.s2 | TfR2-019. 22SG | GNC(M)^G(M)^U(M)G(M)G(F)A(M)G(F)U(F)U(F)U(M)C(M)A(M)A(M)U(M)A(M)U(M)C(M)^A(M)^A(3M) | 7230.30 | 7230.28 | 9 |
| 50 | TfR2-019. 94DUG.e1 | TfR2-019. 22SG.e1 | GNG(M)^C(M)^G(M)U(M)G(M)G(F)A(M)G(F)U(F)U(F)U(M)C(M)A(M)A(M)U(M)A(M)U(M)C(M)^A(M)^A( 3M) | 7589.35 | 7589.35 | 15 |
| 51 | TfR2-019. 94DUG.e2 | TfR2-019. 22SG.e2 | GNG(M)^G(M)^C(M)G(M)U(M)G(M)G(F)A(M)G(F)U(F)U(F)U(M)C(M)A(M)A(M)U(M)A(M)U(M)C(M)^A( M)^A(3M) | 7948.42 | 7948.41 | 16 |
| 52 | TfR2-019. 94DUG.e3 | TfR2-019. 22SG | GNC(M)^G(M)^U(M)G(M)G(F)A(M)G(F)U(F)U(F)U(M)C(M)A(M)A(M)U(M)A(M)U(M)C(M)^A(M)^A(3M) | 7230.30 | 7230.28 | 9 |

**[Table 1-8]**

| Example | Name of compound | Name of antisense strand | Nucleotide sequence of antisense strand region (5'-3') | Molecular weight | Theoretical value | SEQ ID NO |
|---|---|---|---|---|---|---|
| 48 | TfR2-019. 94DUG.s1 | TfR2-019. 94AS.s1 | U(M)^U(F)^G(M)A(M)U(M)A(F)U(M)U(M)G(F)A(M)A(F)A(M)C(M)U(R)^C(M)C(F)A(M)C(M)^G(M)^U( M) | 6599 | 6599.58 | 17 |
| 49 | TfR2-019. 94DUG.s2 | TfR2-019. 94AS.s2 | U(M)^U(F)^G(M)A(M)U(M)A(F)U(M)U(M)G(F)A(M)A(F)A(M)C(M)U(R)^C(M)C(F)A(M)C(M)^G(M) | 6263 | 6263.31 | 18 |
| 50 | TfR2-019. 94DUG.e1 | TfR2-019. 94AS.e1 | U(M)^U(F)^G(M)A(M)U(M)A(F)U(M)U(M)G(F)A(M)A(F)A(M)C(M)U(R)^C(M)C(F)A(M)C(M)G(M)^C(M )^U(M)^U(M) | 7254 | 7255.06 | 19 |
| 51 | TfR2-019. 94DUG.e2 | TfR2-019. 94AS.e2 | U(M)^U(F)^G(M)A(M)U(M)A(F)U(M)U(M)G(F)A(M)A(F)A(M)C(M)U(R)^C(M)C(F)A(M)C(M)G(M)C(M) ^C(M)^U(M)^U(M) | 7574 | 7574.27 | 20 |
| 52 | TfR2-019. 94DUG.e3 | TfR2-019. 94AS.e3 | U(M)^U(F)^G(M)A(M)U(M)A(F)U(M)U(M)G(F)A(M)A(F)A(M)C(M)U(R)^C(M)C(F)A(M)C(M)^G(M)^G( M)^U(M) | 6974 | 6974.88 | 21 |

**[Table 1-9]**

| Name of sense strand | Nucleotide sequence of sense strand (5'-3') | SEQ ID NO |
|---|---|---|
| TfR2-039. 23SG | GNA(M)^C(M)^C(M)U(M)C(F)A(M)A(F)A(F)G(F)C(M)C(M)G(M)U(M)A(M)G(M)U(M)G(M)^U(M)^A(3M) | 2 |
| TfR2-019. 22SG | GNC(M)^G(M)^U(M)G(M)G(F)A(M)G(F)U(F)U(F)U(M)C(M)A(M)A(M)U(M)A(M)U(M)C(M)^A(M)^A(3M) | 9 |
| AD47882. 23SG | GNC(M)^C(M)^A(M)C(M)G(F)U(M)G(F)A(F)U(F)U(M)C(M)U(M)C(M)C(M)U(M)U(M)U(M)^C(M)^U(3M) | 12 |
| TfR2-019. 22SG.e1 | GNG(M)^C(M)^G(M)U(M)G(M)G(F)A(M)G(F)U(F)U(F)U(M)C(M)A(M)A(M)U(M)A(M)U(M)C(M)^A(M)^A(3M) | 15 |
| TfR2-019. 22SG.e2 | GNG(M)^G(M)^C(M)G(M)U(M)G(M)G(F)A(M)G(F)U(F)U(F)U(M)C(M)A(M)A(M)U(M)A(M)U(M)C(M)^A(M)^A(3M) | 16 |

**[Table 1-10]**

| Name of antisense strand | Nucleotide sequence of antisense strand (5'-3') | SEQ ID NO |
|---|---|---|
| TfR2-039. 41AS | U(M)^A(F)^C(M)A(M)C(M)U(F)A(M)C(F)G(M)G(M)C(M)T(D)U(M)U(F)G(M)A(F)G(M)G(M)^U(M)^U(M)^U(M) | 3 |
| TfR2-039. 42AS | U(M)^A(F)^C(M)A(M)C(M)U(F)A(M)C(F)G(M)G(M)C(M)T(D)^U(M)U(F)G(M)A(F)G(M)G(M)^U(M)^U(M)^U(M) | 3 |
| TfR2-039. 43AS | U(M)^A(F)^C(M)A(M)C(M)U(F)A(M)C(F)G(M)G(M)C(E) U(M)U(M)U(F)G(M)A(F)G(M)G(M)^U(M)^U(M)^U(M) | 4 |
| TfR2-039. 45AS | U(M)^A(F)^C(M)A(M)C(M)U(F)A(M)C(F)G(M)G(M)C(M)U(M)T(E)U(F)G(M)A(F)G(M)G(M)^U(M)^U(M)^U(M) | 5 |
| TfR2-019. 85AS | U(M)^U(F)^G(M)A(M)U(M)A(F)U(M)U(M)G(F)A(M)A(M)A(M)C(E)U(F)C(M)C(F)A(M)C(M)^G(M)^U(M)^U(M) | 10 |
| TfR2-019. 86AS | U(M)^U(F)^G(M)A(M)U(M)A(F)U(M)U(M)G(F)A(M)A(E)A(M)C(M)U(F)C(M)C(F)A(M)C(M)^G(M)^U(M)^U(M) | 10 |
| TfR2-019. 87AS | U(M)"U(F)"G(M)A(M)U(M)A(F)U(M)U(M)G(F)A(M)A(M)A(D)C(M)U(F)C(M)C(F)A(M)C(M)"G(M)"U(M)"U(M) | 10 |
| TfR2-019. 71AS | U(M)^U(F)^G(M)A(M)U(M)A(F)U(M)U(F)G(F)A(M)A(M)A(D)C(M)T(E)C(M)C(F)A(M)C(M)^G(M)^U(M)^U(M) | 11 |

**[Table 1-11]**

| Name of antisense strand | Nucleotide sequence of antisense strand (5'-3') | SEQ ID NO |
|---|---|---|
| TfR2-019. 88AS | U(M)^U(F)^G(M)A(F)U(M)A(F)U(M)U(M)G(F)A(M)A(M)A(D)C(M)T(E)C(M)C(F)A(M)C(M)^G(M)^U(M)^U(M) | 11 |
| TfR2-019. 89AS | U(M)^U(F)^G(M)A(M)U(M)A(F)U(M)U(M)G(F)A(F)A(M)A(D)C(M)T(E)C(M)C(F)A(M)C(M)^G(M)^U(M)^U(M) | 11 |
| TfR2-019. 90AS | U(M)^U(F)^G(M)A(M)U(M)A(F)U(M)U(M)G(F)A(M)A(M)A(D)C(M)T(E)C(M)C(F)A(M)C(F)^G(M)^U(M)^U(M) | 11 |
| TfR2-019. 80AS | U(M)^U(F)^G(M)A(M)U(M)A(F)U(M)U(F)G(F)A(M)A(M)A(M)C(E)U(R)^C(M)C(F)A(M)C(M)^G(M)^U(M)^U(M) | 10 |
| TfR2-019. 91AS | U(M)^U(F)^G(M)A(M)U(M)A(F)U(M)U(M)G(F)A(F)A(M)A(M)C(E)U(R)^C(M)C(F)A(M)C(M)^G(M)^U(M)^U(M) | 10 |
| TfR2-019. 92AS | U(M)^U(F)^G(M)A(M)U(M)A(F)U(M)U(M)G(F)A(M)A(F)A(M)C(E)U(R)^C(M)C(F)A(M)C(M)^G(M)^U(M)^U(M) | 10 |
| TfR2-019. 93AS | U(M)^U(F)^G(M)A(M)U(M)A(F)U(M)U(M)G(F)A(M)A(M)A(F)C(E)U(R)^C(M)C(F)A(M)C(M)^G(M)^U(M)^U(M) | 10 |
| TfR2-019. 94AS | U(M)^U(F)^G(M)A(M)U(M)A(F)U(M)U(M)G(F)A(M)A(F)A(M)C(M) U(R)^C(M)C(F)A(M)C(M)^G(M)^U(M)^U(M) | 10 |

**[Table 1-12]**

| Name of antisense strand | Nucleotide sequence of antisense strand (5'-3') | SEQ ID NO |
|---|---|---|
| TfR2-019. 95AS | U(M)^U(F)^G(M)A(M)U(M)A(F)U(M)U(M)G(F)A(M)A(M)A(F)C(M)U(R)^C(M)C(F)A(M)C(M)^G(M)^U(M)^U(M) | 10 |
| AD47882. 41AS | A(M)^G(F)^A(M)A(M)A(M)G(F)G(M)A(F)G(M)A(M)A(M)T(D)C(M)A(F)C(M)G(F)U(M)G(M)^G(M)^U(M)^U(M) | 13 |
| AD47882. 42AS | A(M)^G(F)^A(M)A(M)A(M)G(F)G(M)A(F)G(M)A(M)A(M)T(D)^C(M)A(F)C(M)G(F)U(M)G(M)^G(M)^U(M)^U(M) | 13 |
| AD47882. 43AS | A(M)^G(F)^A(M)A(M)A(M)G(F)G(M)A(F)G(M)A(M)A(E)U(M)C(M)A(F)C(M)G(F)U(M)G(M)G(M)^U(M)^U(M) | 14 |
| AD47882. 44AS | A(M)^G(F)^A(M)A(M)A(M)G(F)G(M)A(F)G(M)A(M)A(L)U(M)C(M)A(F)C(M)G(F)U(M)G(M)G(M)^U(M)^U(M) | 14 |
| AD47882. 45AS | A(M)^G(F)^A(M)A(M)A(M)G(F)G(M)A(F)G(M)A(M)A(M)U(M)C(E)A(F)C(M)G(F)U(M)G(M)G(M)^U(M)^U(M) | 14 |
| AD47882. 46AS | A(M)^G(F)^A(M)A(M)A(M)G(F)G(M)A(F)G(M)A(M)A(M)U(M)C(L)A(F)C(M)G(F)U(M)G(M)G(M)^U(M)^U(M) | 14 |
| AD47882. 47AS | A(M)^G(F)^A(M)A(M)A(M)G(F)G(M)A(F)G(M)A(M)A(m)U(M)C(M)A(F)C(M)G(F)U(M)G(M)G(M)^U(M)^U(M) | 14 |

**[Table 1-13]**

| Name of antisense strand | Nucleotide sequence of antisense strand (5'-3') | SEQ ID NO |
|---|---|---|
| AD47882. 48AS | A(M)^G(F)^A(M)A(M)A(M)G(F)G(M)A(F)G(M)A(M)A(M)U(M)C(m)A(F)C(M)G(F)U(M)G(M)G(M)^U(M)^U(M) | 14 |
| AD47882. 70AS | A(M)^G(F)^A(M)A(M)A(M)G(F)G(M)A(F)G(M)A(M)A(M)U(F)C(M)A(E)C(M)G(F)U(M)G(M)^G(M)^U(M)^U(M) | 14 |
| AD47882. 71AS | A(M)^G(F)^A(M)A(M)A(M)G(F)G(M)A(F)G(F)A(M)A(M)T(D)C(M)A(E)C(M)G(F)U(M)G(M)^G(M)^U(M)^U(M) | 13 |
| AD47882. 72AS | A(M)^G(F)^A(M)A(M)A(M)G(F)G(M)A(F)G(F)A(M)A(E)U(M)C(M)A(E)C(M)G(F)U(M)G(M)^G(M)^U(M)^U(M) | 14 |
| AD47882. 73AS | A(M)^G(F)^A(M)A(M)A(M)G(F)G(M)A(F)G(F)A(M)A(M)U(M)C(E)A(E)C(M)G(F)U(M)G(M)^G(M)^U(M)^U(M) | 14 |
| AD47882. 74AS | A(M)^G(F)^A(M)A(M)A(M)G(F)G(M)A(F)G(F)A(M)A(M)U(M)C(m)A(E)C(M)G(F)U(M)G(M)^G(M)^U(M)^U(M) | 14 |
| AD47882. 75AS | A(M)^G(F)^A(M)A(M)A(M)G(F)G(M)A(F)G(F)A(M)A(M)U(M)C(E)A(F)C(M)G(F)U(M)G(M)^G(M)^U(M)^U(M) | 14 |
| AD47882. 76AS | A(M)^G(F)^A(M)A(M)A(M)G(F)G(M)A(F)G(F)A(M)A(M)U(M)C(E)A(R)C(M)G(F)U(M)G(M)^G(M)^U(M)^U(M) | 14 |

**[Table 1-14]**

| Name of antisense strand | Nucleotide sequence of antisense strand (5'-3') | SEQ ID NO |
|---|---|---|
| AD47882. 77AS | A(M)^G(F)^A(M)A(M)A(M)G(F)G(M)A(F)G(F)A(M)A(M)U(M)C(E)A(R)C(E)G(F)U(M)G(M)^G(M)^U(M)^U(M) | 14 |
| AD47882. 78AS | A(M)^G(F)^A(M)A(M)A(M)G(F)G(M)A(F)G(F)A(M)A(M)U(M)C(E)A(R)C(L)G(F)U(M)G(M)^G(M)^U(M)^U(M) | 14 |
| AD47882. 79AS | A(M)^G(F)^A(M)A(M)A(M)G(F)G(M)A(F)G(F)A(M)A(M)U(M)C(E)A(R)C(m)G(F)U(M)G(M)^G(M)^U(M)^U(M) | 14 |
| AD47882. 80AS | A(M)^G(F)^A(M)A(M)A(M)G(F)G(M)A(F)G(F)A(M)A(M)U(M)C(E)A(R)^C(M)G(F)U(M)G(M)^G(M)^U(M)^U(M) | 14 |
| AD47882. 81AS | A(M)^G(F)^A(M)A(M)A(M)G(F)G(M)A(F)G(F)A(M)A(M)U(M)C(E)A(R)^C(E)G(F)U(M)G(M)^G(M)^U(M)^U(M) | 14 |
| AD47882. 82AS | A(M)^G(F)^A(M)A(M)A(M)G(F)G(M)A(F)G(F)A(M)A(M)U(M)C(E)A(R)^C(L)G(F)U(M)G(M)^G(M)^U(M)^U(M) | 14 |
| AD47882. 83AS | A(M)^G(F)^A(M)A(M)A(M)G(F)G(M)A(F)G(F)A(M)A(M)U(M)C(E)A(R)^C(m)G(F)U(M)G(M)^G(M)^U(M)^U(M) | 14 |
| AD47882. 84AS | A(M)^G(F)^A(M)A(M)A(M)G(F)G(M)A(F)G(F)A(M)A(M)U(M)C(E)A(M)C(M)G(F)U(M)G(M)^G(M)^U(M)^U(M) | 14 |

**[Table 1-15]**

| Name of antisense strand | Nucleotide sequence of antisense strand (5'-3') | SEQ ID NO |
|---|---|---|
| AD47882. 88AS | A(M)^G(F)^A(M)A(F)A(M)G(F)G(M)A(M)G(F)A(M)A(M)T(D)C(M)A(E)C(M)G(F)U(M)G(M)^G(M)^U(M)^U(M) | 13 |
| AD47882. 89AS | A(M)^G(F)^A(M)A(M)A(M)G(F)G(M)A(M)G(F)A(F)A(M)T(D)C(M)A(E)C(M)G(F)U(M)G(M)^G(M)^U(M)^U(M) | 13 |
| AD47882. 90AS | A(M)^G(F)^A(M)A(M)A(M)G(F)G(M)A(M)G(F)A(M)A(M)T(D)C(M)A(E)C(M)G(F)U(M)G(F)^G(M)^U(M)^U(M) | 13 |
| AD47882. 91AS | A(M)^G(F)^A(M)A(M)A(M)G(F)G(M)A(M)G(F)A(F)A(M)U(M)C(E)A(R)^C(M)G(F)U(M)G(M)^G(M)^U(M)^U(M) | 14 |
| AD47882. 92AS | A(M)^G(F)^A(M)A(M)A(M)G(F)G(M)A(M)G(F)A(M)A(F)U(M)C(E)A(R)^C(M)G(F)U(M)G(M)^G(M)^U(M)^U(M) | 14 |
| AD47882. 93AS | A(M)^G(F)^A(M)A(M)A(M)G(F)G(M)A(M)G(F)A(M)A(M)U(F)C(E)A(R)^C(M)G(F)U(M)G(M)^G(M)^U(M)^U(M) | 14 |
| AD47882. 94AS | A(M)^G(F)^A(M)A(M)A(M)G(F)G(M)A(M)G(F)A(M)A(F)U(M)C(M)A(R)^C(M)G(F)U(M)G(M)^G(M)^U(M)^U(M) | 14 |
| AD47882. 9AS | A(M)^G(F)^A(M)A(M)A(M)G(F)G(M)A(F)G(M)A(M)A(M)U(M)C(M)A(F)C(M)G(F)U(M)G(M)G(M)^U(M)^U(M) | 14 |

**[Table 1-16]**

| Name of antisense strand | Nucleotide sequence of antisense strand (5'-3') | SEQ ID NO |
|---|---|---|
| TfR2-039. 9AS | U(M)^A(F)^C(M)A(M)C(M)U(F)A(M)C(F)G(M)G(M)C(M)U(M)U(M)U(F)G(M)A(F)G(M)G(M)U(M)^U(M)^U(M) | 4 |
| TfR2-019. 22AS | U(M)^U(F)^G(M)A(M)U(M)A(F)U(M)U(M)G(F)A(M)A(M)A(M)C(M)U(F)C(M)C(F)A(M)C(M)G(M)^U(M)^U(M) | 10 |
| TfR2-019. 94AS.s1 | U(M)^U(F)^G(M)A(M)U(M)A(F)U(M)U(M)G(F)A(M)A(F)A(M)C(M)U(R)^C(M)C(F)A(M)C(M)^G(M)^U(M) | 17 |
| TfR2-019. 94AS.s2 | U(M)^U(F)^G(M)A(M)U(M)A(F)U(M)U(M)G(F)A(M)A(F)A(M)C(M)U(R)^C(M)C(F)A(M)C(M)^G(M) | 18 |
| TfR2-019. 94AS.e1 | U(M)^U(F)^G(M)A(M)U(M)A(F)U(M)U(M)G(F)A(M)A(F)A(M)C(M)U(R)^C(M)C(F)A(M)C(M)G(M)^C(M)^U(M)^U(M) | 19 |
| TfR2-019. 94AS.e2 | U(M)^U(F)^G(M)A(M)U(M)A(F)U(M)U(M)G(F)A(M)A(F)A(M)C(M)U(R)^C(M)C(F)A(M)C(M)G(M)C(M)^C(M)^U(M)^U(M) | 20 |
| TfR2-019. 94AS.e3 | U(M)^U(F)^G(M)A(M)U(M)A(F)U(M)U(M)G(F)A(M)A(F)A(M)C(M)U(R)^C(M)C(F)A(M)C(M)^G(M)^G(M)^U(M) | 21 |

The "molecular weight" in the tables indicates a measured value from negative ion ESI mass spectrometry. In the "nucleotide sequence" in the tables, N(R) represents RNA, N(D) represents DNA, N(M) represents 2'-OMe RNA, N(m) represents 2'-MOE RNA, N(3M) represents 3'-OMe RNA, N(F) represents 2'-F RNA, N(L) represents LNA, and N(E) represents ENA. The structures of the nucleosides are represented by the above-described structural formulae, respectively.

"GN" represents a GalNAc unit represented by the above-described formula (GN). For the bond between nucleosides, the bond between a nucleoside and a GalNAc unit, or the bond between a nucleoside and a linker, "^" represents a phosphorothioate bond (-P(=S) (OH)-, represented by (PS) in the above structural diagram of inter-nucleoside bonds), and the bond is a phosphodiester bond (-P(=O) (OH)**-,** represented by (P) in the above structural diagram of inter-nucleoside bonds), if no specific indication is given.

The residue at each of the 5'-position of the nucleotide at the 5'-terminus and the 3'-position (2'-position in the case of a nucleotide modified at the 3'-position) of the nucleotide at the 3'-terminus of an oligonucleotide is a hydroxyl group in which a hydrogen atom is bonded to an oxygen atom in the above structural diagram of each nucleoside. However, when "GN" forms the terminus, bonding of a hydrogen atom is not necessary.

### <Test Examples>

### Test Example 1: Screening of HepG2 cells for TfR2 siRNA by reverse transfection method

By a reverse transfection method, screening for siRNA was performed in the following manner. On the day of transfection, each siRNA sample, a negative control (NT) using distilled water instead of siRNA, and a compound as a positive control which is the compound of Reference 2 or 3 and in which siRNA for hTfR2 is chemically modified (TfR2-039.23DUG or TfR2-019.22DUG) were prepared for two wells each.

5 µL of siRNA (compound of Example) was added to a liquid mixture of 14.8 µL of Opti-MEM and 0.2 µL of RNAiMAX, per well. The mixture was incubated at room temperature. For preparation of a reagent for the negative control, 5 µL of distilled water was added, instead of siRNA, to the liquid mixture of Opti-MEM and RNAiMAX. For preparation of a reagent for the positive control, 5 µL of the compound of Reference Example 2 or 3 was added to the liquid mixture of Opti-MEM and RNAiMAX. Next, the liquid mixture containing siRNA, the negative control or the positive control was added to a 96-well cell culture plate (manufactured by SUMIRON Co., Ltd.) at 20 µL per well. In the 96-well plate to which the liquid mixture had been added, 2 × 10⁴ cells/mL HepG2 cells, prepared using DMEM containing 10% FBS and warmed in advance to 37°C, were seeded at 80 µL per well. The final concentration of siRNA was adjusted to be increased by a common ratio of 10 or 50 from 10 nM.

One or two days after the transfection, the cells were washed once with PBS. Using Superprep Cell Lysis RT Kit for qPCR (Takara Bio Inc.), RNA extraction and a reverse transcription reaction were carried out in accordance with the protocol of the kit to synthesize cDNA. Using the obtained cDNA, the mRNA expression level of endogenous hTfR2 was measured by quantitative PCR. For the housekeeping gene, the mRNA expression level of h18S was measured.

The quantitative PCR was performed in the following manner using IDT PrimeTime Gene Expression Master Mix (manufactured by Integrated DNA Technologies, Inc.). The obtained cDNA was used as a stock solution. In a 384-well PCR plate (manufactured by Applied Biosystems), 5 µL of IDT PrimeTime Gene Expression Master Mix, 0.25 µL of a hTfR2 primer (Hs. PT. 58. 20599434, manufactured by Integrated DNA Technologies, Inc.) or 0.25 µL of a h18S primer (Hs. PT. 39a. 22214856.g, manufactured by Integrated DNA Technologies, Inc.), 2.75 µL of distilled water and 2 µL of cDNA (total 10 µL) were mixed per well, the mixture was prepared in a 2-well-equivalent amount for each cDNA, and quantitative PCR was performed. The mRNA expression level of each of hTfR2 and h18S was given as an average value of two wells. The mRNA expression level of hTfR2 was standardized with the mRNA expression level of h18S, which is the housekeeping gene. As the hTfR2 mRNA expression level in the negative control is assumed to be 1.0, relative hTfR2 mRNA expression levels of the siRNA-treated samples were calculated and recorded. Tables 2 to 4 show the results.

**[Table 2-1]**

| Name of compound | Concentration (nM) | Relative value |
|---|---|---|
| NT | - | 1.00 |
| TfR2-039.23DUG | 0.001 | 0.91 |
| | 0.01 | 0.72 |
| | 0.1 | 0.60 |
| | 1 | 0.35 |
| | 10 | 0.27 |
| TfR2-039.41 DUG | 0.001 | 0.81 |
| | 0.01 | 0.63 |
| | 0.1 | 0.31 |
| | 1 | 0.13 |
| | 10 | 0.14 |
| TfR2-039.42DUG | 0.001 | 0.66 |
| | 0.01 | 0.73 |
| | 0.1 | 0.37 |
| | 1 | 0.16 |
| | 10 | 0.12 |

**[Table 2-2]**

| Name of compound | Concentration (nM) | Relative value |
|---|---|---|
| NT | - | 1.00 |
| TfR2-039.23DUG | 0.001 | 0.75 |
| | 0.01 | 0.76 |
| | 0.1 | 0.59 |
| | 1 | 0.25 |
| | 10 | 0.21 |
| TfR2-039.41 DUG | 0.001 | 0.66 |
| | 0.01 | 0.63 |
| | 0.1 | 0.26 |
| | 1 | 0.08 |
| | 10 | 0.07 |
| TfR2-039.43DUG | 0.001 | 0.72 |
| | 0.01 | 0.52 |
| | 0.1 | 0.59 |
| | 1 | 0.42 |
| | 10 | 0.35 |
| TfR2-039.45DUG | 0.001 | 0.48 |
| | 0.01 | 0.47 |
| | 0.1 | 0.44 |
| | 1 | 0.23 |
| | 10 | 0.21 |

**[Table 3-1]**

| Name of compound | Concentration (nM) | Relative value |
|---|---|---|
| NT | - | 1.00 |
| TfR2-019.22DUG | 0.001 | 0.87 |
| | 0.01 | 0.46 |
| | 0.1 | 0.23 |
| | 1 | 0.12 |
| | 10 | 0.15 |
| TfR2-019.87DUG | 0.001 | 0.95 |
| | 0.01 | 0.43 |
| | 0.1 | 0.25 |
| | 1 | 0.15 |
| | 10 | 0.13 |

**[Table 3-2]**

| Name of compound | Concentration (nM) | Relative value |
|---|---|---|
| NT | - | 1.00 |
| TfR2-019.22DUG | 0.001 | 0.89 |
| | 0.01 | 0.51 |
| | 0.1 | 0.22 |
| | 1 | 0.13 |
| | 10 | 0.10 |
| TfR2-019.85DUG | 0.001 | 0.64 |
| | 0.01 | 0.36 |
| | 0.1 | 0.17 |
| | 1 | 0.15 |
| | 10 | 0.09 |
| TfR2-019.86DUG | 0.001 | 0.72 |
| | 0.01 | 0.34 |
| | 0.1 | 0.19 |
| | 1 | 0.15 |
| | 10 | 0.10 |
| TfR2-039.41 DUG | 0.001 | 0.93 |
| | 0.01 | 0.89 |
| | 0.1 | 0.57 |
| | 1 | 0.33 |
| | 10 | 0.28 |

**[Table 4-1]**

| Name of compound | Concentration (nM) | Relative value |
|---|---|---|
| NT | - | 1.00 |
| TfR2-019.22DUG | 0.001 | 1.07 |
| | 0.01 | 0.61 |
| | 0.1 | 0.33 |
| | 1 | 0.25 |
| | 10 | 0.22 |
| TfR2-019.71DUG | 0.001 | 0.97 |
| | 0.01 | 0.88 |
| | 0.1 | 0.57 |
| | 1 | 0.32 |
| | 10 | 0.31 |
| TfR2-019.88DUG | 0.001 | 0.86 |
| | 0.01 | 0.76 |
| | 0.1 | 0.46 |
| | 1 | 0.28 |
| | 10 | 0.24 |
| TfR2-019.89DUG | 0.001 | 0.81 |
| | 0.01 | 0.71 |
| | 0.1 | 0.40 |
| | 1 | 0.30 |
| | 10 | 0.19 |

**[Table 4-2]**

| Name of compound | Concentration (nM) | Relative value |
|---|---|---|
| NT | - | 1.00 |
| TfR2-019.22DUG | 0.001 | 1.09 |
| | 0.01 | 0.73 |
| | 0.1 | 0.30 |
| | 1 | 0.20 |
| | 10 | 0.15 |
| TfR2-019.90DUG | 0.001 | 1.02 |
| | 0.01 | 0.93 |
| | 0.1 | 0.45 |
| | 1 | 0.29 |
| | 10 | 0.22 |
| TfR2-019.80DUG | 0.001 | 1.06 |
| | 0.01 | 0.76 |
| | 0.1 | 0.35 |
| | 1 | 0.26 |
| | 10 | 0.21 |
| TfR2-019.91DUG | 0.001 | 1.02 |
| | 0.01 | 0.66 |
| | 0.1 | 0.33 |
| | 1 | 0.25 |
| | 10 | 0.17 |

**[Table 4-3]**

| Name of compound | Concentration (nM) | Relative value |
|---|---|---|
| NT | - | 1.00 |
| TfR2-019.92DUG | 0.001 | 0.88 |
| | 0.01 | 0.67 |
| | 0.1 | 0.33 |
| | 1 | 0.24 |
| | 10 | 0.24 |
| TfR2-019.22DUG | 0.001 | 0.83 |
| | 0.01 | 0.55 |
| | 0.1 | 0.34 |
| | 1 | 0.23 |
| | 10 | 0.24 |
| TfR2-019.93DUG | 0.001 | 0.85 |
| | 0.01 | 0.67 |
| | 0.1 | 0.40 |
| | 1 | 0.26 |
| | 10 | 0.24 |

**[Table 4-4]**

| Name of compound | Concentration (nM) | Relative value |
|---|---|---|
| NT | - | 1.00 |
| TfR2-019.92DUG | 0.001 | 0.85 |
| | 0.01 | 0.52 |
| | 0.1 | 0.35 |
| | 1 | 0.16 |
| | 10 | 0.13 |
| TfR2-019.93DUG | 0.001 | 0.68 |
| | 0.01 | 0.42 |
| | 0.1 | 0.22 |
| | 1 | 0.11 |
| | 10 | 0.08 |
| TfR2-019.94DUG | 0.001 | 0.65 |
| | 0.01 | 0.48 |
| | 0.1 | 0.28 |
| | 1 | 0.15 |
| | 10 | 0.16 |
| TfR2-019.95DUG | 0.001 | 0.91 |
| | 0.01 | 0.53 |
| | 0.1 | 0.25 |
| | 1 | 0.18 |
| | 10 | 0.09 |

### Test Example 1-1: hTfR2 knockdown activity using HepG2 cells

In the same manner as in Test Example 1, the mRNA expression level of hTfR2 was evaluated 2 days after siRNA was introduced into HepG2 cells by a reverse transfection method. TfR2-019 and TfR2-019.94DUG (Example 16) were used as siRNA. TfR2-019 is a double-stranded oligonucleotide consisting of a sense strand region and an antisense strand region which are oligonucleotides that are independent of each other, and complementary regions of the sense strand region and the antisense strand region all consist of natural RNA (that is, A(R), G(R), C(R) and U(R)). The antisense strand region in TfR2-019 is an oligonucleotide whose target sequence is the nucleotide sequence from positions 2847 to 2865 of hTfR2 mRNA set forth as SEQ ID NO: 1 and which has a complementary region consisting of a sequence fully complementary to the target sequence and has two consecutive T(D) bound as an overhand structure to the 3'-terminus of the complementary region. The sense strand region in TfR2-019 is an oligonucleotide which has a complementary region consisting of a sequence fully complementary to the nucleotide sequence in the corresponding complementary region of the antisense strand region and has two consecutive T(D) bound as an overhang structure to the 3'-terminus of the complementary region. Each inter-nucleoside bond is a phosphodiester bond that is not chemically modified.

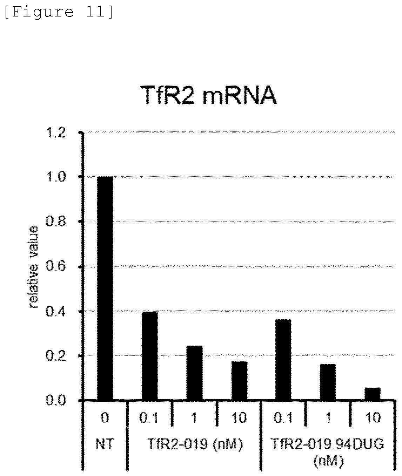

For evaluation of the hTfR2 mRNA expression level, relative hTfR2 mRNA expression levels of the siRNA-treated samples were calculated and recorded as relative values when the hTfR2 mRNA expression level in the negative control (NT) is assumed to be 1.0. The results are shown in Figure 11.

These results demonstrate that TfR2-019.94DUG has particularly high knockdown activity against hTfR2 mRNA.

### Test Example 2: Measurement of RNAi activity of TfR2 siRNA against HEK 293A cells (293A-mTfR2 cells) stably expressing mTfR2 by a reverse transfection method

Plasmids encoding mouse TfR2 (mTfR2) were expressed in excess in HEK293A cells by transfection, and HEK293A cells stably expressing mTfR2 (293A-mTfR2 cells) were then cloned by drug agent selection with GENETICIN (manufactured by Gibco).

By a reverse transfection method, screening for siRNA was performed in the following manner. On the day of transfection, each siRNA sample, a negative control (NT) using distilled water instead of siRNA, and the compound of Reference Example 1 as a positive control in which the sequence of siRNA for mTfR2 described in WO 2012/177921 is chemically modified (AD47882.23DUG) were prepared for two wells each.

To a liquid mixture of 14.8 µL of Opti-MEM and 0.2 µL of RNAiMAX, 5 µL of siRNA (compound of each Example) was added per well. The mixture was incubated at room temperature. For preparation of a reagent for the negative control, 5 µL of distilled water was added, instead of siRNA, to the liquid mixture of Opti-MEM and RNAiMAX. For preparation of a reagent for the positive control, 5 µL of the compound of Reference Example 1 was added to the liquid mixture of Opti-MEM and RNAiMAX. Next, the liquid mixture containing siRNA, the negative control or the positive control was added to a 96-well cell culture plate (manufactured by SUMIRON Co., Ltd.) at 20 µL per well. In the 96-well plate to which the liquid mixture had been added, 2 × 10⁴ cells/mL 293A-mTfR2 cells, prepared using DMEM containing 10% FBS and warmed in advance to 37°C, were seeded at 80 µL per well. The final concentration of siRNA was adjusted to be increased by a common ratio of 10 from 10 nM.

One or two days after the transfection, the cells were washed once with PBS. Using Superprep Cell Lysis RT Kit for qPCR (Takara Bio Inc.), RNA extraction and a reverse transcription reaction were carried out in accordance with the protocol of the kit to synthesize cDNA. Using the obtained cDNA, the mRNA expression level of mTfR2 was measured by quantitative PCR. For the housekeeping gene, the mRNA expression level of human 18S ribosomal RNA (h18S) was measured.

The quantitative PCR was performed in the following manner using IDT PrimeTime Gene Expression Master Mix (manufactured by Integrated DNA Technologies, Inc.). The obtained cDNA was used as a stock solution. In a 384-well PCR plate (manufactured by Applied Biosystems), 5 µL of IDT PrimeTime Gene Expression Master Mix, 0.25 µL of a mTfR2 primer (Mm. PT. 58. 7860185, Integrated DNA Technologies, Inc.) or 0.25 µL of a h18S primer (Hs. PT. 39a. 22214856. g, Integrated DNA Technologies, Inc.), 2.75 µL of distilled water and 2 µL of cDNA (total 10 µL) were mixed per well, the mixture was prepared in a 2-well-equivalent amount for each cDNA, and quantitative PCR was performed. The mRNA expression level of each of mTfR2 and h18S was given as an average value of two wells. The mRNA expression level of mTfR2 was standardized with the mRNA expression level of h18S, which is the housekeeping gene. As the mTfR2 mRNA expression level in the negative control is assumed to be 1.0, relative mTfR2 mRNA expression levels of the siRNA-treated samples were calculated. Tables 5 to 8 show the results.

**[Table 5-1]**

| Name of compound | Concentration (nM) | Relative value |
|---|---|---|
| NT | - | 1.00 |
| AD47882.23DUG | 0.001 | 0.86 |
| | 0.01 | 0.81 |
| | 0.1 | 0.35 |
| | 1 | 0.09 |
| | 10 | 0.05 |
| AD47882.41 DUG | 0.001 | 0.64 |
| | 0.01 | 0.59 |
| | 0.1 | 0.25 |
| | 1 | 0.08 |
| | 10 | 0.05 |
| AD47882.42DUG | 0.001 | 0.76 |
| | 0.01 | 0.64 |
| | 0.1 | 0.27 |
| | 1 | 0.08 |
| | 10 | 0.06 |

**[Table 5-2]**

| Name of compound | Concentration (nM) | Relative value |
|---|---|---|
| NT | - | 1.00 |
| AD47882.23DUG | 0.001 | 1.04 |
| | 0.01 | 0.98 |
| | 0.1 | 0.52 |
| | 1 | 0.14 |
| | 10 | 0.08 |
| AD47882.43DUG | 0.001 | 0.99 |
| | 0.01 | 1.01 |
| | 0.1 | 0.56 |
| | 1 | 0.16 |
| | 10 | 0.09 |
| AD47882.44DUG | 0.001 | 0.98 |
| | 0.01 | 1.02 |
| | 0.1 | 0.53 |
| | 1 | 0.14 |
| | 10 | 0.08 |
| AD47882.45DUG | 0.001 | 0.94 |
| | 0.01 | 0.87 |
| | 0.1 | 0.42 |
| | 1 | 0.12 |
| | 10 | 0.08 |
| AD47882.46DUG | 0.001 | 0.85 |
| | 0.01 | 0.86 |
| | 0.1 | 0.47 |
| | 1 | 0.13 |
| | 10 | 0.07 |

**[Table 5-3]**

| Name of compound | Concentration (nM) | Relative value |
|---|---|---|
| NT | - | 1.00 |
| AD47882.23DUG | 0.001 | 0.97 |
| | 0.01 | 0.90 |
| | 0.1 | 0.41 |
| | 1 | 0.11 |
| | 10 | 0.06 |
| AD47882.47DUG | 0.001 | 1.00 |
| | 0.01 | 0.91 |
| | 0.1 | 0.47 |
| | 1 | 0.10 |
| | 10 | 0.05 |
| AD47882.48DUG | 0.001 | 0.95 |
| | 0.01 | 0.93 |
| | 0.1 | 0.44 |
| | 1 | 0.09 |
| | 10 | 0.05 |

**[Table 6-1]**

| Name of compound | Concentration (nM) | Relative value |
|---|---|---|
| NT | - | 1.00 |
| AD47882.23DUG | 0.001 | 0.89 |
| | 0.01 | 0.59 |
| | 0.1 | 0.22 |
| | 1 | 0.11 |
| | 10 | 0.09 |
| AD47882.70DUG | 0.001 | 0.87 |
| | 0.01 | 0.78 |
| | 0.1 | 0.36 |
| | 1 | 0.16 |
| | 10 | 0.14 |
| AD47882.71 DUG | 0.001 | 0.78 |
| | 0.01 | 0.65 |
| | 0.1 | 0.24 |
| | 1 | 0.12 |
| | 10 | 0.10 |
| AD47882.72DUG | 0.001 | 0.83 |
| | 0.01 | 0.80 |
| | 0.1 | 0.60 |
| | 1 | 0.25 |
| | 10 | 0.18 |

**[Table 6-2]**

| Name of compound | Concentration (nM) | Relative value |
|---|---|---|
| NT | - | 1.00 |
| AD47882.23DUG | 0.001 | 0.90 |
| | 0.01 | 0.56 |
| | 0.1 | 0.20 |
| | 1 | 0.12 |
| | 10 | 0.09 |
| AD47882.73DUG | 0.001 | 0.98 |
| | 0.01 | 0.90 |
| | 0.1 | 0.63 |
| | 1 | 0.28 |
| | 10 | 0.22 |
| AD47882.74DUG | 0.001 | 0.87 |
| | 0.01 | 0.70 |
| | 0.1 | 0.47 |
| | 1 | 0.17 |
| | 10 | 0.15 |
| AD47882.75DUG | 0.001 | 0.78 |
| | 0.01 | 0.46 |
| | 0.1 | 0.16 |
| | 1 | 0.09 |
| | 10 | 0.07 |
| AD47882.76DUG | 0.001 | 0.73 |
| | 0.01 | 0.43 |
| | 0.1 | 0.19 |
| | 1 | 0.10 |
| | 10 | 0.08 |

**[Table 6-3]**

| Name of compound | Concentration (nM) | Relative value |
|---|---|---|
| NT | - | 1.00 |
| AD47882.23DUG | 0.001 | 0.77 |
| | 0.01 | 0.40 |
| | 0.1 | 0.15 |
| | 1 | 0.09 |
| | 10 | 0.08 |
| AD47882.77DUG | 0.001 | 0.84 |
| | 0.01 | 0.64 |
| | 0.1 | 0.24 |
| | 1 | 0.12 |
| | 10 | 0.10 |
| AD47882.78DUG | 0.001 | 0.73 |
| | 0.01 | 0.62 |
| | 0.1 | 0.29 |
| | 1 | 0.11 |
| | 10 | 0.10 |
| AD47882.79DUG | 0.001 | 0.61 |
| | 0.01 | 0.29 |
| | 0.1 | 0.10 |
| | 1 | 0.07 |
| | 10 | 0.06 |
| AD4788280DUG | 0.001 | 0.68 |
| | 0.01 | 0.43 |
| | 0.1 | 0.17 |
| | 1 | 0.08 |
| | 10 | 0.06 |

**[Table 6-4]**

| Name of compound | Concentration (nM) | Relative value |
|---|---|---|
| NT | - | 1.00 |
| AD47882.23DUG | 0.001 | 0.87 |
| | 0.01 | 0.56 |
| | 0.1 | 0.18 |
| | 1 | 0.10 |
| | 10 | 0.08 |
| AD47882.81 DUG | 0.001 | 0.88 |
| | 0.01 | 0.74 |
| | 0.1 | 0.35 |
| | 1 | 0.14 |
| | 10 | 0.11 |
| AD47882.82DUG | 0.001 | 0.77 |
| | 0.01 | 0.64 |
| | 0.1 | 0.33 |
| | 1 | 0.12 |
| | 10 | 0.11 |
| AD47882.83DUG | 0.001 | 0.64 |
| | 0.01 | 0.37 |
| | 0.1 | 0.16 |
| | 1 | 0.08 |
| | 10 | 0.06 |
| AD47882.84DUG | 0.001 | 0.71 |
| | 0.01 | 0.68 |
| | 0.1 | 0.48 |
| | 1 | 0.19 |
| | 10 | 0.15 |

**[Table 7-1]**

| Name of compound | Concentration (nM) | Relative value |
|---|---|---|
| NT | - | 1.00 |
| AD47882.23DUG | 0.001 | 1.04 |
| | 0.01 | 0.94 |
| | 0.1 | 0.42 |
| | 1 | 0.09 |
| | 10 | 0.06 |
| AD47882.71 DUG | 0.001 | 1.07 |
| | 0.01 | 1.02 |
| | 0.1 | 0.60 |
| | 1 | 0.15 |
| | 10 | 0.10 |
| AD47882.88DUG | 0.001 | 1.05 |
| | 0.01 | 1.02 |
| | 0.1 | 0.54 |
| | 1 | 0.16 |
| | 10 | 0.12 |
| AD47882.89DUG | 0.001 | 1.03 |
| | 0.01 | 0.94 |
| | 0.1 | 0.57 |
| | 1 | 0.14 |
| | 10 | 0.08 |
| AD4788290DUG | 0.001 | 1.05 |
| | 0.01 | 0.91 |
| | 0.1 | 0.54 |
| | 1 | 0.14 |
| | 10 | 0.08 |

**[Table 7-2]**

| Name of compound | Concentration (nM) | Relative value |
|---|---|---|
| NT | - | 1.00 |
| AD47882.23DUG | 0.001 | 0.90 |
| | 0.01 | 0.74 |
| | 0.1 | 0.34 |
| | 1 | 0.08 |
| | 10 | 0.05 |
| AD4788280DUG | 0.001 | 0.93 |
| | 0.01 | 0.84 |
| | 0.1 | 0.39 |
| | 1 | 0.09 |
| | 10 | 0.05 |
| AD47882.91 DUG | 0.001 | 0.86 |
| | 0.01 | 0.78 |
| | 0.1 | 0.40 |
| | 1 | 0.10 |
| | 10 | 0.05 |
| AD47882.92DUG | 0.001 | 0.78 |
| | 0.01 | 0.71 |
| | 0.1 | 0.42 |
| | 1 | 0.10 |
| | 10 | 0.05 |
| AD47882.93DUG | 0.001 | 0.78 |
| | 0.01 | 0.65 |
| | 0.1 | 0.32 |
| | 1 | 0.09 |
| | 10 | 0.04 |

**[Table 8]**

| Name of compound | Concentration (nM) | Relative value |
|---|---|---|
| NT | - | 1.00 |
| AD47882.23DUG | 0.001 | 1.03 |
| | 0.01 | 0.66 |
| | 0.1 | 0.20 |
| | 1 | 0.09 |
| | 10 | 0.06 |
| AD47882.92DUG | 0.001 | 1.12 |
| | 0.01 | 0.88 |
| | 0.1 | 0.28 |
| | 1 | 0.10 |
| | 10 | 0.08 |
| AD47882.94DUG | 0.001 | 1.12 |
| | 0.01 | 0.80 |
| | 0.1 | 0.29 |
| | 1 | 0.11 |
| | 10 | 0.08 |

### Test Example 3: Drug efficacy evaluation test for GalNAc-conjugated TfR2 siRNA in normal mice

GalNAc-conjugated TfR2 siRNA has been shown to increase the iron concentration in plasma in normal mice (WO 2012/177921). Various chemical modifications were applied to the nucleotide sequence of AD47882 described in that document, and the following test was conducted using the compound of Reference Example 1 (AD47882.23DUG) and the compounds of Examples 18 to 25 (AD47882.41DUG to 48DUG) as GalNAc-conjugated siRNA.

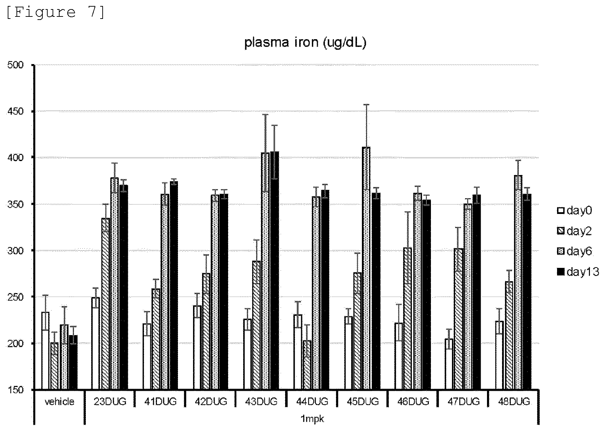

GalNAc-conjugated TfR2 siRNA was subcutaneously (s.c.) administered to male C57BL/6NJcl mice (from CLEA Japan, Inc.) at a dose of 1 mg/kg (1 mpk) (n = 5/group). To the vehicle group, PBS was subcutaneously administered (n = 5/group). Before the treatment with siRNA or PBS (Day 0), 2 days after the treatment (Day 2), 6 days after the treatment (Day 6) and 13 days after the treatment (Day 13), blood was collected from the tail vein, and the iron concentration in plasma was measured using a Metallo-assay iron measurement kit (manufactured by Metallogenics Co., Ltd.). The results for the iron concentration in plasma are shown in Figure 7 (average value ± standard error).

### Test Example 4: Drug efficacy evaluation test for GalNAc-conjugated TfR2 siRNA in normal mice

For evaluating sustained activity of TfR2 siRNA in the mouse body, various chemical modifications were applied to the nucleotide sequence of AD47882, and the following experiment was conducted using the compound of Reference Example 1 (AD47882.23DUG), the compound of Example 22 (AD47882.45DUG) and the compounds of Examples 26 to 40 (AD47882.70 to 84DUG) as GalNAc-conjugated siRNA.

GalNAc-conjugated TfR2 siRNA was subcutaneously (s.c.) administered to male C57BL/6NJcl mice (from CLEA Japan, Inc.) at a dose of 1 mg/kg (1 mpk) (n = 5/group). PBS was subcutaneously administered (n = 5/group) to the vehicle group. Before the treatment with siRNA or PBS (Day 0), 7 days after the treatment (Day 7), 12 days after the treatment (Day 12), 21 days after the treatment (Day 21), and 28 days after the treatment (Day 28), blood was collected from the tail vein, and the iron concentration in plasma was measured using a Metallo-assay iron measurement kit (manufactured by Metallogenics Co., Ltd.). The results for the iron concentration in plasma are shown in Figure 8 (average value ± standard error).

### Test Example 5: Drug efficacy evaluation test for GalNAc-conjugated TfR2 siRNA in normal mice

As in Test Example 4, various chemical modifications were applied to the nucleotide sequence of AD47882, and the following test was conducted using the compound of Reference Example 1 (AD47882.23DUG), the compound of Example 27 (AD47882.71DUG), the compound of Example 36 (AD47882.80DUG) and the compounds of Examples 41 to 46 (AD47882.88 to 93DUG) as GalNAc-conjugated siRNA.

GalNAc-conjugated TfR2 siRNA was subcutaneously (s.c.) administered to male C57BL/6NJcl mice (from CLEA Japan, Inc.) at a dose of 1 mg/kg (1 mpk) (n = 5/group). PBS was subcutaneously administered to the vehicle group (n = 5/group). Before the treatment with siRNA or PBS (Day 0), 7 days after the treatment (Day 7), 14 days after the treatment (Day 14), 21 days after the treatment (Day 21), and 28 days after the treatment (Day 28), blood was collected from the tail vein, and the iron concentration in plasma was measured using a Metallo-assay iron measurement kit (manufactured by Metallogenics Co., Ltd.). The results for the iron concentration in plasma are shown in Figure 9 (average value ± standard error).

In addition, 28 days after the treatment with siRNA or PBS (Day 28), the mice were painlessly killed by bloodletting under anesthesia with isoflurane, the liver was then isolated, and the mRNA expression level of mTfR2 was measured. Using RNeasy Mini Kit (manufactured by QUAGEN), RNA was extracted in accordance with the protocol of the kit. 5,000 ng of RNA obtained was subjected to a reverse transcription reaction using High Capacity RNA to cDNA Kit (manufactured by Applied Biosystems). Using the thus-obtained cDNA, the mRNA level of endogenous mouse TfR2 was measured by quantitative PCR.

The quantitative PCR was performed in the following manner using IDT PrimeTime Gene Expression Master Mix (manufactured by Integrated DNA Technologies, Inc.). The obtained cDNA was diluted 10-fold with distilled water (15 µL of cDNA and 135 µL of distilled water were mixed). In a 384-well PCR plate (manufactured by Applied Biosystems), 5 µL of IDT PrimeTime Gene Expression Master Mix, 0.25 µL of a mTfR2 primer (Mm. PT. 58. 7860185, manufactured by Integrated DNA Technologies, Inc.) or 2.75 µL of distilled water and 2 µL of cDNA (total 10 µL) were mixed per well, the mixture was prepared in a 2-well-equivalent amount for each cDNA, and quantitative PCR was performed. For the housekeeping gene, a mouse β-actin (mActb) primer (Mm. PT. 39a, 22214843. g, manufactured by Integrated DNA Technologies, Inc.) was used, and mixed so as to meet a ratio as described above, the mixture was prepared in a 2-well-equivalent amount for each cDNA, and quantitative PCR was performed. The mRNA level of each of mTfR2 and mActb was given as an average value of two wells. The mRNA expression level of mTfR2 was standardized with the mRNA expression level of mActb, which is the housekeeping gene. As the mRNA level in the vehicle group is assumed to be 1.0, relative mRNA levels in the siRNA treatment groups were calculated and recorded (average value ± standard error). The results for the mRNA expression level of mTfR2 are shown in Figure 9F.

### Test Example 6: Drug efficacy evaluation test for GalNAc-conjugated TfR2 siRNA in model of anemia associated with inflammation

GalNAc-conjugated TfR2 siRNA increases the HGB concentration in a model of anemia associated with inflammation. Various chemical modifications were applied to the nucleotide sequence of AD47882, and the following test was conducted using AD47882.41DUG (compound of Example 18), AD47882.88DUG (compound of Example 41), AD47882.89DUG (compound of Example 42), AD47882.91DUG (compound of Example 44), AD47882.92DUG (compound of Example 45) and AD47882.93DUG (compound of Example 46) as GalNAc-conjugated siRNA.

GalNAc-conjugated TfR2 siRNA was administered to male C57BL/6NJcl mice (from CLEA Japan, Inc.) once at a dose of 1 mg/kg (1 mpk) (Day 0). PBS was subcutaneously administered to the vehicle group. Four days after the treatment with siRNA or PBS (Day 4), turpentine was subcutaneously administered at 150 µL/mouse to induce inflammation. To the control group that would not undergo induction of inflammation, PBS was administered s.c. instead of turpentine. In the present test, n = 5/group. Two days after the first administration of turpentine (Day 6), the iron concentration in plasma was measured. The iron concentration in plasma was measured using a Metallo-assay iron measurement kit (manufactured by Metallogenics Co., Ltd.). The results are shown in Figure 10A.

Seven days after the first administration of turpentine (Day 11), turpentine was subcutaneously administered again at 150 µL/mouse. Three days after the second administration of turpentine (Day 14), blood was collected from the abdominal large vein under anesthesia with isoflurane, and the HGB concentration , the MCH level and the RBC number were measured using a multi-item automatic corpuscle analyzer XT 2000 (manufactured by Sysmex Corporation). The results are shown in Figures 10B, 10C and 10D, respectively.

The mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 10E. As the mRNA level in the control group which did not undergo induction of inflammation is assumed to be 1.0, relative mRNA levels in other groups were calculated and recorded (average value ± standard error).

### Test Example 7: Screening of HepG2 cells for TfR2 siRNA by reverse transfection method

In the same manner as in Test Example 1, a test was conducted on the mRNA expression level of hTfR2 using TfR2-019.94DUG (Example 16), TfR2-019.94DUG. s1 (Example 48), TfR2-019.94. s2DUG (Example 49), TfR2-019.94. elDUG (Example 50), TfR2-019.94DUG. e2 (Example 51) and TfR2-019.94DUG. e3 (Example 52) as siRNA. As the hTfR2 mRNA expression level in the negative control (NT) is assumed to be 1.0, relative hTfR2 mRNA expression levels of the siRNA-treated samples were calculated and recorded. Table 9 below shows the results.

**[Table 9-1]**

| Name of compound | Concentration (nM) | Relative value |
|---|---|---|
| NT | - | 1.00 |
| TfR2-019.94DUG | 0.001 | 0.97 |
| | 0.01 | 0.58 |
| | 0.1 | 0.26 |
| | 1 | 0.05 |
| | 10 | 0.03 |
| TfR2-019.94DUG.s1 | 0.001 | 1.13 |
| | 0.01 | 0.48 |
| | 0.1 | 0.17 |
| | 1 | 0.07 |
| | 10 | 0.02 |
| TfR2-019.94DUG.s2 | 0.001 | 1.02 |
| | 0.01 | 0.53 |
| | 0.1 | 0.26 |
| | 1 | 0.08 |
| | 10 | 0.04 |
| TfR2-019.94DUG.e1 | 0.001 | 0.92 |
| | 0.01 | 0.40 |
| | 0.1 | 0.20 |
| | 1 | 0.06 |
| | 10 | 0.02 |
| TfR2-019.94DUG.e2 | 0.001 | 0.89 |
| | 0.01 | 0.51 |
| | 0.1 | 0.21 |
| | 1 | 0.08 |
| | 10 | 0.04 |

**[Table 9-2]**

| Name of compound | Concentration (nM) | Relative value |
|---|---|---|
| NT | - | 1.00 |
| TfR2-019.94DUG | 0.001 | 0.86 |
| | 0.01 | 0.63 |
| | 0.1 | 0.30 |
| | 1 | 0.10 |
| | 10 | 0.06 |
| TfR2-019.94DUG e3 | 0.001 | 0.99 |
| | 0.01 | 0.60 |
| | 0.1 | 0.27 |
| | 1 | 0.09 |
| | 10 | 0.03 |

### Test Example 8: Drug efficacy evaluation test for GalNAc-conjugated TfR2 siRNA in normal mice

As in Test Example 5, various chemical modifications were applied to the nucleotide sequence of AD47882, and the following experiment was conducted using the compound of Reference Example 1 (AD47882.23DUG), the compound of Example 45 (AD47882.92DUG) and the compound of Example 47 (AD47882.94DUG) as GalNAc-conjugated siRNA.

The GalNAc-conjugated TfR2 siRNA was subcutaneously (s.c.) administered to male C57BL/6NJcl mice (from CLEA Japan, Inc.) at a dose of 1 mg/kg (1 mpk) for AD47882.23DUG (23DUG), at a dose of 0.3 mg/kg (0.3 mpk) or 1 mg/kg (1 mpk) for AD47882.92DUG (92DUG), and at a dose of 0.3 mg/kg (0.3 mpk) or 1 mg/kg (1 mpk) for AD47882.94DUG (n = 4 or 5/group). PBS was subcutaneously administered to the vehicle group (n = 4/group). Before the treatment with siRNA or PBS (Day 0), 9 days after the treatment (Day 9), 14 days after the treatment (Day 14), 21 days after the treatment (Day 21) and 28 days after the treatment (Day 28), blood was collected from the tail vein, and the iron concentration in plasma was measured using a Metallo-assay iron measurement kit (manufactured by Metallogenics Co., Ltd.). The results for the iron concentration in plasma are shown in Figures 12A to 12E (average value ± standard error).

In addition, 28 days after the treatment with siRNA or PBS (Day 28), the mice were painlessly killed by bloodletting under anesthesia with isoflurane, the liver was then isolated, and the mRNA expression level of mTfR2 was measured. Extraction of RNA and quantitative PCR were performed in the manner described in Test Example 5. The mRNA expression level of mTfR2 was standardized with the mRNA expression level of mActb, which is the housekeeping gene. The results for the mRNA expression level of mTfR2 are shown in Figure 12F. As the mRNA level in the vehicle group is assumed to be 1.0, the relative mRNA levels in other groups were calculated and recorded (average value ± standard error).

### Test Example 9: Drug efficacy evaluation test for GalNAc-conjugated TfR2 siRNA in human TfR2-expressing mice

To verify the effect of GalNAc-conjugated TfR2 siRNA on human TfR2 mRNA, the following experiment was conducted using male or female TfR2 humanized mice having a human TfR2 gene locus and generated in Institute of Immunology Co., Ltd. As GalNAc-conjugated TfR2 siRNA, the compounds TfR2-019.88DUG (Example 9), TfR2-019.92DUG (Example 14), TfR2-019.93DUG (Example 15) and TfR2-019.94DUG (Example 16) were used.

To the GalNAc-conjugated TfR2 siRNA treatment group, the siRNA was subcutaneously (s.c.) administered at a dose of 3 mg/kg (3 mpk) or 0.3 mg/kg (0.3 mpk) once (n = 3/group). PBS was subcutaneously administered once to the vehicle group (n = 4/group).

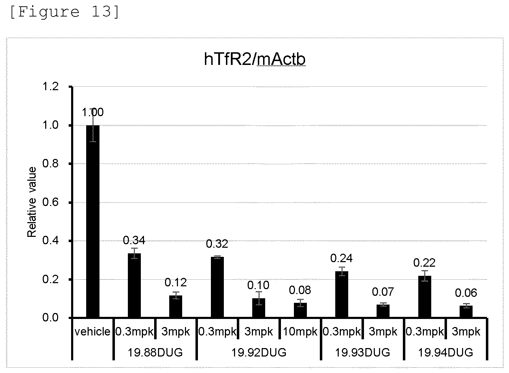

Seven days after the start of the drug agent treatment, the mice were painlessly killed by bloodletting under anesthesia with isoflurane, the liver was then isolated, and the mRNA expression level of hTfR2 was measured. Extraction of RNA and quantitative PCR were performed in the manner described in Test Example 5. The mRNA expression level of hTfR2 was standardized with the mRNA expression level of mActb, which is the housekeeping gene. As the primer for hTfR2, those described in Test Examples 1 and 2 were used. The results for the mRNA expression level of hTfR2 are shown in Figure 13. As the mRNA level in the vehicle group is assumed to be 1.0, relative mRNA levels in other groups were calculated and written (average value ± standard error).

These results demonstrate that treatment with GalNAc-conjugated TfR2 siRNA enables suppression of the expression level of human TfR2 mRNA. In addition, they indicate that suppression of the expression level of human TfR2 mRNA may increase the HGB concentration , and enable the provision of a therapeutic effect for anemic diseases such as anemia associated with bone-marrow dysfunction, for example, anemia associated with bone-marrow fibrosis, and anemic diseases such as anemia associated with inflammation, for example, anemia associated with chronic kidney disease.

### Free Text of Sequence Listing

SEQ ID NO: 1 - Nucleotide sequence of hTfR2 mRNA
SEQ ID NO: 2 - Nucleotide sequence of a sense strand of TfR2-039
SEQ ID NOS: 3 to 8 - Nucleotide sequences of antisense strands of TfR2-039
SEQ ID NO: 9 - Nucleotide sequence of a sense strand of TfR2-019
SEQ ID NOS: 10 and 11 - Nucleotide sequences of antisense strands of TfR2-019
SEQ ID NO: 12 - Nucleotide sequence of a sense strand of AD47882
SEQ ID NOS: 13 and 14 - Nucleotide sequences of antisense strands of AD47882
SEQ ID NOS: 15 and 16 - Nucleotide sequences of sense strand regions of a double-stranded oligonucleotide
SEQ ID NOS: 17 to 21 - Nucleotide sequences of antisense strand regions of a double-stranded oligonucleotide

## Claims

1. An oligonucleotide or a pharmaceutically acceptable salt thereof comprising a sense strand region and an antisense strand region, and having the following characteristics (a) to (g), the sense strand region consisting of an oligonucleotide represented by the following formula (I), the antisense strand region consisting of an oligonucleotide represented by the following formula (II):
sense strand region: 5' S_{O5}-Sₐ-S₁₉-S₁₈-S₁₇-S₁₆-S₁₅-S₁₄-S₁₃-S₁₂-S₁₁-S₁₀-S₉-S₈-S₇-S₆-S₅-S₄-S₃-S₂-S₁-S_{O3} 3' (I)
antisense strand region: 5' A₀₅-A₁-A₂-A₃-A₄-A₅-A₆-A₇-A₈-A₉-A₁₀-A₁₁-A₁₂-A₁₃-A₁₄-A₁₅-A₁₆-A₁₇-A₁₈-A₁₉-Aₐ-A₀₃ 3' (II)
(a) S₁ is a 3'-modified nucleoside, S₂ to S₁₉ each represents a nucleoside, and are each independently 2'-OMe RNA, 2'-F RNA or DNA, Sₐ represents 0 to 5 nucleosides, where the nucleosides each independently represent 2'-OMe RNA, 2'-F RNA or DNA, S_{O3} and S_{O5} each independently represent 0 to 5 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
(b) A₁₁, A₁₂, A₁₃, A₁₄ and A₁₅ each represents a nucleoside, at least one of A₁₁, A₁₂, A₁₃, A₁₄ and A₁₅ is DNA, RNA, a 2'-O,4'-C-bridging-modified nucleoside or 2'-MOE RNA, the others are 2'-OMe RNA or 2'-F RNA, A₁ to A₁₀ and A₁₆ to A₁₉ each represents a nucleoside, and each independently represent 2'-OMe RNA, 2'-F RNA or DNA, Aₐ represents 0 to 5 nucleosides, where the nucleosides each independently represent 2'-OMe RNA, 2'-F RNA or DNA, A_{O3} and A_{O5} each independently represent 0 to 5 nucleosides, where the nucleosides each independently represent 2'-OMe RNA, 2'-F RNA, DNA or a 2'-O,4'-C-bridging-modified nucleoside, and each inter-nucleoside bond represents a phosphodiester bond that may be chemically modified;
(c) the nucleotide sequence between A₂ and Aₐ consists of a nucleotide sequence substantially complementary to a target sequence, A₁ is a nucleoside having a base complementary to a corresponding nucleoside of the target sequence, a nucleoside comprising adenine, a nucleoside comprising thymine, or a nucleoside comprising uracil, and when A₀₃ and A₀₅ are present, the nucleotide sequences thereof are each independently a nucleotide sequence selected independently of a corresponding nucleoside of the target sequence;
(d) the nucleotide sequence between S₁ and Sₐ and the nucleotide sequence between A₁ and Aₐ are nucleotide sequences substantially complementary to each other, and form a double-stranded structure;
(e) when both S_{O5} and A_{O3} are present, S_{O5} and A_{O3} are nucleotide sequences not complementary to each other;
(f) when both S_{O3} and A_{O5} are present, S_{O3} and A_{O5} are nucleotide sequences not complementary to each other; and
(g) the sense strand region and/or the antisense strand region may be chemically modified at the 5'-position of the nucleoside at the 5'-terminus and/or the 3'-position of the nucleoside at the 3'-terminus, or the 2'-position thereof in the case where the nucleoside at the 3'-terminus is a 3'-modified nucleoside.

2. The oligonucleotide or pharmaceutically acceptable salt thereof according to claim 1, wherein in formula (II), S₁ is 3'-OMe RNA.

3. The oligonucleotide or pharmaceutically acceptable salt thereof according to claim 1 or 2, wherein the sense strand region and the antisense strand region are independent oligonucleotides and form a double-stranded oligonucleotide.

4. The oligonucleotide or pharmaceutically acceptable salt thereof according to any one of claims 1 to 3, wherein the 2'-O,4'-C-bridging-modified nucleoside is LNA or ENA.

5. The oligonucleotide or pharmaceutically acceptable salt thereof according to any one of claims 1 to 4, wherein in formula (II), two or more of T₁₁, A₁₂, A₁₃, A₁₄ and A₁₅ are the same or different, and are DNA, RNA, a 2'-O,4'-C-bridging-modified nucleoside or 2'-MOE RNA.

6. The oligonucleotide or pharmaceutically acceptable salt thereof according to claim 5, wherein in formula (II), A₁₄ is RNA or a 2'-O,4'-C-bridging-modified nucleoside.

7. The oligonucleotide or pharmaceutically acceptable salt thereof according to claim 6, wherein in formula (II), A₁₃ is a 2'-O,4'-C-bridging-modified nucleoside or 2'-OMe RNA, and A₁₄ is RNA.

8. The oligonucleotide or pharmaceutically acceptable salt thereof according to claim 7, wherein in formula (II), A₁₁-A₁₂-A₁₃-A₁₄-A₁₅ is one of:
A₁₁ (2'-OMe RNA) -A₁₂(2'-OMe RNA) -A₁₃ (ENA) -A₁₄ (RNA) -A₁₅ (2'-OMe RNA),
A₁₁ (2'-F RNA) -A₁₂(2'-OMe RNA) -A₁₃ (ENA) -A₁₄ (RNA) -A₁₅ (2'-OMe RNA),
A₁₁ (2'-OMe RNA) -A₁₂ (2'-F RNA) -A₁₃ (ENA) -A₁₄ (RNA) -A₁₅ (2'-OMe RNA),
A₁₁ (2'-OMe RNA) -A₁₂(2'-OMe RNA) -A₁₃ (LNA) -A₁₄ (RNA) -A₁₅ (2'-OMe RNA),
A₁₁ (2'-F RNA)-A₁₂ (2'-OMe RNA)-A₁₃ (LNA) -A₁₄ (RNA) -A₁₅ (2'-OMe RNA),
A₁₁ (2'-OMe RNA)-A₁₂ (2'-F RNA)-A₁₃ (LNA) -A₁₄ (RNA) -A₁₅ (2'-OMe RNA),
A₁₁ (2'-F RNA)-A₁₂ (2'-OMe RNA)-A₁₃ (2'-OMe RNA)-A₁₄ (RNA) - A₁₅ (2'-OMe RNA), or
A₁₁ (2'-OMe RNA)-A₁₂ (2'-F RNA)-A₁₃ (2'-OMe RNA)-A₁₄ (RNA) - A₁₅ (2'-OMe RNA) .

9. The oligonucleotide or pharmaceutically acceptable salt thereof according to claim 6, wherein in formula (II), A₁₂ is DNA, and A₁₄ is a 2'-O,4'-C-bridging-modified nucleoside.

10. The oligonucleotide or pharmaceutically acceptable salt thereof according to claim 9, wherein in formula (II), A₁₁-A₁₂-A₁₃-A₁₄-A₁₅ is one of:
A₁₁ (2'-F RNA) -A₁₂ (DNA) -A₁₃ (2'-OMe RNA) -A₁₄ (ENA)-A₁₅ (2'-OMe RNA),
A₁₁ (2'-OMe RNA) -A₁₂ (DNA) -A₁₃ (2'-OMe RNA) -A₁₄ (ENA) -A₁₅ (2'-OMe RNA), A₁₁ (2'-F RNA)-A₁₂ (DNA) -A₁₃ (2'-OMe RNA)-A₁₄ (LNA) - A₁₅ (2'-OMe RNA), or
A₁₁ (2'-OMe RNA) -A₁₂ (DNA) -A₁₃ (2'-OMe RNA) -A₁₄ (LNA) -A₁₅ (2'-OMe RNA) .

11. The oligonucleotide or pharmaceutically acceptable salt thereof according to any one of claims 1 to 10, wherein in formula (II), A₂, A₆ and A₁₆ are 2'-F RNA.

12. The oligonucleotide or pharmaceutically acceptable salt thereof according to claim 11, wherein in formula (II), one or two nucleosides selected from A₈, A₉ and A₁₀ are 2'-F RNA, and the nucleosides A₁, A₃ to A₅, A₇, A₁₇ to A₁₉ and Aₐ are 2'-OMe RNA.

13. The oligonucleotide or pharmaceutically acceptable salt thereof according to claim 12, wherein in formula (II), A₂, A₆, A₈, A₁₀ and A₁₆ are 2'-F RNA, and the nucleosides A₁, A₃ to A₅, A₇, A₉, A₁₇ to A₁₉ and Aₐ are 2'-OMe RNA.

14. The oligonucleotide or pharmaceutically acceptable salt thereof according to any one of claims 1 to 13, wherein in formula (I), S₁₁, S₁₂, S₁₃ and S₁₅ are 2'-F RNA.

15. The oligonucleotide or pharmaceutically acceptable salt thereof according to claim 14, wherein in formula (I), S₂ to S₁₀, S₁₄, S₁₆ to S₁₉ and Sₐ are 2'-OMe RNA.

16. The oligonucleotide or pharmaceutically acceptable salt thereof according to any one of claims 1 to 15, wherein when RNA is used in formula (II), the bond between the RNA and a 3' adjacent nucleoside is a phosphorothioate bond.

17. The oligonucleotide or pharmaceutically acceptable salt thereof according to any one of claims 1 to 16, wherein in 2 to 5 nucleotides from each of the 5'-terminus and the 3'-terminus of the oligonucleotide, each inter-nucleoside bond is a phosphorothioate bond.

18. The oligonucleotide or pharmaceutically acceptable salt thereof according to any one of claims 1 to 17, wherein the number of nucleosides in each of S_{O3}, S_{O5,} A_{O5} and A_{O3} is 0 to 2.

19. The oligonucleotide or pharmaceutically acceptable salt thereof according to claim 18, wherein the number of nucleosides in S_{O3} is 0.

20. The oligonucleotide or pharmaceutically acceptable salt thereof according to claim 19, wherein the number of nucleosides in each of S_{O3}, S_{O5} and A_{O5} is 0, and the number of nucleosides in A_{O3} is 2.

21. The oligonucleotide or pharmaceutically acceptable salt thereof according to any one of claims 1 to 20, wherein a chemical modification at the 5'-position of the nucleoside at the 5'-terminus and/or the 3'-position of the nucleoside at the 3'-terminus of the oligonucleotide, or the 2'-position thereof in the case where the nucleoside at the 3'-terminus is a 3'-modified nucleoside is a modification with a unit for delivery to an intended tissue.

22. The oligonucleotide or pharmaceutically acceptable salt thereof according to claim 21, wherein the unit for delivery to an intended tissue is a GalNAc unit or a fatty acid unit.

23. The oligonucleotide or pharmaceutically acceptable salt thereof according to claim 22, wherein the GalNAc unit is a unit represented by: 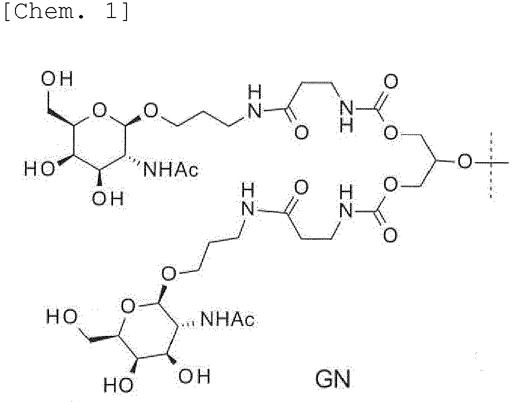 wherein the broken line represents a phosphodiester bond formed with a phosphate group at the 5'-terminus and/or a phosphate group at the 3'-terminus (a phosphate group at the 2'-position in the case of a nucleotide modified at the 3'-position) of an adjacent nucleotide.
